# EUROPEAN PATENT SPECIFICATION

(11) **EP 2 758 534 B1**
(45) Date of publication and mention of the grant of the patent: **29.04.2020**
(21) Application number: 12762377.5
(22) Date of filing: 18.09.2012
(51) Int. Cl.: C12N 15/85, A01K 67/027, C07K 16/46

(54) **ANIMALS, REPERTOIRES & METHODS FOR THE PRODUCTION OF HUMAN ANTIBODIES**
TIERE, REPERTOIRES UND VERFAHREN DAFÜR
ANIMAUX, RÉPERTOIRES & PROCÉDÉS

(30) Priority: 19.09.2011 GB 201116122; 19.09.2011 GB 201116120; 24.02.2012 GB 201203257; 15.03.2012 GB 201204592; 29.03.2012 GB 201205702; 04.05.2012 GB 201207814; 18.05.2012 GB 201208749; 02.07.2012 GB 201211692
(43) Date of publication of application: 30.07.2014
(73) Proprietor: Kymab Limited, Cambridge CB22 3AT (GB)
(72) Inventor: BRADLEY, Allan, Cambridge CB223AT (GB); LEE, E-Chiang, Cambridge CB22 3AT (GB)
(74) Representative: CMS Cameron McKenna Nabarro Olswang LLP
(86) International application number: PCT/GB2012/052297
(87) International publication number: WO 2013/041845

(56) References cited:
- WO-A1-2011/004192
- BRUEGGEMANN M ET AL: "HUMAN ANTIBODY PRODUCTION IN TRANSGENIC MICE: EXPRESSION FROM 100 KB OF THE HUMAN IGH LOCUS", EUROPEAN JOURNAL OF IMMUNOLOGY, WILEY - V C H VERLAG GMBH & CO. KGAA, DE, vol. 21, 1 May 1991 (1991-05-01), pages 1323-1326, XP000944561, ISSN: 0014-2980, DOI: 10.1002/EJI.1830210535
- GREEN L L: "Antibody engineering via genetic engineering of the mouse: XenoMouse strains are a vehicle for the facile generation of therapeutic human monoclonal antibodies", JOURNAL OF IMMUNOLOGICAL METHODS, ELSEVIER SCIENCE PUBLISHERS B.V.,AMSTERDAM, NL, vol. 231, no. 1-2, 10 December 1999 (1999-12-10), pages 11-23, XP004187631, ISSN: 0022-1759, DOI: 10.1016/S0022-1759(99)00137-4
- MENDEZ M J ET AL: "FUNCTIONAL TRANSPLANT OF MEGABASE HUMAN IMMUNOGLOBULIN LOCI RECAPITULATES HUMAN ANTIBODY RESPONSE IN MICE", NATURE GENETICS, NATURE PUBLISHING GROUP, NEW YORK, US, vol. 15, no. 2, 1 February 1997 (1997-02-01), pages 146-156, XP002067603, ISSN: 1061-4036, DOI: 10.1038/NG0297-146
- Anonymous: "Antibody Gene Repertoire Sectoring and Transgenic Mice", ip.com Prior Art Database , 6 July 2012 (2012-07-06), XP002689253, Retrieved from the Internet: URL:http://ip.com/IPCOM/000219567 [retrieved on 2012-12-11]
- MAGADAN S ET AL: "PRODUCTION OF ANTIGEN-SPECIFIC HUMAN MONOCLONAL ANTIBODIES: COMPARISON OF MICE CARRYING IGH/KAPPA OR IGH/KAPPA/LAMBDA TRANSLOCI", BIOTECHNIQUES, INFORMA HEALTHCARE, US, vol. 33, no. 3, 1 September 2002 (2002-09-01), XP001182373, ISSN: 0736-6205
- BEERLI ROGER R ET AL: "Mining human antibody repertoires", MABS, LANDES BIOSCIENCE, US, vol. 2, no. 4, 1 July 2010 (2010-07-01), pages 365-378, XP008148581, ISSN: 1942-0862, DOI: 10.4161/MABS.2.4.12187 [retrieved on 2010-07-01]
- NICHOLSON I C ET AL: "Antibody repertoires of four- and five-feature translocus mice carrying human immunoglobulin heavy chain and kappa and lambda light chain yeast artificial chromosomes", THE JOURNAL OF IMMUNOLOGY, THE AMERICAN ASSOCIATION OF IMMUNOLOGISTS, US, vol. 163, 1 January 1999 (1999-01-01), pages 6898-6906, XP002974255, ISSN: 0022-1767
- WEICHHOLD G M ET AL: "The Human Immunoglobulin kappa Locus Consists of Two Copies That Are Organized in Opposite Polarity", GENOMICS, ACADEMIC PRESS, SAN DIEGO, US, vol. 16, no. 2, 1 May 1993 (1993-05-01), pages 503-511, XP024796526, ISSN: 0888-7543, DOI: 10.1006/GENO.1993.1217 [retrieved on 1993-05-01]
- WEICHHOLD G M ET AL: "MEGABASE INVERSIONS IN THE HUMAN GENOME AS PHYSIOLOGICAL EVENTS", NATURE (LONDON), vol. 347, no. 6288, 1990, pages 90-92, XP002697684, ISSN: 0028-0836
- LEFRANC M P: "Nomenclature of the human immunoglobulin kappa (IGK) genes", EXPERIMENTAL AND CLINICAL IMMUNOGENETICS, S.KARGER, BASEL, CH, vol. 18, no. 3, 1 January 2001 (2001-01-01), pages 161-174, XP009096781, ISSN: 0254-9670, DOI: 10.1159/000049195 cited in the application
- ANDREW MURPHY: "VelocImmune: Immunoglobulin Variable Region Humanized Mice", 1 January 2009 (2009-01-01), RECOMBINANT ANTIBODIES FOR IMMUNOTHERAPY, CAMBRIDGE UNIVERSITY PRESS, GB, PAGE(S) 100 - 107, XP008160379, ISBN: 978-0-521-88732-8 page 104
- JAKOBOVITS AYA ET AL: "From XenoMouse technology to panitumumab, the first fully human antibody product from transgenic mice", NATURE BIOTECHNOLOGY, NATURE PUBLISHING GROUP, NEW YORK, NY, US, vol. 25, no. 10, 1 October 2007 (2007-10-01), pages 1134-1143, XP002556637, ISSN: 1087-0156, DOI: 10.1038/NBT1337 [retrieved on 2007-10-05]

## Description

### FIELD OF THE INVENTION:

### Antibody Gene Repertoire Sectoring & Gene Segment Inversion

The present invention is directed to the concept of sectoring antibody gene segment repertoires in order to enable the development of novel, synthetic antibody chain repertoires not seen in nature. Sectoring exploits the finite B-cell compartments of non-human vertebrates (mice and rats) by artificially biasing the antibody gene segment repertoire available for the production of antibody sequences in the B-cell compartments of individual naive and immunised vertebrates. A plurality of these vertebrates together are useful as a population in immunisation schedules and research programmes to provide for access to a combined, synthetic antibody gene segment repertoire that is beyond that seen in nature and in prior art transgenic vertebrates in which antibody loci have been engineered.

The present invention is also directed to the realisation of the inventors that sectoring can also alter gene segment expression by providing new arrangements of gene segment clusters relative to other gene segments and regulatory elements in transgenic immunoglobulin loci, thereby providing for new synthetic antibody chain sequence repertoires.

To this end, herein described are novel, synthetically-extended antibody repertoires and immunoglobulin heavy and light chain sequence repertoires in non-human vertebrates . Also described are methods of selecting an antibody from said repertoires as well as populations of non-human vertebrates (such as mice or rats) that together provide the novel synthetic (non-naturally occurring) repertoires. The invention provides for use of particular non-human vertebrates that are biased to human lambda variable region expression in the absence of kappa chain expression in sectoring. Disclosed herein are vertebrates useful in sectoring the kappa and lambda V gene repertoires to provide for novel light chain sequence repertoires.

Also described is inversion of vertebrate gene segments and use of these to construct transgenic antibody chain loci in which the inverted gene segments are functional and can contribute to new, synthetic, antibody chain and variable region repertoires.

### BACKGROUND

The state of the art provides non-human vertebrates (eg, mice and rats) and cells comprising transgenic immunoglobulin loci, such loci comprising human variable (V), diversity (D) and/or joining (J) segments, and optionally human constant regions. Alternatively, endogenous constant regions of the host vertebrate (eg, mouse or rat constant regions) are provided in the transgenic loci. Methods of constructing such transgenic vertebrates and use of these to generate antibodies and nucleic acids thereof following antigen immunisation are known in the art, eg, see US7501552 (Medarex); US5939598 & US6130364 (Abgenix); WO02/066630, WO2011163311 & WO2011163314 (Regeneron); WO2011004192 & WO2011158009 (Kymab Limited); WO2009076464, WO2009143472, EP1414858, WO2009013620A2, WO2010070263A1 & WO2010109165A2 (Harbour Antibodies); EP1399559 (Crescendo Biologics) and WO2010039900 (Ablexis).

It would be desirable to improve upon the prior art transgenic non-human vertebrates to provide for novel and potentially expanded repertoires and diversity of antibodies in naive and immunised non-human vertebrates bearing transgenic immunoglobulin loci.

### SUMMARY OF THE INVENTION

The present inventors addressed this by devising ways of sectoring antibody gene segment repertoires by dividing the repertoire across members of a population of antibody-generating non-human vertebrates. In devising this concept, the inventors realised that the potential for accessing greater gene segment sequence diversity (eg, human antibody gene segment and human antibody variable region diversity) would be made possible by biasing B-cell compartments of individual vertebrates to restricted gene segment sub-repertoires, so that overall the population of vertebrates enables one to access novel gene segment (and resultant antibody sequence) repertoires and potentially explore extended ranges of antibody diversities that are not produced by the prior art transgenic vertebrate collections.

By re-distributing gene segment repertoires by sectoring according to the invention, new arrangements of gene segments in immunoglobulin loci can be provided that are not found in nature or in the prior art transgenic animals. For example, when sectoring a substantially complete human functional VH gene repertoire, according to the disclosure, it is possible to provide a new location for VH gene segments that are usually distal in the natural human heavy chain locus (in this instance, VH gene segments that are further away from the DJC region of the locus are more distal than those VH gene segments that are closer, ie proximal VH gene segments). This can be done, for example, by placing a distal VH gene segment cluster (eg, 5 to 10 distal human germline VH gene segments) directly upstream of D and JH genes (eg, a substantially complete human D and JH repertoire). This effectively omits the VH gene segments that are between the distal VH gene cluster and the DJ region in a natural human heavy chain locus. In doing so, a synthetic arrangement is made in which distal VH gene segments are now much closer to the influence of proximal regulatory elements. In this respect, it has been observed that proximal V gene segments in natural loci are often recombined more frequently than distal V gene segments. Thus, by moving the distal gene segments more proximally, there is provided the possibility of altering the usage of these gene segments beyond that seen in nature. This aids the provision of novel synthetic repertoires in the present disclosure. Additionally, this enables the skilled person to omit gene segments that tend to dominate immune responses in vertebrates so that the utility of other gene segments can be more fully explored. The omitted gene segments may, for example, lead to relatively low affinity antibodies that dominate immune antibody repertoires in immunised mice or other non-human vertebrates. The omission removes these undesirable antibodies from the repertoire used for selection of antibody drug candidate leads. Additionally or alternatively, omission of dominating antibodies that bind a specific epitope may be desirable to enable generation and selection of antibodies that bind novel, desirable epitopes on the target antigen, eg, novel neutralising epitopes.

To this end, the invention is set out in the claims and the disclosure provides the following.

### In a first Configuration

In a first aspect: A method of providing a synthetic antibody heavy chain sequence repertoire, the method comprising providing a heavy chain variable region gene segment repertoire that is divided across the genomes of two or more non-human vertebrates in which endogenous heavy chain expression is substantially inactive, the repertoire gene segments in the genomes being provided as part of transgenic heavy chain loci comprising one or more VH gene segments, one or more D gene segments and one or JH gene segments functionally connected upstream of a heavy chain constant region (eg, Cmu and/or Cgamma), wherein the genomes can express different repertoires of antibody heavy chain sequences derived from VH, D and JH gene segments;

Wherein the gene segment repertoire is selected from the group consisting of:
(a) a VH gene repertoire (eg, a human VH gene repertoire or a substantially complete functional human VH gene repertoire);
(b) a D gene repertoire (eg, a human D gene repertoire or a substantially complete functional human D gene repertoire); and
(c) a JH gene repertoire (eg, a human JH gene repertoire or a substantially complete functional human JH gene repertoire);

Optionally wherein the D and JH segments in the loci are human D and JH segments.

In a second aspect: A method of providing a synthetic antibody kappa chain sequence repertoire, the method comprising providing a kappa chain variable region gene segment repertoire that is divided across the genomes of two or more non-human vertebrates in which endogenous kappa chain (and optionally also endogenous lambda chain) expression is substantially inactive, the repertoire gene segments in the genomes being provided as part of transgenic light chain loci comprising one or more Vκ gene segments and one or more Jκ gene segments functionally connected upstream of a light chain constant region (eg, Cκ and/or Cλ), wherein the genomes can express different repertoires of antibody kappa chain sequences derived from Vκ and Jκ gene segments;

Wherein the gene segment repertoire is selected from the group consisting of:
(a) a Vκ gene repertoire (eg, a human Vκ gene repertoire or a substantially complete functional human Vκ gene repertoire); and
(c) a Jκ gene repertoire (eg, a human Jκ gene repertoire or a substantially complete functional human Jκ gene repertoire);

Optionally wherein the Jκ segments in the loci are human Jκ segments.

In a third aspect: A method of providing a synthetic antibody lambda chain sequence repertoire, the method comprising providing a lambda chain variable region gene segment repertoire that is divided across the genomes of two or more non-human vertebrates in which endogenous lambda chain (and optionally also endogenous kappa chain) expression is substantially inactive, the repertoire gene segments in the genomes being provided as part of transgenic light chain loci comprising one or more Vλ gene segments and one or more Jλ gene segments functionally connected upstream of a light chain constant region (eg, Cκ and/or Cλ), wherein the genomes can express different repertoires of antibody lambda chain sequences derived from Vλ and Jλ gene segments;

Wherein the gene segment repertoire is selected from the group consisting of:
(a) a Vλ gene repertoire (eg, a human Vλ gene repertoire or a substantially complete functional human Vλ gene repertoire); and
(c) a Jλ gene repertoire (eg, a human Jλ gene repertoire or a substantially complete functional human Jλ gene repertoire);

Optionally wherein the Jλ segments in the loci are human Jλ segments.

In a fourth aspect: A method of providing a synthetic antibody light chain sequence repertoire, the method comprising providing

a Vκ gene repertoire in the genomes of a first group of non-human vertebrates in which endogenous lambda chain (and optionally also endogenous kappa chain) expression is substantially inactive, the Vκ genes in the genomes being provided as part of transgenic light chain loci comprising one or more Jκ gene segments functionally connected upstream of a light chain constant region (eg, Cκ and/or Cλ, eg, human Cλ), wherein the genomes can express repertoires of antibody kappa light chain sequences derived from Vκ and Jκ gene segments substantially in the absence of lambda light chain expression; and

a Vλ gene repertoire in the genomes of a second group of non-human vertebrates in which endogenous kappa chain (and optionally also endogenous lambda chain) expression is substantially inactive, the Vλ genes in the genomes being provided as part of transgenic light chain loci comprising one or more Jλ gene segments functionally connected upstream of a light chain constant region (eg, Cκ and/or Cλ, eg, human Cλ), wherein the genomes can express repertoires of antibody lambda light chain sequences derived from human Vλ and Jλ gene segments substantially in the absence of kappa light chain expression;

Optionally wherein the Vκ gene repertoire is a human Vκ gene repertoire (eg, a substantially complete functional human Vκ gene repertoire), the Vλ gene repertoire is a human Vλ gene repertoire (eg, a substantially complete functional human Vλ gene repertoire), the Jκ segments in the loci are human Jκ segments and the Jλ segments in the loci are human Jλ segments;

Optionally wherein the genomes of the first and second groups comprise a substantially complete functional VH gene repertoire of a human.

In a fifth aspect: A method of providing a synthetic antibody heavy chain sequence repertoire, the method comprising providing a VH gene repertoire (eg, a substantially complete functional human VH gene repertoire) that is divided across the genomes of two or more non-human vertebrates in which endogenous heavy chain expression is substantially inactive, the VH genes in the genomes being provided as part of transgenic heavy chain loci comprising one or more human JH gene segments and one or more human D gene segments functionally connected upstream of a heavy chain constant region (eg, Cmu and/or Cgamma), wherein the genomes can express different repertoires of antibody heavy chain sequences derived from human VH, D and JH gene segments. Optionally the VH gene repertoire is a human VH gene repertoire.

In a sixth aspect: A method of providing a synthetic antibody heavy chain sequence repertoire, the method comprising providing a JH gene repertoire (eg, a substantially complete functional human JH gene repertoire) that is divided across the genomes of two or more non-human vertebrates in which endogenous heavy chain expression is substantially inactive, the JH genes in the genomes being provided as part of transgenic heavy chain loci comprising one or more human VH gene segments and one or more human D gene segments functionally connected upstream of a heavy chain constant region (eg, Cmu and/or Cgamma), wherein the genomes can express different repertoires of antibody heavy chain sequences derived from human VH, D and JH gene segments. Optionally the JH gene repertoire is a human JH gene repertoire.

In a seventh aspect: A method of providing a synthetic antibody heavy chain sequence repertoire, the method comprising providing a D gene repertoire (eg, a substantially complete functional human JH gene repertoire) that is divided across the genomes of two or more non-human vertebrates in which endogenous heavy chain expression is substantially inactive, the D genes in the genomes being provided as part of transgenic heavy chain loci comprising one or more human VH gene segments and one or more human JH gene segments functionally connected upstream of a heavy chain constant region (eg, Cmu and/or Cgamma), wherein the genomes can express different repertoires of antibody heavy chain sequences derived from human VH, D and JH gene segments. Optionally the D gene repertoire is a human D gene repertoire.

### In a second Configuration

A method of providing a synthetic antibody kappa light chain sequence repertoire, the method comprising providing a Vκ gene repertoire (eg, a substantially complete functional human Vκ gene repertoire) that is divided across the genomes of two or more non-human vertebrates in which endogenous kappa chain (and optionally also endogenous lambda chain) expression is substantially inactive, the Vκ genes in the genomes being provided as part of transgenic light chain loci comprising one or more human Jκ gene segments functionally connected upstream of a light chain constant region (eg, Cκ and/or Cλ), wherein the genomes can express different repertoires of antibody kappa light chain sequences derived from human Vκ and Jκ gene segments. Optionally, the Vκ gene repertoire is a human Vκ gene repertoire.

A method of providing a synthetic antibody kappa light chain sequence repertoire, the method comprising providing a Jκ gene repertoire (eg, a substantially complete functional human Jκ gene repertoire) that is divided across the genomes of two or more non-human vertebrates in which endogenous kappa chain (and optionally also endogenous lambda chain) expression is substantially inactive, the Jκ genes in the genomes being provided as part of transgenic light chain loci comprising one or more human Vκ gene segments functionally connected upstream of a light chain constant region (eg, Cκ and/or Cλ), wherein the genomes can express different repertoires of antibody kappa light chain sequences derived from human Vκ and Jκ gene segments. Optionally, the Jκ gene repertoire is a human Jκ gene repertoire.

### In a third Configuration

A method of providing a synthetic antibody lambda light chain sequence repertoire, the method comprising providing a Vλ gene repertoire (eg, a substantially complete functional human Vλ gene repertoire) that is divided across the genomes of two or more non-human vertebrates in which endogenous lambda chain (and optionally also endogenous kappa chain) expression is substantially inactive, the Vλ genes in the genomes being provided as part of transgenic light chain loci comprising one or more human Jλ gene segments functionally connected upstream of a light chain constant region (eg, Cκ and/or Cλ, eg, human Cλ), wherein the genomes can express different repertoires of antibody lambda light chain sequences derived from human Vλ and Jλ gene segments. Optionally, the Vλ gene repertoire is a human Vλ gene repertoire.

A method of providing a synthetic antibody lambda light chain sequence repertoire, the method comprising providing Jλ gene repertoire (eg, a substantially complete functional human Jλ gene repertoire) that is divided across the genomes of two or more non-human vertebrates in which endogenous lambda chain (and optionally also endogenous kappa chain) expression is substantially inactive, the Jλ genes in the genomes being provided as part of transgenic light chain loci comprising one or more human Vλ gene segments functionally connected upstream of a light chain constant region (eg, Cκ and/or Cλ, eg, human Cλ), wherein the genomes can express different repertoires of antibody lambda light chain sequences derived from human Vλ and Jλ gene segments. Optionally, the Jλ gene repertoire is a human Jλ gene repertoire.

### In a fourth Configuration

A method of providing a synthetic antibody light chain sequence repertoire, the method comprising providing
a Vκ gene repertoire (eg, a substantially complete functional human Vκ gene repertoire) in the genomes of a first group of non-human vertebrates in which endogenous lambda chain (and optionally also endogenous kappa chain) expression is substantially inactive, the Vκ genes in the genomes being provided as part of transgenic light chain loci comprising one or more human Jκ gene segments functionally connected upstream of a light chain constant region (eg, Cκ and/or Cλ, eg, human Cλ), wherein the genomes can express repertoires of antibody kappa light chain sequences derived from human Vκ and Jκ gene segments substantially in the absence of kappa light chain expression; and
a Vλ gene repertoire (eg, a substantially complete functional human Vλ gene repertoire) in the genomes of a second group of non-human vertebrates in which endogenous kappa chain (and optionally also endogenous lambda chain) expression is substantially inactive, the Vλ genes in the genomes being provided as part of transgenic light chain loci comprising one or more human Jλ gene segments functionally connected upstream of a light chain constant region (eg, Cκ and/or Cλ, eg, human Cλ), wherein the genomes can express repertoires of antibody lambda light chain sequences derived from human Vλ and Jλ gene segments substantially in the absence of kappa light chain expression;
Optionally wherein the genomes of the first and second groups comprise a substantially complete functional VH gene repertoire of a human;
Optionally wherein the Vκ and Vλ gene repertoires are human Vκ and Vλ gene repertoires.

### In a fifth Configuration

A method of providing a synthetic antibody heavy chain sequence repertoire, the method comprising
(a) providing a population of transgenic non-human vertebrates (optionally mice or rats), wherein the population provides a repertoire of different human VH gene segments, the repertoire being divided between two or more vertebrates of said population,
(b) a first vertebrate of said population comprising a transgenic heavy chain locus comprising one or more human VH gene segments (first VH gene sub-repertoire), D segments and J segments operably connected upstream of a constant region ; and
(c) a second vertebrate of said population comprising a transgenic heavy chain locus comprising one or more human VH gene segments (second VH gene sub-repertoire), D segments and J segments operably connected upstream of a constant region;
(d) wherein the first VH gene sub-repertoire is different from the second VH gene sub-repertoire, whereby the first vertebrate can produce a heavy chain sequence repertoire that is different from the heavy chain sequence repertoire produced by the second vertebrate.

### In a sixth Configuration

A method of providing a synthetic antibody light chain sequence repertoire, the method comprising
(a) providing a population of transgenic non-human vertebrates (optionally mice or rats), wherein the population provides a repertoire of different human VL gene segments, the repertoire being divided between two or more vertebrates of said population,
(b) a first vertebrate of said population comprising a transgenic light chain locus comprising one or more human VL gene segments (first VL gene sub-repertoire) and J segments operably connected upstream of a constant region ; and
(c) a second vertebrate of said population comprising a transgenic light chain locus comprising one or more human VL gene segments (second VL gene sub-repertoire) and J segments operably connected upstream of a constant region;
(d) wherein the first VL gene sub-repertoire is different from the second VL gene sub-repertoire, whereby the first vertebrate can produce a light chain sequence repertoire that is different from the light chain sequence repertoire produced by the second vertebrate.

### In a seventh Configuration

A method of selecting an antibody that binds a predetermined antigen, the method comprising
(a) providing a repertoire of antibodies (first repertoire) that bind said antigen, wherein the antibodies comprise human heavy and light chain variable regions and the repertoire comprises
   i. A sub-repertoire of antibodies (lambda sub-repertoire) whose light chain variable regions are produced by rearrangement of a human Vλ gene segment with a human J_{L} gene segment; and
   ii. A sub-repertoire of antibodies (kappa sub-repertoire) whose light chain variable regions are produced by rearrangement of a human Vκ gene segment with a human J_{L} gene segment;
(b) selecting one or more antibodies from the lambda sub-repertoire according to a desired antibody characteristic (eg, binding affinity for said antigen);
(c) selecting one or more antibodies from the kappa sub-repertoire according to a desired antibody characteristic;
wherein a repertoire (second repertoire) of selected lambda and kappa antibodies is produced, the antibodies of the second repertoire comprising human variable regions that bind said antigen; and (d) Selecting one or more antibodies from said second repertoire on the basis of said desired antibody characteristic;
Wherein in step (a)(i) the lambda sub-repertoire is produced by immunisation of one or more non-human vertebrates (optionally mice or rats) (lambda vertebrates)with said antigen, wherein the lambda vertebrates express more human lambda-type antibodies (antibodies whose light chain variable regions are derived from the rearrangement of a human Vλ gene segment) than kappa-type antibodies (antibodies whose light chain variable regions are derived from the rearrangement of a VK gene segment);
Wherein in step (a)(ii) the kappa sub-repertoire is produced immunisation of one or more non-human vertebrates (optionally mice or rats) (kappa vertebrates) with said antigen, wherein the kappa vertebrates express more human kappa-type antibodies (antibodies whose light chain variable regions are derived from the rearrangement of a human Vκ gene segment) than lambda-type antibodies (antibodies whose light chain variable regions are derived from the rearrangement of a Vλ gene segment).

### In an eighth Configuration

A population of transgenic non-human vertebrates (optionally mice or rats), wherein the population provides a repertoire of different human VH gene segments, the repertoire being divided between two or more vertebrates of said population,
(a) a first vertebrate of said population comprising a transgenic heavy chain locus comprising one or more human VH gene segments (first VH gene sub-repertoire), D segments and J segments operably connected upstream of a constant region ; and
(b) a second vertebrate of said population comprising a transgenic heavy chain locus comprising one or more human VH gene segments (second VH gene sub-repertoire), D segments and J segments operably connected upstream of a constant region;
(c) wherein the first VH gene sub-repertoire is different from the second VH gene sub-repertoire for expression of first and second antibody heavy chain sequence repertoires respectively that are different from each other, whereby the population provides a synthetic repertoire of antibody heavy chain sequences.

### In a ninth Configuration

A population of transgenic non-human vertebrates (optionally mice or rats), wherein the population provides a repertoire of different human VL gene segments, the repertoire being divided between two or more vertebrates of said population,
(a) a first vertebrate of said population comprising a transgenic light chain locus comprising one or more human VL gene segments (first VL gene sub-repertoire) and J segments operably connected upstream of a constant region ; and
(b) a second vertebrate of said population comprising a transgenic light chain locus comprising one or more human VL gene segments (second VL gene sub-repertoire) and J segments operably connected upstream of a constant region;
(c) wherein the first VL gene sub-repertoire is different from the second VL gene sub-repertoire for expression of first and second antibody light chain sequence repertoires respectively that are different from each other, whereby the population provides a synthetic repertoire of antibody light chain sequences.

### In a tenth Configuration

A population of non-human vertebrates (optionally mice or rats), wherein the genome of each vertebrate comprises:
(a) One or more transgenic immunoglobulin heavy chain loci, each locus comprising one or more human V gene segments, one or more human D gene segments and one or more human J gene segments upstream of one or more heavy chain constant regions; and
(b) One or more transgenic immunoglobulin light chain loci, each locus comprising one or more human V_{L} gene segments and one or more human J_{L} gene segments upstream of one or more light chain constant regions;
Wherein in each vertebrate the gene segments in transgenic heavy chain loci are operably linked to the constant region thereof, and the gene segments in transgenic light chain loci are operably linked to the constant region thereof, so that upon immunisation the vertebrate is capable of producing an antibody comprising heavy chains produced by recombination of a heavy chain locus and light chains produced by recombination of a light chain locus, wherein the heavy and light chains comprise human variable regions;
Wherein the population comprises
(i) a first vertebrate type (lambda vertebrates) wherein said light chain loci comprise one or more human Vλ gene segments, wherein following rearrangement the loci express light chain sequences comprising variable region sequences derived from human Vλ gene segments (human lambda light chain sequences), wherein the lambda vertebrates express more lambda light chain sequences than kappa light chain sequences (sequences of light chains comprising variable region sequences derived from Vκ gene segments); and
(ii) a second vertebrate type (kappa vertebrates) wherein said light chain loci comprise one or more human Vκ gene segments, wherein following rearrangement the loci express light chain sequences comprising variable region sequences derived from human Vκ gene segments (human kappa light chain sequences), wherein the kappa vertebrates express more kappa light chain sequences than lambda light chain sequences (sequences of light chains comprising variable regions derived from Vλ gene segments);
wherein the vertebrates of said population can be immunised with the same antigen to produce a repertoire of antibodies comprising human heavy and light chain variable regions, wherein the repertoire comprises a sub-repertoire of human lambda antibodies (lambda sub-repertoire) produced by vertebrates of the first type and a sub-repertoire of human kappa antibodies (kappa sub-repertoire) produced by vertebrates of the second type.

### In an eleventh Configuration

A non-human vertebrate (optionally a mouse or rat), wherein the genome of each vertebrate comprises:
(c) One or more transgenic immunoglobulin heavy chain loci, each locus comprising one or more human V gene segments, one or more human D gene segments and one or more human J gene segments upstream of one or more heavy chain constant regions; and
(d) One or more transgenic immunoglobulin light chain loci, each locus comprising a human Vλ gene segment repertoire and one or more human Jλ gene segments upstream of one or more light chain constant regions;
Wherein following rearrangement the light chain loci express light chain sequences comprising variable region sequences derived from human Vλ gene segments (human lambda light chain sequences);
Wherein the kappa (and optionally endogenous lambda) light chain expression has been substantially inactivated so that the vertebrate expresses more human lambda light chain sequences than kappa light chain sequences (sequences of light chains comprising variable region sequences derived from Vκ gene segments);
Wherein endogenous heavy chain expression has been substantially inactivated; and Wherein each said transgenic light chain locus comprises a substantially complete functional Vλ gene segment repertoire of a human.

### In a twelfth configuration:

A method of providing a synthetic antibody heavy chain repertoire, the method comprising
(a) Dividing a human VH gene segment repertoire (eg, a substantially complete functional human VH gene repertoire) across the genomes of at least first and second non-human vertebrates (eg, mice or rats), the repertoire comprising
   a first cluster of VH gene segments corresponding to a distal VH gene cluster of the heavy chain locus of a human; and
   a second cluster of VH gene segments corresponding to a proximal VH gene cluster of the heavy chain locus of a human, wherein the proximal cluster is arranged proximally to the distal cluster in said human locus;
   Wherein the distal cluster is provided in a heavy chain locus of said first vertebrate upstream of one or more D gene segments, one or more JH gene segments and one or more constant regions; Wherein the proximal cluster is provided in a heavy chain locus of said second vertebrate upstream of one or more D gene segments, one or more JH gene segments and one or more constant regions; Wherein the proximal VH gene cluster is not present between the distal cluster and the D gene segments in the heavy chain locus of the first vertebrate (optionally wherein no further VH gene segments are present between the distal cluster and the D gene segments in the heavy chain locus of the first vertebrate); and
(b) Expressing said heavy chain loci of the first and second vertebrates to provide a repertoire of synthetic antibody heavy chains.

A method of providing a synthetic antibody kappa chain repertoire, the method comprising
(a) Dividing a human Vκ gene segment repertoire (eg, a substantially complete functional human Vκ gene repertoire) across the genomes of at least first and second non-human vertebrates (eg, mice or rats), the repertoire comprising
   a first cluster of Vκ gene segments corresponding to a distal Vκ gene cluster of the kappa chain locus of a human; and
   a second cluster of Vκ gene segments corresponding to a proximal Vκ gene cluster of the kappa chain locus of a human, wherein the proximal cluster is arranged proximally to the distal cluster in said human locus;
   Wherein the distal cluster is provided in a kappa chain locus of said first vertebrate upstream of one or more Jκ gene segments and one or more constant regions;
   Wherein the proximal cluster is provided in a kappa chain locus of said second vertebrate upstream of one or more Jκ gene segments and one or more constant regions;
   Wherein the proximal Vκ gene cluster is not present between the distal cluster and the Jκ gene segments in the kappa chain locus of the first vertebrate (optionally wherein no further Vκ gene segments are present between the distal cluster and the Jκ gene segments in the kappa chain locus of the first vertebrate); and
(b) Expressing said kappa chain loci of the first and second vertebrates to provide a repertoire of synthetic antibody kappa chains.

A method of providing a synthetic antibody lambda chain repertoire, the method comprising
(a) Dividing a human Vλ gene segment repertoire (eg, a substantially complete functional human Vλ gene repertoire) across the genomes of at least first and second non-human vertebrates (eg, mice or rats), the repertoire comprising
   a first cluster of Vλ gene segments corresponding to a distal Vλ gene cluster of the lambda chain locus of a human; and
   a second cluster of Vλ gene segments corresponding to a proximal Vλ gene cluster of the lambda chain locus of a human, wherein the proximal cluster is arranged proximally to the distal cluster in said human locus;
   Wherein the distal cluster is provided in a lambda chain locus of said first vertebrate upstream of one or more Jλ gene segments and one or more constant regions;
   Wherein the proximal cluster is provided in a lambda chain locus of said second vertebrate upstream of one or more Jλ gene segments and one or more constant regions;
   Wherein the proximal Vλ gene cluster is not present between the distal cluster and the Jλ gene segments in the lambda chain locus of the first vertebrate (optionally wherein no further Vλ gene segments are present between the distal cluster and the Jλ gene segments in the lambda chain locus of the first vertebrate); and
(b) Expressing said lambda chain loci of the first and second vertebrates to provide a repertoire of synthetic antibody lambda chains.

### In a thirteenth configuration:

A non-human vertebrate (optionally a mouse or a rat) or vertebrate cell (optionally a mouse cell or a rat cell) whose genome comprises a transgenic antibody chain locus comprising one or more human V gene segments and one or more human J gene segments (and optionally one or more human D gene segments) upstream of a constant region, the locus comprising one or more inverted vertebrate species gene segments, the inverted gene segment(s) being present in the locus in a 5'-3' orientation that is opposite to the vertebrate species germline orientation of such segment(s), and wherein the non-human vertebrate or cell is capable of expressing an antibody chain sequence comprising a sequence that is derived from an inverted gene segment.

Thus, the gene segments are naturally present in an opposite orientation to the corresponding CL or Cµ in the germline locus of a vertebrate of the relevant species (eg, human), but by virtue of the present disclosure these are inverted so that the gene segment orientation is the same as the CL or Cµ.

A non-human vertebrate (optionally a mouse or a rat) or vertebrate cell (optionally a mouse cell or a rat cell) whose genome comprises a transgenic antibody chain locus comprising one or more human V gene segments and one or more human J gene segments (and optionally one or more human D gene segments) upstream of a constant region, wherein the locus comprises one or more inverted human gene segments, the inverted human gene segment(s) being present in the locus in a 5'-3' orientation that is opposite to the human germline orientation of such segment(s), and wherein the vertebrate or cell is capable of expressing an antibody chain sequence comprising a variable region that is derived from recombination of an inverted gene segment.

A method of providing an artificial human antibody variable region repertoire, the method comprising inserting one or more human V gene segment(s) (inverted gene segments) upstream of one or more J gene segments, optionally one or more D gene segments, and a constant region in an antibody chain locus of a non-human vertebrate or non-human vertebrate cell, the V gene segment(s) being present in the locus in a 5'-3' orientation that is opposite to the human germline orientation of such segment(s), and wherein the non-human vertebrate or cell (or a non-human vertebrate progeny derived from the cell) is capable of expressing an antibody chain sequence comprising a variable region sequence that is derived from recombination of an inverted gene segment.

A method of providing an artificial human antibody variable region repertoire, the method comprising isolating serum or lymphoid cells (eg, spleen cells or B-cells) from a vertebrate described above, and optionally isolating from the serum or cells one or more antibodies that specifically bind a predetermined antigen.

### BRIEF DESCRIPTION OF THE FIGURES

Figure 1: Schematic showing calculation of potential IgH antibody variable region diversity for a transgenic human immunoglobulin heavy chain locus containing 6 human JH gene segments, 27 human D gene segments and 40 VH gene segments.
Figure 2: Schematic showing calculation of potential Igκ and Igλ antibody variable region diversity for transgenic human immunoglobulin light chain kappa and lambda loci, wherein each light chain locus contains 5 human J_{L} gene segments and 40 V_{L} gene segments.
Figure 3: Schematic showing calculation of potential IgH, Igκ and Igλ antibody variable region diversity for the transgenic human immunoglobulin loci, giving a total potential antibody repertoire size of 4x10¹⁴.
Figure 4: Schematic showing the typical sampling size in prior art methods of antibody selection from transgenic mice and the typical resultant hybridoma population size.
Figure 5: Schematic showing sectoring of the light chain diversity across several mice in a population according to the disclosure and the resultant beneficial new and extended repertoire for sampling.
Figure 6: Schematic showing sectoring of human functional light chain diversity and human functional VH gene segment diversity across several mice in a population according to the disclosure and the resultant beneficial new and extended repertoire for sampling.
Figure 7: shows the human gene segment repertoires contained in the first, second and third BACs used to construct three different mice lines, K1, K2 and K3:-
   K1: comprises an endogenous Ig kappa locus in which has been inserted the human gene segments Vκ1-19 to Jκ5;
   K2: comprises an endogenous Ig kappa locus in which has been inserted the human gene segments Vκ2-24 to Jκ5; and
   K3: comprises an endogenous Ig kappa locus in which has been inserted the human gene segments Vκ2D-40 to Jκ5.

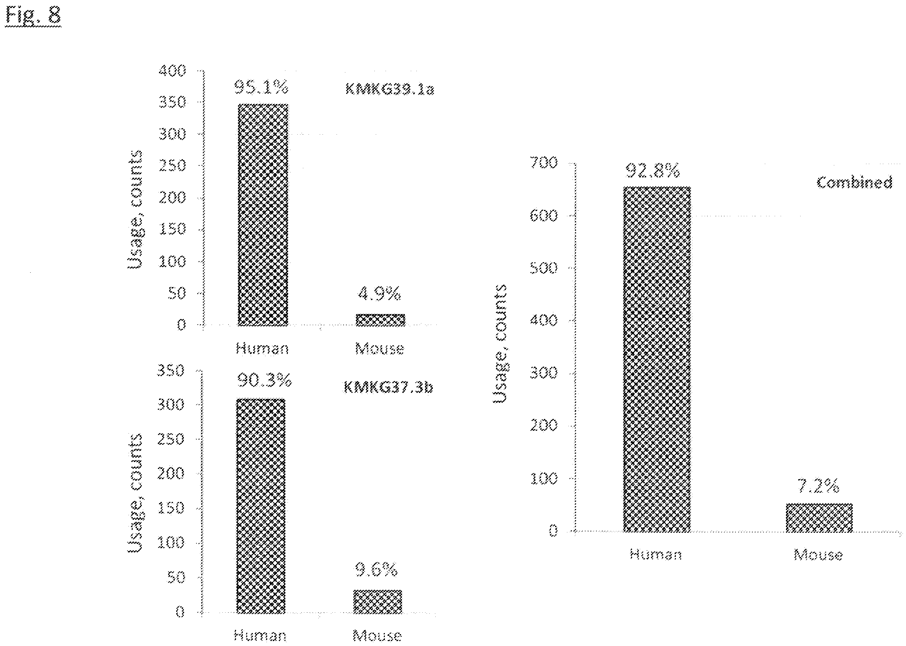

Figure 8: shows total human Vκ gene segment usage versus mouse Vκ usage in transcripts from mice comprising an insertion of human Vκ and Jκ gene segments into an endogenous mouse kappa locus between the 3'-most mouse Jκ and the mouse Cκ.
   K3/KA - K3 - the first endogenous kappa allele has three kappa chain locus DNA insertions between the most 3' endogenous Jκ and the mouse Cκ, providing an insertion of 20 human Vκ and Jκ1-Jκ5; and (ii) KA - the second endogenous kappa allele has been inactivated (by insertion of an endogenous interrupting sequence). This arrangement encodes exclusively for kappa light chains from the first endogenous kappa allele.

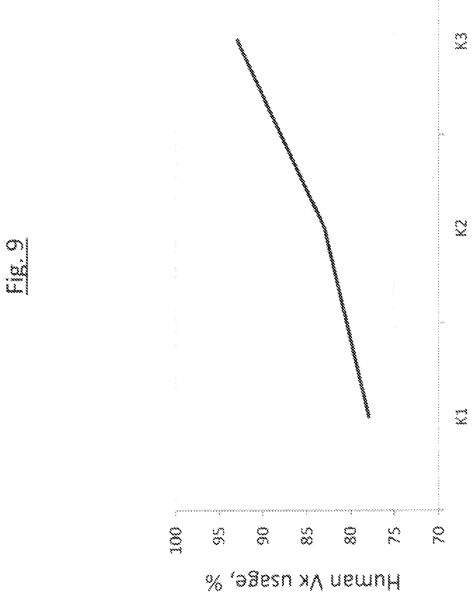

Figure 9: illustrates the improvement in human Vκ gene segment usage in transcripts (and thus improvement in inactivation of endogenous mouse Vκ gene segment usage) as human kappa locus DNA is inserted in successive BACs 1-3. BAC1 inserts 6 human Vκ and Jκ1-Jκ5 between the 3' endogenous Jκ and the mouse Cκ. BAC2 inserts an additional 8 human Vκ gene segments 5' to the human DNA inserted from the BAC1, resulting in 14 human Vκ and Jκ1-Jκ5 between the 3' endogenous Jκ and the mouse Cκ. BAC3 inserts an additional 6 human Vκ gene segments 5' to the human DNA inserted from the BAC2, resulting in 20 human Vκ and Jκ1-Jκ5 between the 3' endogenous Jκ and the mouse Cκ. With each insertion, the endogenous mouse VJ region is pushed further upstream (5') and inactivation is enhanced.

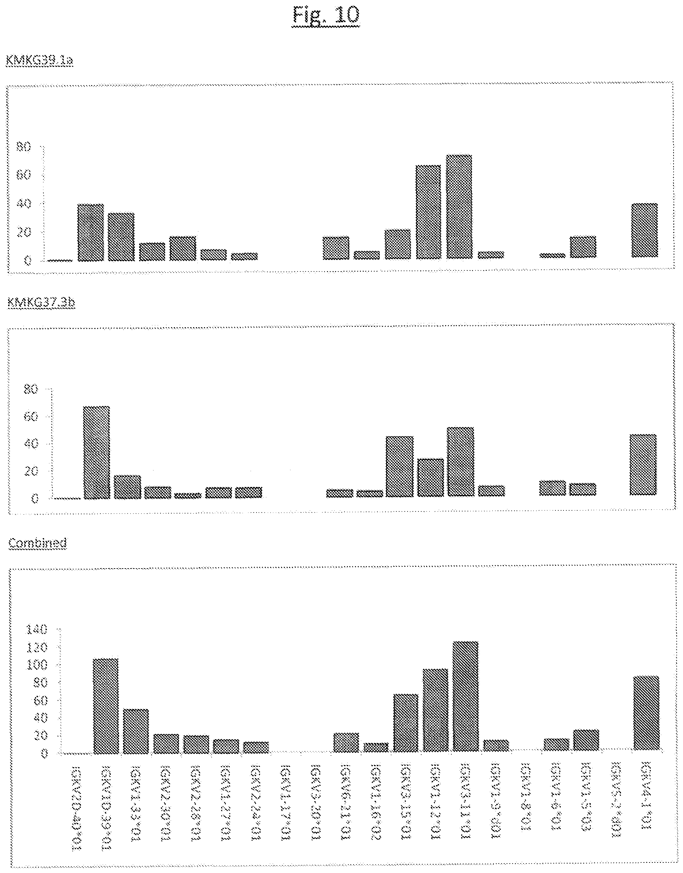

Figure 10: illustrates the distribution of human Vκ usage at the transcript level from the K3/KA mice.

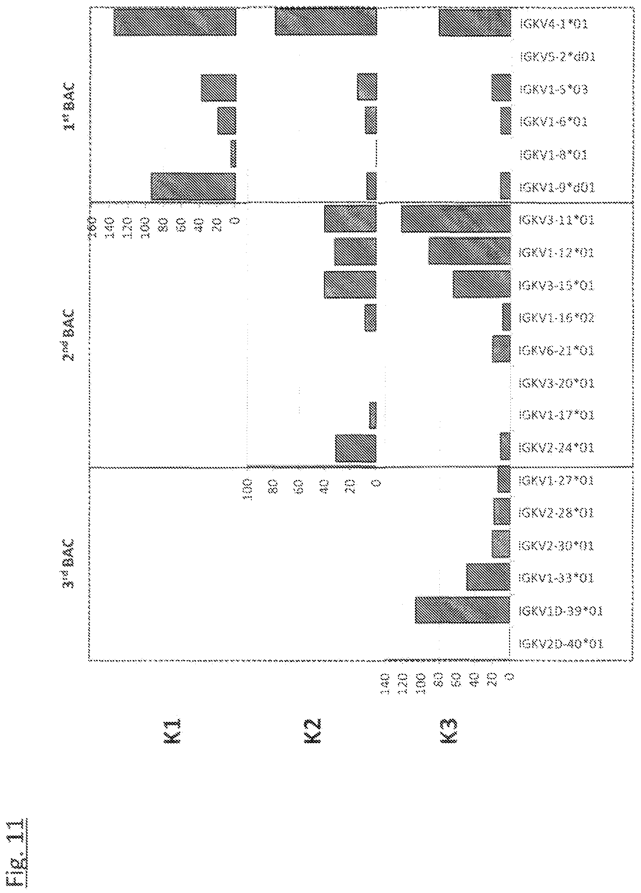

Figure 11: compares the effect of sectoring the 20 human Vκ gene segment repertoire across three different mice-types (corresponding to insertion of human gene segment DNA from BAC1 only (K1 genotype), BACs1+2 (K2 genotype) and BACs1+2+3 (K3 genotype). Illustrated is the distribution of human Vκ usage at the transcript level from the mice. Different Vκ usage was seen resulting in mice that produced different kappa chain repertoires (and corresponding different human kappa variable region repertoires) as a result of the human gene repertoire sectoring.

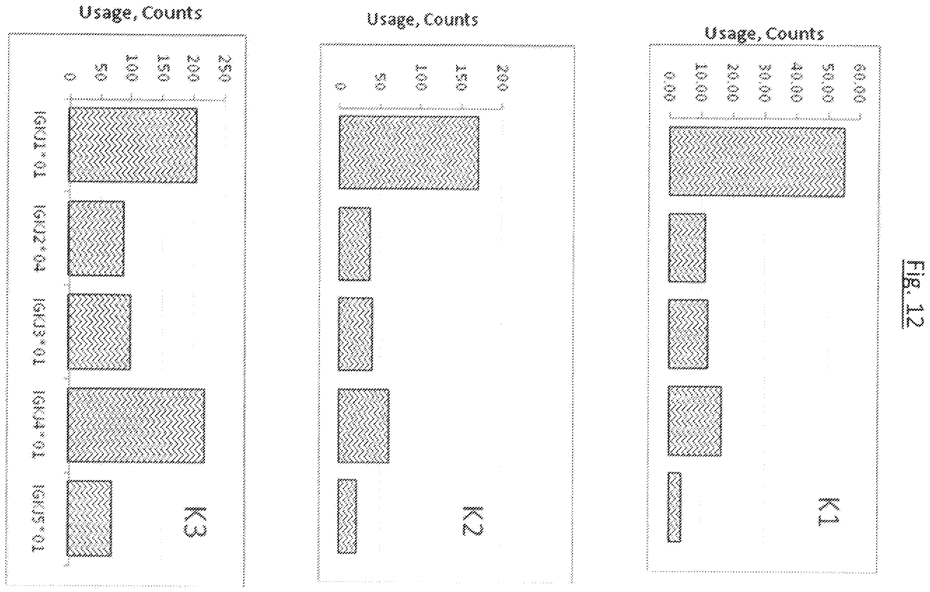

Figure 12: compares the effect of sectoring the human Jκ gene segment repertoire across three different mice-types (K1, K2 and K3). Illustrated is the distribution of human Jκ usage at the transcript level from the mice. Different Jκ usage was seen resulting in mice that produced different kappa chain repertoires (and corresponding different human kappa variable region repertoires) as a result of the human gene repertoire sectoring.

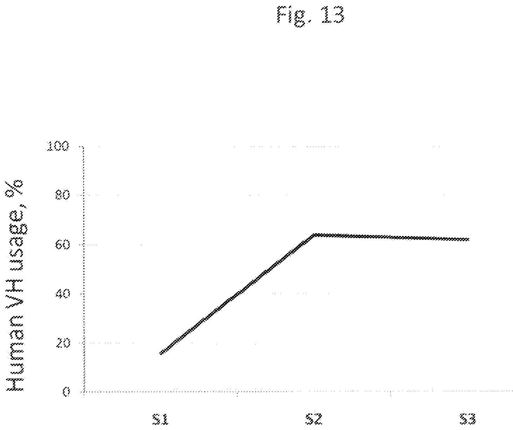

Figure 13: illustrates the improvement in human VH gene segment usage in transcripts (and thus improvement in inactivation of endogenous mouse VH gene segment usage) as human heavy chain locus DNA is inserted in successive BACs 1-3 (producing S1 chimaeric heavy chain locus (human VDJ gene segments from BAC1 only have been inserted), S2 chimaeric heavy chain locus (human VDJ gene segments from BACs1 & 2 have been inserted) and S3 chimaeric heavy chain locus ((human VDJ gene segments from BACs 1, 2 & 3 have been inserted)). BAC1 inserts 6 human VH and all functional human DH and JH gene segments between the 3' endogenous JH (mouse JH4) and the mouse C-mu. BAC2 inserts an additional 5 human VH gene segments 5' to the human DNA inserted from the BAC1, resulting in 11 human VH and all functional human DH and JH between the 3' endogenous JH4 and the mouse C-mu. BAC3 inserts an additional 7 human VH gene segments 5' to the human DNA inserted from the BAC2, resulting in 18 human VH and all functional human DH and JH between the 3' endogenous JH4 and the mouse C-mu. With each insertion, the endogenous mouse heavy chain VDJ region is pushed further upstream (5') and inactivation is enhanced.
   BAC1 human gene segments: VH2-5, 7-4-1, 4-4, 1-3, 1-2, 6-1, and all the human D and JH gene segments D1-1, 2-2, 3-9, 3-10, 4-11, 5-12, 6-13, 1-14, 2-15, 3-16, 4-17, 5-18, 6-19, 1-20, 2-21, 3-22, 4-23, 5-24, 6-25, 1-26 and 7-27; and J1, J2, J3, J4, J5 and J6.
   BAC2 human gene segments: VH3-7, 1-8, 3-9, 3-11 and 3-13.
   BAC3 human gene segments: VH3-15, 1-18, 3-20, 3-21, 3-23, 1-24 and 2-26.

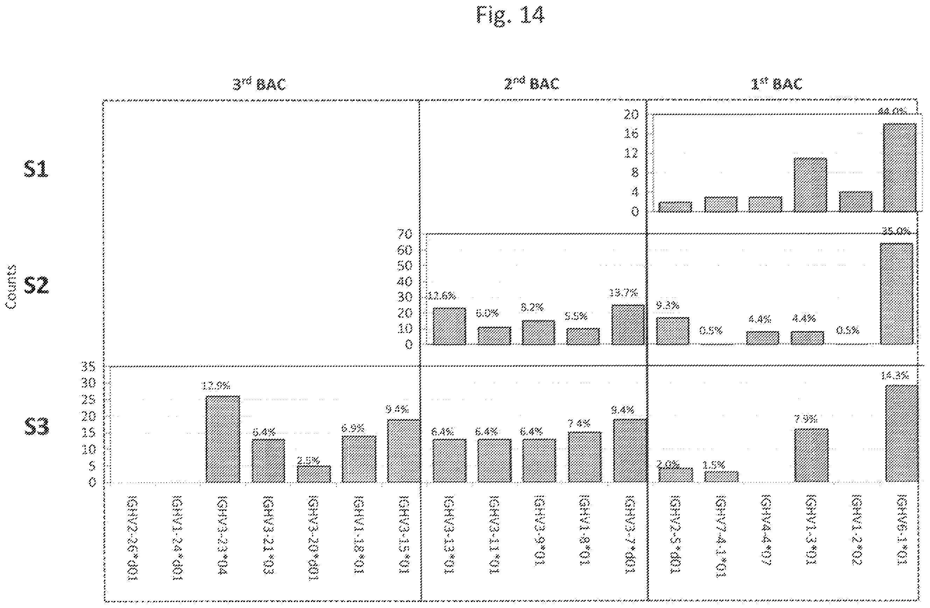

Figure 14: compares the effect of sectoring the 18 human VH gene segment repertoire across three different mice-types (corresponding to insertion of human gene segment DNA from BAC1 only (S1 genotype), BACs1+2 (S2 genotype) and BACs1+2+3 (S3 genotype). Illustrated is the distribution of human VH usage at the transcript level from the mice. Different VH usage was seen resulting in mice that produced different heavy chain repertoires (and corresponding different human heavy chain variable region repertoires) as a result of the human gene repertoire sectoring.

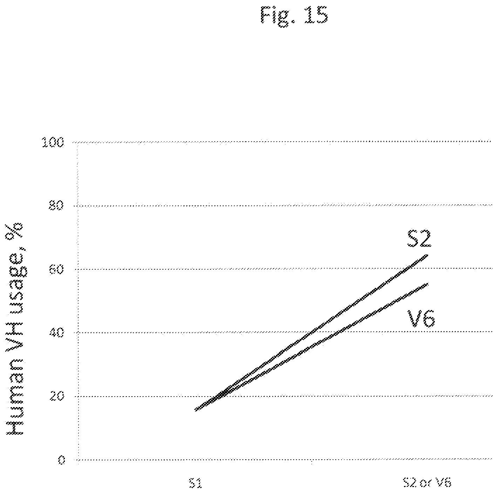

Figure 15: illustrates the improvement in human VH gene segment usage in transcripts (and thus improvement in inactivation of endogenous mouse VH gene segment usage) as human heavy chain locus DNA is inserted in successive BACs for the S1 chimaeric heavy chain locus (human VDJ gene segments from BAC1 only have been inserted) versus the S2 chimaeric heavy chain locus (human VDJ gene segments from BACs1 & 2 have been inserted) and V6 chimaeric heavy chain locus ((human VDJ gene segments from BACs 1 & 6 have been inserted)). BAC1 inserts 6 human VH and all functional human DH and JH gene segments between the 3' endogenous JH (mouse JH4) and the mouse C-mu. BAC2 inserts an additional 5 human VH gene segments 5' to the human DNA inserted from the BAC1, resulting in 11 human VH and all functional human DH and JH between the 3' endogenous JH4 and the mouse C-mu. BAC6 adds 8 human VH gene segments 5' to the human DNA inserted from BAC1, resulting in 14 human VH and all functional human DH and JH between the 3' endogenous JH4 and the mouse C-mu. With each insertion, the endogenous mouse heavy chain VDJ region is pushed further upstream (5') and inactivation is enhanced.
   BAC6 human gene segments: VH3-66, 3-64, 4-61, 4-59, 1-58, 3-53, 5-51 and 3-49.

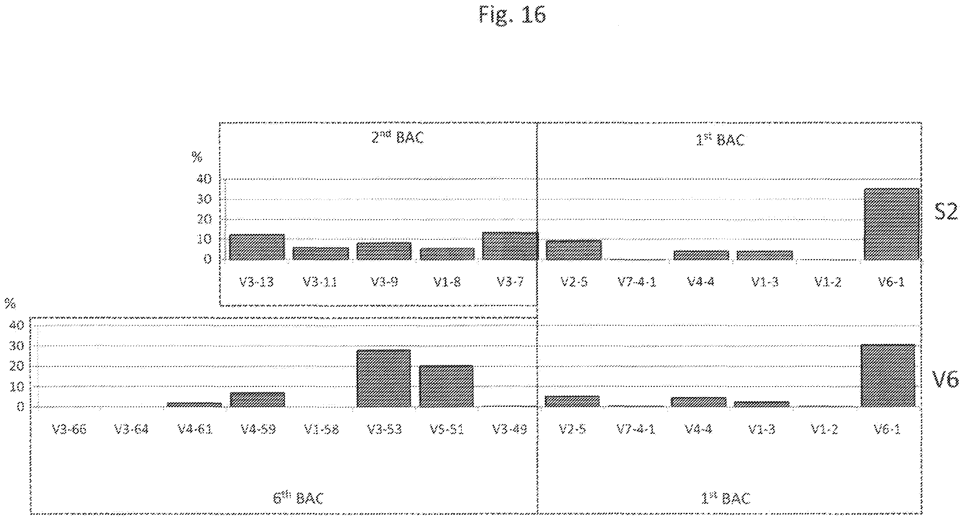

Figure 16: compares the effect of sectoring a 19 human VH gene segment repertoire across two different mice-types (corresponding to insertion of human gene segment DNA from BACs1+2 (S2 genotype) and BACs1+6 (V6 genotype). Illustrated is the distribution of human VH usage at the transcript level from the mice. Different VH usage was seen resulting in mice that produced different heavy chain repertoires (and corresponding different human heavy chain variable region repertoires) as a result of the human gene repertoire sectoring.

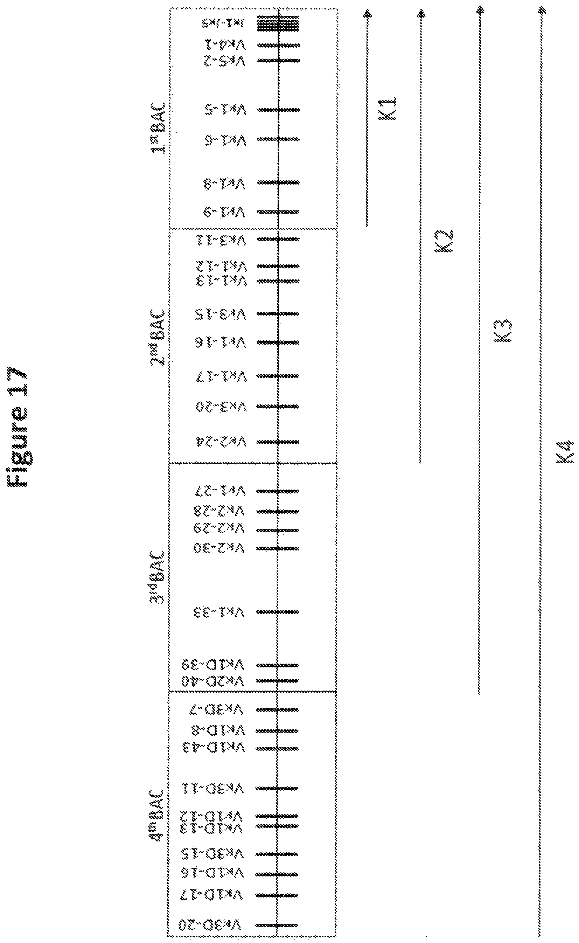

Figure 17: shows the human gene segment repertoires contained in the first, second, third and fourth BACs used to construct four different mice lines, K1, K2, K3 and K4:-
   K4: comprises an endogenous Ig kappa locus in which has been inserted the human gene segments Vκ3D-40 to 3D-7, Vκ2D-40, Vκ1D-39 and Vκ1-33 to Jκ5.

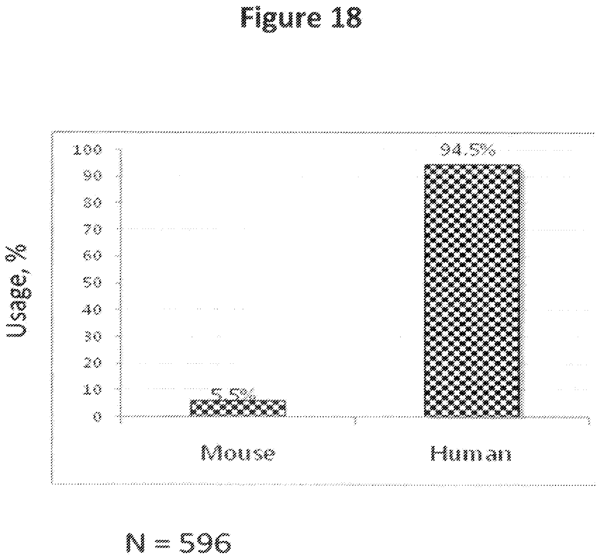

Figure 18: shows total human Vκ gene segment usage versus mouse Vκ usage in transcripts from K4 mice.

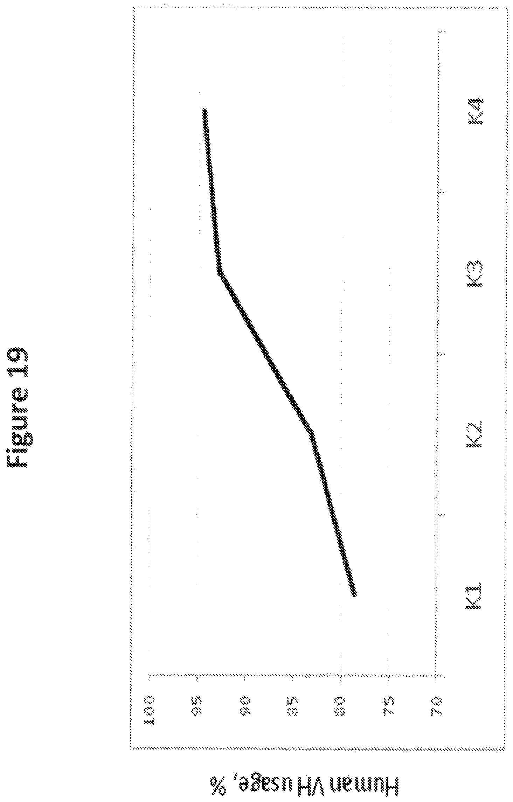

Figure 19: illustrates the improvement in human Vκ gene segment usage in transcripts (and thus improvement in inactivation of endogenous mouse Vκ gene segment usage) as human kappa locus DNA is inserted in successive BACs 1-4.

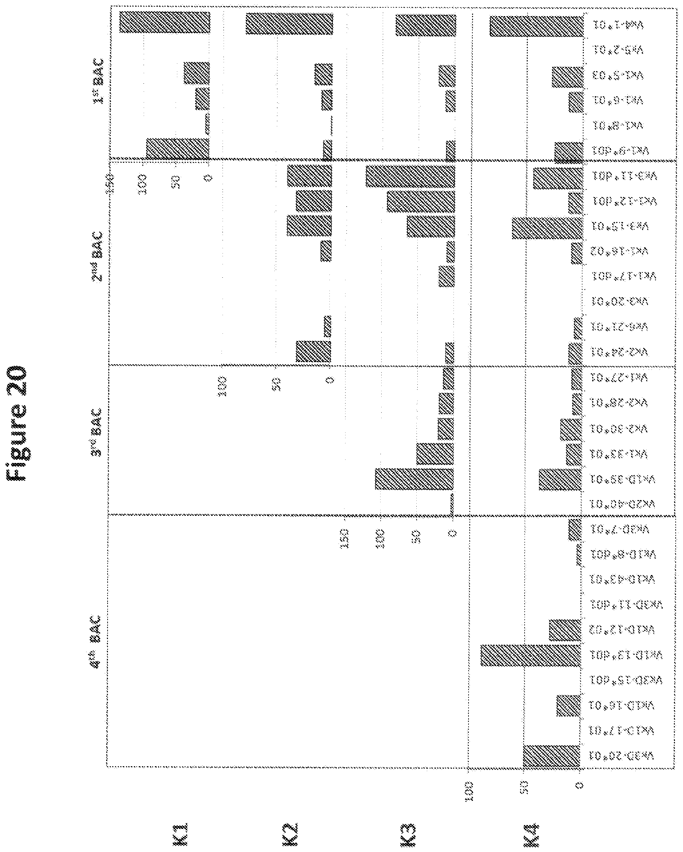

Figure 20: compares the effect of sectoring a 6, 14, 20 and 30 human Vκ gene segment repertoire across four different mice-types (corresponding to insertion of human gene segment DNA from BAC1 only (K1 genotype), BACs1+2 (K2 genotype), BACs1+2+3 (K3 genotype) and BACs1+2+3+4 (K4 genotype). Illustrated is the distribution of human Vκ usage at the transcript level from the mice. Different Vκ usage was seen resulting in mice that produced different kappa chain repertoires (and corresponding different human kappa variable region repertoires) as a result of the human gene repertoire sectoring. The nucleotide sequence of the Vκ variants mentioned are known in the art, eg, known from the IMGT database mentioned herein or 1000 Genomes database (release 1, version 3, 16 March 2012), or are disclosed in the Sequence Listing herein (for variants labelled *d01).

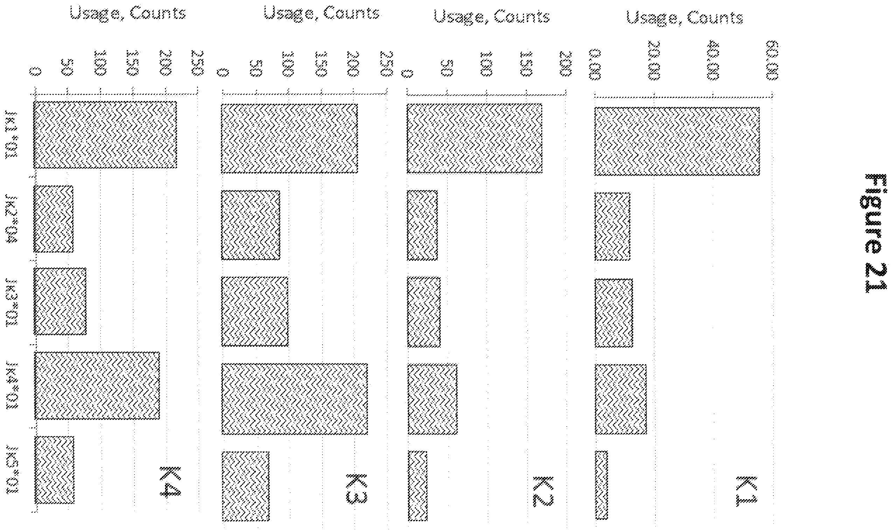

Figure 21: compares the effect of sectoring the human Jκ gene segment repertoire across four different mice-types (K1, K2, K3 and K4). Illustrated is the distribution of human Jκ usage at the transcript level from the mice. Different Jκ usage was seen resulting in mice that produced different kappa chain repertoires (and corresponding different human kappa variable region repertoires) as a result of the human gene repertoire sectoring.

In some of the figures (eg, figure 20), specific human gene segment variants (alleles) are disclosed as having been used to construct the vertebrates exemplifying the aspects of the invention (eg, Vκ2D-40*01, nomenclature being according to IMGT). Also, in embodiments of the invention specific gene segments recited can be provided as one, more or all of the specific variants shown in the figures. Use of such variants is desirable, since they are naturally-occurring in humans and thus are useful for generating Ig loci, cells and vertebrates for generating Ig chains, antibody variable domains and antibodies for use in human medicine.

### DETAILED DESCRIPTION OF THE INVENTION

The present invention allows one to dedicate the B-cell compartment of individual vertebrates within the population to a gene segment sub-repertoire from one sector of the overall desired repertoire to be accessed. For example, the invention in one embodiment enables the skilled person to restrict individual vertebrates in the population to one type of light chain (lambda or kappa) predominantly or exclusively, thereby biasing the antibody repertoire within that individual vertebrate to (predominantly) lambda- or kappa-type antibodies. Thus, on an individual vertebrate basis, the finite capacity of the B-cell compartment (the accessible compartment being up to around 2x10⁸ B-cells in a mouse) is controlled artificially to produce antibodies of substantially only the lambda or kappa type in this example. This is important because the potential repertoire encoded by lambda and kappa light chain V and J gene segments totals much more than 2x10⁸. When taken as a whole, however, a population of such vertebrates (ie, a combination of lambda-biased vertebrates with kappa-biased vertebrates) provides for an overall repertoire of antibodies that is not seen in nature (where the B-cell compartments in the prior art vertebrate collections do not have the synthetic restrictions imposed by the present invention). This repertoire according to the present example comprises a sub-repertoire of lambda-type antibodies from vertebrates biased to lambda chain expression as well as a second sub-repertoire of kappa-type antibodies from vertebrates biased to kappa chain expression. Such an approach is quite different from the approach in the art that is directed to providing mice expressing only kappa-type antibodies (perhaps with very low-level lambda-type antibodies at the natural range of 5% in a mouse). Use of such mice ignores the desirability to much more fully explore lambda gene diversity when expressing antibodies. In some prior art examples, transgenic heavy, lambda and kappa chain loci in a single mouse is discussed (eg, see WO02/066630 (Regeneron)), but again here the ratio of lambda:kappa chains is not manipulated to more fully explore the potential lambda as well as kappa diversity. In such mice, diversity is limited by the size of the accessible B-cell compartment so that it is not possible to sample more of the potential VJ light chain repertoire (which is greater than the approximately 2x10⁸ accessible B-cells available in a mouse to express antibodies). Thus, these prior art mice are used in methods that sample only a very small amount of the large potential diversity encoded by recombination of the lambda and kappa V and J gene segments. See the examples below for an illustration.

As will be readily apparent to the skilled person, the operable connection of a gene segment (eg, a V or J gene segment) upstream of a constant region in an Ig locus in any configuration of the invention enables the gene segment to be recombined and expressed in an immunoglobulin chain comprising sequence encoded by the constant region of the locus.

By "divided" in connection with dividing gene segment repertoires between vertebrates of the population, it is meant that the gene segment repertoire is present in the population as a whole, but the genomes of first and second individual members of the population (ie, individual vertebrates) have different collections of gene segments (different "sub-repertoires"), for example, different collections of human VH gene segments. The sub-repertoires can comprise common human gene segments from the overall repertoire (ie, overlapping sub-repertoires) or comprise no common gene segments from the repertoire (ie, non-overlapping sub-repertoires).

In an example, the antibody heavy chain sequence repertoire disclosed herein is a repertoire of RNA sequences (eg, mRNA) each comprising a sequence derived from the recombination of a human VH gene segment with a D and JH gene segment and a constant region (eg, a C-mu gene segment or Cgamma gene segment). In an example, the antibody heavy chain sequence repertoire is a repertoire of RNA sequences (eg, mRNA) each encoding an antibody heavy chain. In an example, the antibody heavy chain sequence repertoire is a repertoire of antibody heavy chains (eg, provided as part of antibodies). Similarly, an antibody light chain sequence repertoire can be a repertoire of RNA sequences (eg, mRNA) each comprising a sequence derived from the recombination of a human VL gene segment (eg, Vλ or Vκ) with a JL gene segment (eg, Jλ or Jκ respectively) and a constant region (eg, a C-kappa gene segment or C-lambda gene segment). In an example, the antibody light chain sequence repertoire is a repertoire of RNA sequences (eg, mRNA) each encoding an antibody light chain. In an example, the antibody light chain sequence repertoire is a repertoire of antibody light chains (eg, provided as part of antibodies).

The term "antibody" includes monoclonal antibodies (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., dAb, Fab, F(ab')2, and Fv). The term "antibody" also includes H2 antibodies that comprise a dimer of a heavy chain (5'- VH-(optional Hinge)-CH2-CH3-3') and are devoid of a light chain (akin to naturally-occurring H2 antibodies; see, eg, Nature. 1993 Jun 3;363(6428):446-8; Naturally occurring antibodies devoid of light chains; Hamers-Casterman C, Atarhouch T, Muyldermans S, Robinson G, Hamers C, Songa EB, Bendahman N, Hamers R). Thus, in an embodiment of the present invention, RNA produced from the transgenic heavy chain locus encodes for heavy chains that re devoid of a CH1 gene segment and comprise no functional antibody light chain. In an example, RNA produced from the transgenic heavy chain locus encodes for VH single variable domains (dAbs; domain antibodies). These can optionally comprise a constant region.

Examples of antibodies are classic 4-chain antibodies comprising two heavy chains paired with two light chains (such as, a dimer of 5'- VH-CH1-Hinge-CH2-CH3-3' paired with 5'-VL-CL-3') or H2 antibodies that comprise a dimer of a heavy chain (5'- VH-(optional Hinge)-CH2-CH3-3') and are devoid of a light chain Thus, in an embodiment of the present invention, the heavy chain sequence repertoire encodes for heavy chains that re devoid of a CH1 gene segment and comprise no functional antibody light chain. In an example, the heavy chain sequence repertoire encodes a repertoire of VH single variable domains (dAbs; domain antibodies). These can optionally comprise a constant region.

In an example of any configuration of the invention, a repertoire comprises a plurality of different members (thus, for example, a heavy chain sequence repertoire comprises a plurality of different heavy chain sequences, such as sequences differing in their variable regions). In an example of any configuration of the invention, a repertoire comprises or consists of 2, 3, 4, 5, 6, 7, 8, 9, 10, at least 15, at least 20, at least 30, at least 40, at least 50, at least 60, at least 70, at least 80, at least 90, at least 100, at least 10³, at least 10⁴, at least 10⁵, at least 10⁶, at least 10⁷, at least 10⁸, at least 10⁹, at least 10¹⁰, at least 10¹¹, at least 10¹², at least 10¹³, or at least 10¹⁴ members. For example, a repertoire of antibody chain sequences or antibodies comprises or consists of at least 100, at least 10³, at least 10⁴, at least 10⁵, at least 10⁶, at least 10⁷, at least 10⁸, at least 10⁹, at least 10¹⁰, at least 10¹¹, at least 10¹², at least 10¹³, or at least 10¹⁴ antibody chain sequences or antibodies respectively. For example, a repertoire comprises or consists of 2, 3, 4, 5, 6, 7, 8, 9, 10, at least 15, at least 20, at least 30, at least 40, at least 50, at least 60, at least 70, at least 80, at least 90, at least 100, at least 10³, at least 10⁴, at least 10⁵, at least 10⁶, at least 10⁷, at least 10⁸, at least 10⁹, at least 10¹⁰, at least 10¹¹, at least 10¹², at least 10¹³, or at least 10¹⁴ different members. For example, a repertoire of gene segments (eg, human VH gene segments or VL gene segments or Vλ gene segments or Vκ gene segments) comprises or consists of 2, 3, 4, 5, 6, 7, 8, 9, 10, at least 15, at least 20, at least 30, at least 40, at least 50 gene segments. Optionally, all of the gene segments are different from each other.

A repertoire of human gene segments used in the invention, in one example, comprises additionally non-human gene segments, eg, non-human vertebrate gene segments and/or synthetic gene segments. Such combinations of gene segments is desirable to enhance possible variable region diversity.

In an example of any configuration of the invention, a population comprises a plurality of different members. Thus, for example, a population of transgenic non-human vertebrates (eg, mice or rats) comprises a plurality of vertebrates wherein at least two or more of the vertebrates comprise non-identical genomes. The genomes can, for example, differ in their respective repertoire of human gene segments (eg, human VH gene segments). In an example of any configuration of the invention, a population of non-human vertebrates comprises or consists of 2, 3, 4, 5, 6, 7, 8, 9, 10, at least 15, at least 20, at least 30, at least 40, at least 50, at least 60, at least 70, at least 80, at least 90, at least 100, at least 10³ non-human vertebrates. When the population consists of two vertebrates, the genomes of vertebrates are different, eg, the genomes comprise different repertoires of human heavy chain and/or light chain locus gene segments, eg, VH or VL gene segments; or eg, a first of the genomes comprises human lambda locus gene segments (eg, a repertoire of human Vλ and/or Jλ) and (substantially) no human kappa locus gene segments and a second of the genomes comprises human kappa locus gene segments (eg, a repertoire of human Vκ and/or Jκ) and optionally (substantially) no human lambda locus gene segments).

In the invention, each vertebrate is a mouse or rat For example, all vertebrates are of the same vertebrate species, eg, all mice or all rats.

In any configuration of the invention, the inserted human genes may be derived from the same individual or different individuals, or be synthetic or represent human consensus sequences.

Techniques for constructing non-human vertebrates and vertebrate cells whose genomes comprise a transgene containing human V, J and D regions are well known in the art. For example, reference is made to WO2011004192, US7501552, US6673986, US6130364, WO2009076464 and US6586251.

In one aspect the human heavy chain gene segments are inserted into the genome so that they are placed under control of the host regulatory sequences (eg, enhancers, promoters and/or switches) or other (non-human, non-host) sequences. In one aspect reference to human coding regions includes both human introns and exons, or in another aspect simply exons and no introns, which may be in the form of cDNA.

Alternatively it is possible to use recombineering in ES cells, or other recombinant DNA technologies, to insert a non human-vertebrate (e.g. mouse) promoter or other control region, such as a promoter for a V region, into a BAC containing a human Ig region. The recombineering step then places a portion of human DNA under control of the mouse promoter or other control region.

In one aspect a (or each) non-human vertebrate of any configuration of the invention is able to generate a diversity of at least 1 X 10⁶ different functional chimaeric immunoglobulin sequence combinations.

Optionally in any configuration of the invention each constant region is endogenous to the vertebrate and optionally comprises an endogenous switch (when the constant region is a heavy chain constant region). In one embodiment, the constant region comprises a Cgamma (CΥ) gene segment (eg, a human Cgamma) and/or a Smu (Sµ) switch. Switch sequences are known in the art, for example, see Nikaido et al, Nature 292: 845-848 (1981) and also WO2011004192, US7501552, US6673986, US6130364, WO2009/076464 and US6586251, eg, SEQ ID NOs: 9-24 disclosed in US7501552. Optionally the constant region comprises an endogenous S gamma switch and/or an endogenous Smu switch. One or more endogenous switch regions can be provided, in one embodiment, by constructing a transgenic immunoglobulin locus in the vertebrate or cell genome in which at least one human V region, at least one human J region, and optionally at least one human D region, or a rearranged VDJ or VJ region, are inserted into the genome in operable connection with a constant region that is endogenous to the vertebrate or cell. For example, the human V(D)J regions or rearranged VDJ or VJ can be inserted in a cis orientation onto the same chromosome as the endogenous constant region. A *trans* orientation is also possible, in which the human V(D)J regions or rearranged VDJ or VJ are inserted into one chromosome of a pair (eg, the chromosome 6 pair in a mouse or the chromosome 4 in a rat) and the endogenous constant region is on the other chromosome of the pair, such that trans-switching takes place in which the human V(D)J regions or rearranged VDJ or VJ are spliced inoperable linkage to the endogenous constant region. In this way, the vertebrate can express antibodies having a chain that comprises a variable region encoded all or in part by human V(D)J or a rearranged VDJ or VJ, together with a constant region (eg, a Cgamma or Cmu) that is endogenous to the vertebrate.

Human variable regions are suitably inserted upstream of a non-human vertebrate constant region, the latter comprising all of the DNA required to encode the full constant region or a sufficient portion of the constant region to allow the formation of an effective chimaeric antibody capable of specifically recognising an antigen.

In one aspect the chimaeric antibodies or antibody chains isolated from vertebrates disclosed herein have a part of a host constant region sufficient to provide one or more effector functions seen in antibodies occurring naturally in a host vertebrate, for example that they are able interact with Fc receptors, and/or bind to complement.

Reference to a chimaeric antibody or antibody chain having a non-human vertebrate constant region herein therefore is not limited to the complete constant region but also includes chimaeric antibodies or chains which have all of the host constant region, or a part thereof sufficient to provide one or more effector functions. This also applies to non-human vertebrates used in the invention in which human variable region DNA may be inserted into the host genome such that it forms a chimaeric antibody chain with all or part of a host constant region. In one aspect the whole of a host non-human vertebrate constant region is operably linked to human variable region DNA.

The host non-human vertebrate constant region herein is optionally the endogenous host wild-type constant region located at the wild type locus, as appropriate for the heavy or light chain. For example, the human heavy chain DNA is suitably inserted on mouse chromosome 12, suitably adjacent the mouse heavy chain constant region, where the vertebrate is a mouse.

In one optional aspect where the vertebrate is a mouse, the insertion of human DNA, such as a human VDJ region is targeted to the region between the J4 exon and the Cµ locus in the mouse genome IgH locus, and in one aspect is inserted between coordinates 114,667,090 and 114,665,190, suitably at coordinate 114,667,091, after 114,667,090. In one aspect the insertion of a human DNA, such as a human light chain kappa VJ region is targeted into mouse chromosome 6 between coordinates 70,673,899 and 70,675,515, suitably at position 70,674,734, or an equivalent position in the lambda mouse locus on chromosome 16.

All nucleotide co-ordinates for the mouse are those corresponding to NCBI m37 for the mouse C57BL/6J strain, e.g. April 2007 ENSEMBL Release 55.37h, e.g. NCBI37 July 2007 (NCBI build 37) (e.g. UCSC version mm9 see www.genome.ucsc.edu and http://genome.ucsc.edu/FAQ/FAQreleases.html) unless otherwise specified. Human nucleotides coordinates are those corresponding to GRCh37 (e.g. UCSC version hg 19, http://genome.ucsc.edu/FAQ/FAQreleases.html), Feb 2009 ENSEMBL Release 55.or are those corresponding to NCBI36, Ensemble release 54 unless otherwise specified. Rat nucleotides are those corresponding to RGSC 3.4 Dec 2004 ENSEMBL release 55.34w, or Baylor College of Medicine HGSC v3.4 Nov 2004(e.g., UCSC rn4, see www.genome.ucsc.edu and http://genome.ucsc.edu/FAQ/FAQreleases.html) unless otherwise specified.

In one aspect the host non-human vertebrate constant region for forming the chimaeric antibody may be at a different (non endogenous) chromosomal locus. In this case the inserted human DNA, such as the human variable VDJ or VJ region(s) may then be inserted into the non-human genome at a site which is distinct from that of the naturally occurring heavy or light constant region. The native constant region may be inserted into the genome, or duplicated within the genome, at a different chromosomal locus to the native position, such that it is in a functional arrangement with the human variable region such that chimaeric antibodies disclosed herein can still be produced.

In one aspect the human DNA is inserted at the endogenous host wild-type constant region located at the wild type locus between the host constant region and the host VDJ region.

Reference to location of the variable region or a gene segment upstream of a constant region means that there is a suitable relative location of the two antibody portions, variable/gene segment and constant, to allow the portions to form a chimaeric antibody or antibody chain *in vivo* in the vertebrate. Thus, the inserted human DNA and host constant region are in operable connection with one another for antibody or antibody chain production.

In one aspect the inserted human DNA is capable of being expressed with different host constant regions through isotype switching. In one aspect isotype switching does not require or involve trans switching. Insertion of the human variable region DNA on the same chromosome as the relevant host constant region means that there is no need for trans-switching to produce isotype switching.

In the present invention, optionally host non-human vertebrate constant regions are maintained and in an example at least one non-human vertebrate enhancer or other control sequence, such as a switch region, is maintained in functional arrangement with the non-human vertebrate constant region, such that the effect of the enhancer or other control sequence, as seen in the host vertebrate, is exerted in whole or in part in the transgenic animal. This approach is designed to allow the full diversity of the human locus to be sampled, to allow the same high expression levels that would be achieved by non-human vertebrate control sequences such as enhancers, and is such that signalling in the B-cell, for example isotype switching using switch recombination sites, would still use non-human vertebrate sequences.

A non-human vertebrate having such a genome would produce chimaeric antibodies with human variable and non-human vertebrate constant regions, but these are readily humanized, for example in a cloning step.

For example, V (and/or other gene segments) which are naturally inverted in a human genome or are pseudogenes may be omitted from the repertoire provided by the population of the invention.

Reference to "functional" human gene segments in any configuration of the invention acknowledges that in a human Ig locus some V or other gene segments are non-functional pseudogenes (eg, Vλ3-17, Vλ3-15, Vλ3-13, Vλ3-7, Vλ3-6, Vλ2-5, Vλ3-4, Vλ3-2; see the IMGT database: http://www.imgt.org/IMGTrepertoire/index.php?section=LocusGenes&repertoire=locus&species=h uman&group=IGL). Also, Jλ4-Cλ4 and Jλ5-Cλ5 are not functional in humans. The term "functional" when referring to gene segments excludes pseudogenes. An example of functional human Vλ gene segments is the group Vλ2-18, Vλ3-16, V2-14, Vλ3-12, Vλ2-11, Vλ3-10, Vλ3-9, Vλ2-8, Vλ4-3 and Vλ3-1. An example of functional human Jλ gene segments is the group Jλ1, Jλ2 and Jλ3; or Jλ1, Jλ2 and Jλ7; or Jλ2, Jλ3 and Jλ7; or Jλ1, Jλ2, Jλ3 and Jλ7. An example of functional human Cλ gene segments is the group Cλ1, Cλ2 and Cλ3; or Cλ1, Cλ2 and Cλ7; or Cλ2, Cλ3 and Cλ7; or Cλ1, Cλ2, Cλ3 and Cλ7.

The term "germline configuration" refers to a germline genomic configuration. For example, human immunoglobulin gene segments of a transgenic immunoglobulin locus are in a germline configuration when the relative order of the gene segments is the same as the order of corresponding gene segments in a human germline genome. For example, when the transgenic locus is a heavy chain locus disclosed herein comprising hypothetical human immunoglobulin gene segments A, B and C, these would be provided in this order (5' to 3' in the locus) when the corresponding gene segments of a human germline genome comprises the arrangement 5'-A-B-C-3'. In an example, when elements of a human immunoglobulin locus (eg, gene segments, enhancers or other regulatory elements) are provided in a transgenic immunoglobulin locus disclosed herein, the human Ig locus elements are in germline configuration when the relative order of the gene segments is the same as the order of corresponding gene segments in a human germline genome and human sequences between the elements are included, these corresponding to such sequences between corresponding elements in the human germline genome. Thus, in a hypothetical example the transgenic locus comprises human elements in the arrangement 5'-A-S1-B-S2-C-S3-3', wherein A, B and C are human immunoglobulin gene segments and S1-S3 are human inter-gene segment sequences, wherein the corresponding arrangement 5'-A-S1-B-S2-C-S3-3' is present in a human germline genome. For example, this can be achieved by providing in a transgenic immunoglobulin locus disclosed herein a DNA insert corresponding to the DNA sequence from A to C in a human germline genome (or the insert comprising the DNA sequence from A to C). The arrangements in human germline genomes and immunoglobulin loci are known in the art (eg, see the IMGT, Kabat and other antibody resources).

As a source of antibody gene segment sequences, the skilled person will also be aware of the following available databases and resources (including updates thereof):
The Kabat Database (G. Johnson and T. T.Wu, 2002; World Wide Web (www) kabatdatabase.com). Created by E. A. Kabat and T. T. Wu in 1966, the Kabat database publishes aligned sequences of antibodies, T-cell receptors, major histocompatibility complex (MHC) class I and II molecules, and other proteins of immunological interest. A searchable interface is provided by the Seqhuntll tool, and a range of utilities is available for sequence alignment, sequence subgroup classification, and the generation of variability plots. See also Kabat, E. A.,Wu, T. T., Perry, H., Gottesman, K., and Foeller, C. (1991) Sequences of Proteins of Immunological Interest, 5th ed., NIH Publication No. 91-3242, Bethesda, MD, in particular with reference to human gene segments for use in the present invention.

KabatMan (A. C. R. Martin, 2002; World Wide Web (www) bioinf.org.uk/abs/simkab.html). This is a web interface to make simple queries to the Kabat sequence database.

***IMGT*** (the International ImMunoGeneTics Information System®; M.-P. Lefranc, 2002; World Wide Web (*www*) imgt.cines.fr). IMGT is an integrated information system that specializes in antibodies, T cell receptors, and MHC molecules of all vertebrate species. It provides a common portal to standardized data that include nucleotide and protein sequences, oligonucleotide primers, gene maps, genetic polymorphisms, specificities, and two-dimensional (2D) and three-dimensional (3D) structures. IMGT includes three sequence databases *(IMGT*/*LIGM-DB, IMGT*/*MHC-DB, IMGT*/*PRIMERDB),* one genome database *(IMGT*/*GENE-DB),* one 3D structure database *(IMGT*/*3Dstructure-DB),* and a range of web resources ("*IMGT* Marie-Paule page") and interactive tools.

V-BASE (I. M. Tomlinson, 2002; World Wide Web (www) mrc-cpe.cam.ac.uk/vbase). V-BASE is a comprehensive directory of all human antibody germline variable region sequences compiled from more than one thousand published sequences. It includes a version of the alignment software DNAPLOT (developed by Hans-Helmar Althaus and Werner Müller) that allows the assignment of rearranged antibody V genes to their closest germline gene segments.

Antibodies-Structure and Sequence (A. C. R. Martin, 2002; World Wide Web (www) bioinf.org.uk/abs). This page summarizes useful information on antibody structure and sequence. It provides a query interface to the Kabat antibody sequence data, general information on antibodies, crystal structures, and links to other antibody-related information. It also distributes an automated summary of all antibody structures deposited in the Protein Databank (PDB). Of particular interest is a thorough description and comparison of the various numbering schemes for antibody variable regions.

AAAAA (A Ho's Amazing Atlas of Antibody Anatomy; A. Honegger, 2001; World Wide Web (www) unizh.ch/∼antibody). This resource includes tools for structural analysis, modelling, and engineering. It adopts a unifying scheme for comprehensive structural alignment of antibody and T-cell-receptor sequences, and includes Excel macros for antibody analysis and graphical representation.

***WAM*** (Web Antibody Modeling; N. Whitelegg and A. R. Rees, 2001; World Wide Web (www) antibody.bath.ac.uk). Hosted by the Centre for Protein Analysis and Design at the University of Bath, United Kingdom. Based on the AbM package (formerly marketed by Oxford Molecular) to construct 3D models of antibody Fv sequences using a combination of established theoretical methods, this site also includes the latest antibody structural information.

Mike's Immunoglobulin Structure/Function Page (M. R. Clark, 2001; World Wide Web (www) path.cam.ac.uk/~mrc7/mikeimages.html*)* These pages provide educational materials on immunoglobulin structure and function, and are illustrated by many colour images, models, and animations. Additional information is available on antibody humanization and Mike Clark's Therapeutic Antibody Human Homology Project, which aims to correlate clinical efficacy and anti-immunoglobulin responses with variable region sequences of therapeutic antibodies.

The Antibody Resource Page (The Antibody Resource Page, 2000; World Wide Web (www) antibodyresource.com). This site describes itself as the "complete guide to antibody research and suppliers." Links to amino acid sequencing tools, nucleotide antibody sequencing tools, and hybridoma/cell-culture databases are provided.

Humanization bY Design (J. Saldanha, 2000; World Wide Web (www) people.cryst.bbk.ac.uk/~ubcg07s). This resource provides an overview on antibody humanization technology. The most useful feature is a searchable database (by sequence and text) of more than 40 published humanized antibodies including information on design issues, framework choice, framework back-mutations, and binding affinity of the humanized constructs.

See also Antibody Engineering Methods and Protocols, Ed. Benny K C Lo, Methods in Molecular Biology™, Human Press. Also at World Wide Web (*www*) blogsua.com/pdf/antibody-engineering-methods-and-protocolsantibody-engineering-methods-and-protocols.pdf

Samples from which B-cells can be obtained include but are not limited to blood, serum, spleen, splenic tissue, bone marrow, lymph, lymph node, thymus, and appendix. Antibodies and immunoglobulin chains can be obtained from each of the previous-mentioned samples and also from the following non-limiting list of B-cells, ascites fluid, hybridomas, and cell cultures.

"Plurality" is used in the ordinary sense of the term and means "at least one" or "more than one".

"Derived from" is used in the ordinary sense of the term. Exemplary synonyms include "produced as", "resulting from", "received from", "obtained from", "a product of", "consequence of", and "modified from" For example, a human variable region of a heavy chain can be derived from recombination of human VH, D and JH gene segments and this reflects the *in vivo* recombination of these gene segments in, for example, a transgenic heavy chain locus disclosed herein with any accompanying mutation (eg, junctional mutation).

In one embodiment in any configuration of the disclosure, the genome of a or each vertebrate has been modified to prevent or reduce the expression of fully-endogenous antibody. Examples of suitable techniques for doing this can be found in WO2011004192, US7501552, US6673986, US6130364, WO2009/076464, EP1399559 and US6586251. In one embodiment, the non-human vertebrate VDJ region of the endogenous heavy chain immunoglobulin locus, and optionally VJ region of the endogenous light chain immunoglobulin loci (lambda and/or kappa loci), have been inactivated. For example, all or part of the non-human vertebrate VDJ region is inactivated by inversion in the endogenous heavy chain immunoglobulin locus of the mammal, optionally with the inverted region being moved upstream or downstream of the endogenous Ig locus. For example, all or part of the non-human vertebrate VJ region is inactivated by inversion in the endogenous kappa chain immunoglobulin locus of the mammal, optionally with the inverted region being moved upstream or downstream of the endogenous Ig locus. For example, all or part of the non-human vertebrate VJ region is inactivated by inversion in the endogenous lambda chain immunoglobulin locus of the mammal, optionally with the inverted region being moved upstream or downstream of the endogenous Ig locus. In one embodiment the endogenous heavy chain locus is inactivated in this way as is one or both of the endogenous kappa and lambda loci.

Additionally or alternatively, the or each vertebrate has been generated in a genetic background which prevents the production of mature host B and T lymphocytes, optionally a RAG-1-deficient and/or RAG-2 deficient background. See US5859301 for techniques of generating RAG-1 deficient animals.

In an embodiment, the immunoglobulin loci of the vertebrates differ only in the repertoire of said human gene segments.

The term "immunoglobulin" (Ig) is used interchangeably with "antibody" herein.

An "isolated" antibody is one that has been identified, separated and/or recovered from a component of its production environment (e.g., naturally or recombinantly). Preferably, the isolated polypeptide is free of association with all other components from its production environment, eg, so that the antibody has been isolated to an FDA-approvable or approved standard. Contaminant components of its production environment, such as that resulting from recombinant transfected cells, are materials that would typically interfere with research, diagnostic or therapeutic uses for the antibody, and may include enzymes, hormones, and other proteinaceous or non-proteinaceous solutes. In preferred embodiments, the polypeptide will be purified: (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of a spinning cup sequenator, or (3) to homogeneity by SDS-PAGE under non-reducing or reducing conditions using Coomassie blue or, preferably, silver stain. Isolated antibody includes the antibody in situ within recombinant cells since at least one component of the antibody's natural environment will not be present. Ordinarily, however, an isolated polypeptide or antibody will be prepared by at least one purification step.

An "antibody fragment" comprises a portion of an intact antibody, preferably the antigen binding and/or the variable region of the intact antibody. Examples of antibody fragments include dAb, Fab, Fab', F(ab')2 and Fv fragments; diabodies; linear antibodies; single-chain antibody molecules and multispecific antibodies formed from antibody fragments.

An antibody that "specifically binds to" or is "specific for" a particular polypeptide, antigen, or epitope is one that binds to that particular polypeptide, antigen, or epitope without substantially binding to other polypeptides, antigens or epitopes. For example, binding to the antigen or epitope is specific when the antibody binds with a K_{D} of 100 µM or less, 10 µM or less, 1 µM or less, 100 nM or less, eg, 10 nM or less, 1 nM or less, 500 pM or less, 100 pM or less, or 10pM or less. The binding affinity (K_{D}) can be determined using standard procedures as will be known by the skilled person, eg, binding in ELISA and/or affinity determination using surface plasmon resonance (eg, Biacore™ or KinExA™ solution phase affinity measurement which can detect down to fM affinities (Sapidyne Instruments, Idaho)).

"Pharmaceutically acceptable" refers to approved or approvable by a regulatory agency of the USA Federal or a state government or listed in the U.S. Pharmacopeia or other generally recognized pharmacopeia for use in animals, including humans. A "pharmaceutically acceptable carrier, excipient, or adjuvant" refers to an carrier, excipient, or adjuvant that can be administered to a subject, together with an agent, e.g., any antibody or antibody chain described herein, and which does not destroy the pharmacological activity thereof and is nontoxic when administered in doses sufficient to deliver a therapeutic amount of the agent.

In one embodiment of any configuration of the invention when the vertebrate is a mouse, (i) the constant region comprises a mouse or rat Sµ switch and optionally a mouse Cµ region. For example the constant region is provided by the constant region endogenous to the mouse, eg, by inserting human V(D)J region sequences into operable linkage with the endogenous constant region of a mouse genome or mouse cell genome.

In one embodiment of any configuration of the invention when the vertebrate is a rat, (i) the constant region comprises a mouse or rat Sµ switch and optionally a rat Cµ region. For example the constant region is provided by the constant region endogenous to the rat, eg, by inserting human V(D)J region sequences into operable linkage with the endogenous constant region of a rat genome or rat cell genome.

In one embodiment of any configuration of the vertebrate disclosed herein (excluding a kappa vertebrate) the genome comprises an antibody light chain transgene which comprises all or part of the human Igλ locus including at least one human Jλ region and at least one human Cλ region, optionally C_{λ}6 and/or C_{λ}7. Optionally, the transgene comprises a plurality of human Jλ regions, optionally two or more of J_{λ}1, J_{λ}2, J_{λ}6 and J_{λ}7, optionally all of J_{λ}1, J_{λ}2, J_{λ}6 and J_{λ}7. The human lambda immunoglobulin locus comprises a unique gene architecture composed of serial J-C clusters. In order to take advantage of this feature, the invention in optional aspects employs one or more such human J-C clusters inoperable linkage with the constant region in the transgene, eg, where the constant region is endogenous to the non-human vertebrate or non-human vertebrate cell. Thus, optionally the transgene comprises at least one human J_{λ}-C_{λ} cluster, optionally at least J_{λ}7-C_{λ}7. The construction of such transgenes is facilitated by being able to use all or part of the human lambda locus such that the transgene comprises one or more J-C clusters in germline configuration, advantageously also including intervening sequences between clusters and/or between adjacent J and C regions in the human locus. This preserves any regulatory elements within the intervening sequences which may be involved in VJ and/or JC recombination and which may be recognised by AID (activation-induced deaminase) or AID homologues.

Where endogenous regulatory elements are involved in CSR (class-switch recombination) in the non-human vertebrate, these can be preserved by including in the transgene a constant region that is endogenous to the non-human vertebrate. In the invention, one can match this by using an AID or AID homologue that is endogenous to the vertebrate or a functional mutant thereof. Such design elements are advantageous for maximising the enzymatic spectrum for SHM (somatic hypermutation) and/or CSR and thus for maximising the potential for antibody diversity.

Optionally, the lambda transgene comprises a human Eλ enhancer. Optionally, the kappa transgene comprises a human Eκ enhancer. Optionally, the heavy chain transgene comprises a heavy chain human enhancer.

In one embodiment of any configuration of the invention the constant region is endogenous to the non-human vertebrate or derived from such a constant region. For example, the vertebrate is a mouse and the constant region is endogenous to the mouse. For example, the vertebrate is a rat and the constant region is endogenous to the rat.

In one embodiment of any configuration of the invention the heavy chain transgene comprises a plurality human IgH V regions, a plurality of human D regions and a plurality of human J regions.

In one embodiment of any configuration of the invention, the vertebrate comprises a heavy chain further transgene, the further transgene comprising at least one human IgH V region, at least one human D region and at least one human J region.

An aspect provides a method of isolating an antibody or nucleotide sequence encoding said antibody, the method comprising
(a) immunising (see e.g. Harlow, E. & Lane, D. 1998, 5th edition, Antibodies: A Laboratory Manual, Cold Spring Harbor Lab. Press, Plainview, NY; and Pasqualini and Arap, Proceedings of the National Academy of Sciences (2004) 101:257-259) a vertebrate population according to any configuration or aspect of the invention with an antigen such that the vertebrates produce antibodies; and
(b) isolating from immunised vertebrates an antibody that specifically binds to said antigen and/or a nucleotide sequence encoding at least the heavy and/or the light chain variable regions of said antibody;
optionally wherein the variable regions of said antibody are subsequently joined to a human constant region. Such joining can be effected by techniques readily available in the art, such as using conventional recombinant DNA and RNA technology as will be apparent to the skilled person. See e.g. Sambrook, J and Russell, D. (2001, 3'd edition) Molecular Cloning: A Laboratory Manual (Cold Spring Harbor Lab. Press, Plainview, NY).

Suitably an immunogenic amount of the antigen is delivered. Disclosed herein is a method for detecting a target antigen comprising detecting an antibody produced as above with a secondary detection agent which recognises a portion of that antibody.

Isolation of the antibody in step (b) can be carried out using conventional antibody selection techniques, eg, panning for antibodies against antigen that has been immobilised on a solid support, optionally with iterative rounds at increasing stringency, as will be readily apparent to the skilled person.

As a further optional step, after step (b) the amino acid sequence of the heavy and/or the light chain variable regions of the antibody are mutated to improve affinity for binding to said antigen. Mutation can be generated by conventional techniques as will be readily apparent to the skilled person, eg, by error-prone PCR. Affinity can be determined by conventional techniques as will be readily apparent to the skilled person, eg, by surface plasmon resonance, eg, using Biacore™.

Additionally or alternatively, as a further optional step, after step (b) the amino acid sequence of the heavy and/or the light chain variable regions of the antibody are mutated to improve one or more biophysical characteristics of the antibody, eg, one or more of melting temperature, solution state (monomer or dimer), stability and expression (eg, in CHO or *E coli*).

An aspect provides an antibody produced by the method disclosed herein, optionally for use in medicine, eg, for treating and/or preventing a medical condition or disease in a patient, eg, a human.

An aspect provides a nucleotide sequence encoding the antibody disclosed herein , optionally wherein the nucleotide sequence is part of a vector. Suitable vectors will be readily apparent to the skilled person, eg, a conventional antibody expression vector comprising the nucleotide sequence together in operable linkage with one or more expression control elements.

An aspect provides a pharmaceutical composition comprising the antibody disclosed herein and a diluent, excipient or carrier, optionally wherein the composition is contained in an IV container (eg, and IV bag) or a container connected to an IV syringe.

An aspect provides the use of the antibody disclosed herein in the manufacture of a medicament for the treatment and/or prophylaxis of a disease or condition in a patient, eg a human.

In a further aspect the disclosure relates to a method for producing an antibody specific to a desired antigen the method comprising immunizing a population of non-human vertebrates as above with a predetermined antigen and recovering a chimaeric antibody (see e.g. Harlow, E. & Lane, D. 1998, 5th edition, Antibodies: A Laboratory Manual, Cold Spring Harbor Lab. Press, Plainview, NY; and Pasqualini and Arap, Proceedings of the National Academy of Sciences (2004) 101:257-259). Suitably an immunogenic amount of the antigen is delivered. The disclosure also relates to a method for detecting a target antigen comprising detecting an antibody produced as above with a secondary detection agent which recognises a portion of that antibody.

In a further aspect the disclosure relates to a method for producing a fully humanised antibody comprising immunizing a population of non-human vertebrates as above with a predetermined antigen, recovering a chimaeric antibody or cells expressing the antibody, and then replacing the non-human vertebrate constant region with a human constant region. This can be done by standard cloning techniques at the DNA level to replace the non-human vertebrate constant region with an appropriate human constant region DNA sequence - see e.g. Sambrook, J and Russell, D. (2001, 3'd edition) Molecular Cloning: A Laboratory Manual (Cold Spring Harbor Lab. Press, Plainview, NY).

In a further aspect the disclosure relates to humanised antibodies and antibody chains produced according to the present disclosure, both in chimaeric and fully humanised form, and use of said antibodies in medicine. The disclosure also relates to a pharmaceutical composition comprising such an antibody and a pharmaceutically acceptable carrier or other excipient.

Antibody chains containing human sequences, such as chimaeric human-non-human antibody chains, are considered humanised herein by virtue of the presence of the human protein coding regions region. Fully humanised antibodies may be produced starting from DNA encoding a chimaeric antibody chain disclosed herein using standard techniques.

Methods for the generation of both monoclonal and polyclonal antibodies are well known in the art, and the present disclosure relates to both polyclonal and monoclonal antibodies of chimaeric or fully humanised antibodies produced in response to antigen challenge in a population of non-human vertebrates used in the present invention.

In a yet further aspect, chimaeric antibodies or antibody chains generated using the present invention may be manipulated, suitably at the DNA level, to generate molecules with antibody-like properties or structure, such as a human variable region from a heavy chain absent a constant region, for example a domain antibody; or a human variable region with any constant region from either heavy or light chain from the same or different species; or a human variable region with a non-naturally occurring constant region; or human variable region together with any other fusion partner. The disclosure relates to all such chimaeric antibody derivatives derived from chimaeric antibodies identified using the present invention.

In a further aspect, the disclosure relates to use of a population of non-human vertebrates of the present disclosure in the analysis of the likely effects of drugs and vaccines in the context of a quasi-human antibody repertoire.

The disclosure also relates to a method for identification or validation of a drug or vaccine, the method comprising delivering the vaccine or drug to a population of vertebrates of the disclosure and monitoring one or more of: the immune response, the safety profile; the effect on disease.

The disclosure also relates to a kit comprising an antibody or antibody derivative as disclosed herein and either instructions for use of such antibody or a suitable laboratory reagent, such as a buffer, antibody detection reagent.

The disclosure also relates to a method for making an antibody, or part thereof, the method comprising providing:
(i) a nucleic acid encoding an antibody, or a part thereof, obtained using the population of the present invention; or
(ii) sequence information from which a nucleic acid encoding an antibody obtained using the population of the present invention, or part thereof, can be expressed to allow an antibody to be produced.

In an embodiment, each vertebrate of the population of the first aspect of the invention is a non-human vertebrate, mouse or rat, whose genome comprises
(a) said transgenic heavy chain locus; and
(b) an antibody kappa light chain locus transgene and/or an antibody lambda chain locus transgene;
wherein all of the V, D and J in said transgenes are human V, D and J;
wherein endogenous antibody heavy and light chain expression has been inactivated; and optionally wherein said genome is homozygous for said transgenic heavy and light chain loci.

In an embodiment, the kappa and lambda chain transgenic loci comprise constant regions of said non-human vertebrate species capable of pairing with the constant region of the heavy chain.

In an embodiment, the kappa chain transgenic loci comprises a substantially complete human functional Vκ and Jκ repertoire; and the lambda chain transgene comprises a substantially complete human functional Vλ and Jλ repertoire.

Throughout this text, and with application to any configuration, aspect, embodiment or example of the invention, the term "endogenous" (eg, endogenous constant region) in relation to a non-human vertebrate indicates that the constant region etc is a type of constant region etc that is normally found in the vertebrate (as opposed to an exogenous constant region whose sequence is not normally found in such a vertebrate, eg a human sequence). For example, the endogenous constant region can be those encoded by the wild-type genome of the non-human vertebrate. So, in an example wherein the vertebrate is a mouse, the endogenous constant region would be a mouse constant region. Going further, the endogenous regions are, in an example, strain-matched to the vertebrate. So, in one embodiment, the vertebrate cell is a mouse 129 ES cell, the endogenous constant region would be mouse 129 constant region. In another embodiment, the vertebrate is a JM8 strain mouse, the endogenous constant region would be mouse JM8 constant region. In another embodiment, the vertebrate is a Black 6 mouse, the endogenous constant region would be mouse Black 6 constant region.

In any configuration of the invention, the constant region of the heavy chain transgenic locus is a non-human vertebrate constant region (eg, mouse or rat constant region). Optionally, the constant region is endogenous to said non-human vertebrate. Alternatively, the constant region of the heavy chain transgene is human constant region. For example, the constant region is human and devoid of a CH1. This is useful for producing human H2 antibodies (especially when the vertebrate is not capable of expressing light chains).

In one example of a or each vertebrate used in the invention, the constant region is or comprises a Cmu, eg , a mouse or rat Cmu. For example, where the vertebrate is a mouse the Cmu is an endogenous mouse Cmu. The transgenic heavy chain locus, in an example, comprises a Smu switch 5' of the Cmu and a Cgamma 3' of the Cmu, with a S gamma switch between the Cmu and Cgamma. In an embodiment, the Cmu, Cgamma and switches are endogenous mouse C regions and switches. For example, the C regions and switches are mouse 129 C regions and switches; or the C regions and switches are mouse Black 6 C regions and switches. In another embodiment, the S gamma and C regions are mouse S gamma and C regions, and the Smu is a rat Smu.

In one aspect each vertebrate of the population is a mouse whose genetic background is selected from mouse strains C57BL/6, M129 such as 129/SV, BALB/c, and any hybrid of C57BL/6, M129 such as 129/SV, or BALB/c. In an embodiment, each of these vertebrates have the same genetic background but two or more of the vertebrates of the population differ in their human gene segment repertoires as per the invention.

Disclosed herein is a method of providing a synthetic antibody heavy chain sequence repertoire in a population of non-human vertebrates, the method comprising
(a) providing a population of transgenic non-human vertebrates (optionally mice or rats), wherein the population provides a repertoire of different human VH gene segments, the repertoire being divided between two or more vertebrates of said population,
(b) a first vertebrate of said population comprising a transgenic heavy chain locus comprising one or more human VH gene segments (first VH gene sub-repertoire), D segments and J segments operably connected upstream of a constant region ; and
(c) a second vertebrate of said population comprising a transgenic heavy chain locus comprising one or more human VH gene segments (second VH gene sub-repertoire), D segments and J segments operably connected upstream of a constant region;
(d) wherein the first VH gene sub-repertoire is different from the second VH gene sub-repertoire, whereby the first vertebrate can produce a heavy chain sequence repertoire that is different from the heavy chain sequence repertoire produced by the second vertebrate.

Thus, the population provides an overall heavy chain repertoire comprising the heavy chain sequence repertoires of the first and second vertebrates. The vertebrates in the population can be immunised with the same antigen in a method of selecting and isolating one or more heavy chains (eg, provided as part of antibodies) that specifically bind to the antigen.

The VH gene segments of a repertoire can, in one embodiment, be recombined VH, ie, provided as part of a variable region sequence derived from the recombination of human VH with D and JH (eg, where the VH, D and JH are human).

In an embodiment, the population comprises a third non-human vertebrate, the third vertebrate comprising a transgenic heavy chain locus comprising one or more human VH gene segments (third VH gene sub-repertoire), D segments and J segments operably connected upstream of a constant region; wherein the third VH gene sub-repertoire is different from the first and second VH gene sub-repertoires, whereby the third vertebrate can produce a heavy chain sequence repertoire that is different from the heavy chain sequence repertoire produced by the first and second vertebrates. Thus, the population provides an overall heavy chain repertoire comprising the heavy chain sequence repertoires of the first, second and third vertebrates. The vertebrates in the population can be immunised with the same antigen in a method of selecting and isolating one or more heavy chains (eg, provided as part of antibodies) that specifically bind to the antigen.

In an embodiment, VH gene segment repertoire provided by said population comprises a substantially complete repertoire of functional human VH gene segments; optionally providing at least 6 different human JH gene segments, 27 different human D segments and at least 40 different human VH gene segments.

In an embodiment, the VH gene segment repertoire provided by said population comprises at least 20, 25, 30, 35 or 40 different human VH gene segments.

In an embodiment, the J segments of each transgenic heavy chain locus are human JH gene segments; optionally wherein each heavy chain locus comprises a substantially complete functional repertoire of human JH gene segments.

In an embodiment, each heavy chain locus comprises at least 2, 3, 4, 5 or 6 different human JH gene segments.

In an embodiment, the D segments of each transgenic heavy chain locus are human D gene segments; optionally wherein each heavy chain locus comprises a substantially complete functional repertoire of human D gene segments.

In an embodiment, each heavy chain locus comprises at least 5, 10, 15, 20, 25, 26 or 27 different human D gene segments.

In an embodiment, the heavy chain loci of said vertebrates comprise identical human D and JH gene segment repertoires, but differ in their VH gene repertoires.

In an embodiment, each heavy chain locus comprises at least two human JH gene segments selected from the group consisting of J1, J2, J3, J4, J5 and J6; optionally all of the gene segments of the group.

In an embodiment, each vertebrate comprises human VH gene segments selected from the group consisting of V6-1, V1-2, V1-3, V4-4, V7-41, V2-5, V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46, V3-48, V3-49, V5-51, V3-53, V1-58, V4-59, V4-61, V3-64, V3-66, V1-69, V2-70, V3-72, V3-73 and V3-74; wherein the VH gene repertoire comprises a substantially complete human functional VH gene repertoire.

In an embodiment, endogenous antibody heavy chain expression has been inactivated in the vertebrates. For example, less than 10, 5, 4, 3, 2, 1 or 0.5% of heavy chains are provided by endogenous heavy chains (ie, heavy chains whose variable regions are derived from recombination of non-human vertebrate V, D and J gene segments).

In an embodiment, the method of the disclosure comprises the step of immunising the vertebrates of the population with the same antigen (eg, a human antigen). Thus, the vertebrates are a population and are used as such.

In an embodiment, immunisation of two, more or all of said vertebrates is separated by no more than 12, 9, 6, 5, 4, 3, 2 or 1 months or 3, 2 or 1 week or 6, 5, 4, 3, 2, or 1 day. Thus, the vertebrates are a population and are used as such.

In an embodiment, the method of the disclosure comprises the step of selecting one or more heavy chains or antibodies from each of said immunised vertebrates on the basis of a common desired antibody or heavy chain characteristic (eg, binding affinity for said antigen), wherein the selected antibodies or heavy chains comprise heavy chain variable region sequences derived from the human VH gene segment repertoire provided by the population.

In an embodiment, the selected antibodies or heavy chains provide a repertoire of selected antibodies or heavy chains (selected repertoire), the method further comprising selecting one or more antibodies or heavy chains from the selected repertoire on the basis of a desired antibody or heavy chain characteristic (eg, binding affinity for said antigen or a different antigen (eg, a related antigen; which is useful for producing bispecific antibodies); or on the basis of the epitope bound by the antibody or heavy chain); optionally wherein the selected repertoire is formed by pooling the selected antibodies or heavy chains.

In an example, the vertebrates share the same genetic background, with the exception of the heavy chain loci thereof (and optionally one or more of the light chain loci thereof).

In any configuration of the invention, in an embodiment vertebrates are derived from transgenic non-human vertebrate ancestor embryonic stem cells that have been genetically modified to include human immunoglobulin locus DNA (eg, human heavy chain V, D and J gene segments and/or human light chain V and J gene segments), the ancestor stem cells being identical or related (eg, clonally related); optionally wherein the genome of the ancestor stem cells comprise a common sequence junction that is a junction between a non-human vertebrate sequence and a human sequence (eg, the ancestor genomes comprise a common transgenic immunoglobulin locus or a common human/non-human vertebrate (eg, human/mouse or human/rat) DNA junction). For example, the genomes comprise a common junction within or at the boundary of one or more of their immunoglobulin chain loci (eg, heavy chain loci and/or light chain loci). For example, the vertebrates of the population are mice whose genomes comprise a common human-mouse DNA junction within their heavy chain loci and/or one or more light chain loci. This is indicative that the mice form a population. For example, by producing variant vertebrates all stemming back from a common ancestor, the vertebrates can all share the same genetic background with the exception of one or more human gene segment repertoires in their genomes. This means that, with the exception of the expression profile resulting from the different gene segment sub-repertoires, there are no other introduced genetic variables between the members of the population, which enhances consistency of performance of the members of the population. This also simplifies breeding to produce the variants making up the population.

Disclosed herein is a method of providing a synthetic antibody light chain sequence repertoire in a population of non-human vertebrates, the method comprising
(a) providing a population of transgenic non-human vertebrates (optionally mice or rats), wherein the population provides a repertoire of different human VL gene segments, the repertoire being divided between two or more vertebrates of said population,
(b) a first vertebrate of said population comprising a transgenic light chain locus comprising one or more human VL gene segments (first VL gene sub-repertoire) and J segments operably connected upstream of a constant region ; and
(c) a second vertebrate of said population comprising a transgenic light chain locus comprising one or more human VL gene segments (second VL gene sub-repertoire) and J segments operably connected upstream of a constant region;
(d) wherein the first VL gene sub-repertoire is different from the second VL gene sub-repertoire, whereby the first vertebrate can produce a light chain sequence repertoire that is different from the light chain sequence repertoire produced by the second vertebrate.

Thus, the population provides an overall light chain repertoire comprising the light chain sequence repertoires of the first and second vertebrates. The vertebrates in the population can be immunised with the same antigen in a method of selecting and isolating one or more light chains (eg, provided as part of antibodies) that specifically bind to the antigen.

The VL gene segments of a repertoire can, in one embodiment, be recombined VL, ie, provided as part of a variable region sequence derived from the recombination of human VL with JL (eg, where the VL and JL are human).

In an embodiment, the population comprises a third non-human vertebrate, the third vertebrate comprising a transgenic light chain locus comprising one or more human VL gene segments (third VL gene sub-repertoire) and J segments operably connected upstream of a constant region; wherein the third VL gene sub-repertoire is different from the first and second VL gene sub-repertoires, whereby the third vertebrate can produce a light chain sequence repertoire that is different from the light chain sequence repertoire produced by the first and second vertebrates. Thus, the population provides an overall light chain repertoire comprising the light chain sequence repertoires of the first, second and third vertebrates. The vertebrates in the population can be immunised with the same antigen in a method of selecting and isolating one or more light chains (eg, provided as part of antibodies) that specifically bind to the antigen.

In an embodiment, the J segments of each transgenic light chain locus are human JL gene segments; optionally wherein each light chain locus comprises a substantially complete functional repertoire of human Jκ or Jλ gene segments (eg, each transgenic locus comprises human Vλ gene segments and Jλ gene segments, optionally a substantially complete functional repertoire of human Jλ gene segments; or each transgenic locus comprises human Vκ gene segments and Jκ gene segments, optionally a substantially complete functional repertoire of human Jκ gene segments).

In an embodiment, the repertoire provided by said population comprises a substantially complete repertoire of functional human Vκ gene segments; optionally providing at least 5 different human Jκ gene segments and at least at least 40 different human Vκ gene segments. In an example, the repertoire if provided by a population of non-human vertebrates of the disclosure, wherein each vertebrate comprises an IgK locus whose Vκ repertoire consists of 30 or less, 20 or less, 14 or less or 6 or less human Vκ gene segment types, as shown in the examples.

In an embodiment, the repertoire provided by said population comprises at least 20, 25, 30, 35 or 40 different human Vκ gene segments.

In an embodiment, the repertoire provided by said population comprises a substantially complete repertoire of functional human Vλ gene segments; optionally providing at least 5 different human Jλ gene segments and at least 40 different human Vλ gene segments.

In an embodiment, the repertoire provided by said population comprises at least 20, 25, 30, 35 or 40 different human Vλ gene segments.

In an embodiment, each light chain locus comprises at least 2, 3, 4, 5 or 6 different human Jκ or Jλ gene segments.

In an embodiment, the light chain loci of said vertebrates comprise identical human JL gene segment repertoires, but differ in their VL gene repertoires.

In an embodiment, endogenous antibody kappa and/or lambda light chain expression has been inactivated in the vertebrates.

In an embodiment, said transgenic light chain loci of the vertebrates are kappa light chain loci (at the endogenous kappa loci, ie, corresponding to the position of a kappa locus in a wild-type non-human vertebrate genome). For example, a transgenic kappa locus can comprise human Vκ gene segments and Jκ gene segments upstream of a constant region (eg, a CH, Cλ or Cκ gene segment; optionally which is an endogenous gene segment). For example, a transgenic kappa locus can comprise human Vλ gene segments and Jλ gene segments upstream of a constant region (eg, a CH, Cλ or Cκ gene segment; optionally which is an endogenous gene segment).

In an embodiment, said transgenic light chain loci of the vertebrates are lambda light chain loci (at the endogenous lambda loci, ie, corresponding to the position of a lambda locus in a wild-type non-human vertebrate genome). For example, a transgenic lambda locus can comprise human Vκ gene segments and Jκ gene segments upstream of a constant region (eg, a CH, Cλ or Cκ gene segment; optionally which is an endogenous gene segment). For example, a transgenic lambda locus can comprise human Vλ gene segments and Jλ gene segments upstream of a constant region (eg, a CH, Cλ or Cκ gene segment; optionally which is an endogenous gene segment).

In an embodiment, the method of the disclosure comprises the step of immunising the vertebrates of the population with the same antigen (eg, a human antigen). Thus, the vertebrates are a population and are used as such.

In an embodiment, immunisation of two, more or all of said vertebrates is separated by no more than 12, 9, 6, 5, 4, 3, 2 or 1 months or 3, 2 or 1 week or 6, 5, 4, 3, 2, or 1 day. Thus, the vertebrates are a population and are used as such.

In an embodiment, the method of the disclosure comprises the step of selecting one or more light chains or antibodies from each of said immunised vertebrates on the basis of a common desired antibody or heavy chain characteristic (eg, binding affinity for said antigen), wherein the selected antibodies or light chains comprise light chain variable region sequences derived from the human VL gene segment repertoire provided by the population.

In an embodiment, the selected antibodies or light chains provide a repertoire of selected antibodies or light chains (selected repertoire), the method further comprising selecting one or more antibodies or light chains from the selected repertoire on the basis of a desired antibody or light chain characteristic (eg, binding affinity for said antigen or a different antigen (eg, a related antigen; which is useful for producing bispecific antibodies); or on the basis of the epitope bound by the antibody or light chain); optionally wherein the selected repertoire is formed by pooling the selected antibodies or light chains.

Pooling, as mentioned herein, refers to providing a combination or collection for further use (eg, for further selection of members of the combination or collection on the basis of desirable antibody or antibody chain characteristic). In an example, the members are physically pooled (ie, mixed in a single or a relatively small number of containers, eg, two, three, four, five or six containers).

In an embodiment, the vertebrates share the same genetic background, with the exception of said transgenic light chain loci thereof (and optionally one or more of the heavy chain loci thereof).

In an embodiment, the vertebrates are derived from transgenic non-human vertebrate ancestor embryonic stem cells that have been genetically modified to include human immunoglobulin locus DNA, the ancestor stem cells being identical or related; optionally wherein the genome of the ancestor stem cells comprise a common sequence junction that is a junction between a non-human vertebrate sequence and a human sequence (eg, the ancestor genomes comprise a common transgenic immunoglobulin locus or a common human/non-human vertebrate (eg, human/mouse or human/rat) DNA junction).

The disclosure provides a method of selecting an antibody that binds a predetermined antigen (eg, a human antigen), the method comprising
(a) providing a repertoire of antibodies (first repertoire) that bind said antigen, wherein the antibodies comprise human heavy and light chain variable regions and the repertoire comprises
   i. A sub-repertoire of antibodies (lambda sub-repertoire) whose light chain variable regions are produced by rearrangement of a human Vλ gene segment with a human J_{L} gene segment; and
   ii. A sub-repertoire of antibodies (kappa sub-repertoire) whose light chain variable regions are produced by rearrangement of a human Vκ gene segment with a human J_{L} gene segment;
(b) selecting one or more antibodies from the lambda sub-repertoire according to a desired antibody characteristic (eg, binding affinity for said antigen);
(c) selecting one or more antibodies from the kappa sub-repertoire according to said desired antibody characteristic;
   wherein a repertoire (second repertoire) of selected lambda and kappa antibodies is produced, the antibodies of the second repertoire comprising human variable regions that bind said antigen; and
(d) Selecting one or more antibodies from said second repertoire on the basis of a desired antibody characteristic (eg, binding affinity for said antigen);

Wherein in step (a)(i) the lambda sub-repertoire is produced by immunisation of one or more non-human vertebrates (optionally mice or rats) (lambda vertebrates) with said antigen, wherein the lambda vertebrates express more human lambda-type antibodies (antibodies whose light chain variable regions are derived from the rearrangement of a human Vλ gene segment) than kappa-type antibodies (antibodies whose light chain variable regions are derived from the rearrangement of a VK gene segment);

Wherein in step (a)(ii) the kappa sub-repertoire is produced by immunisation of one or more non-human vertebrates (optionally mice or rats) (kappa vertebrates) with said antigen, wherein the kappa vertebrates express more human kappa-type antibodies (antibodies whose light chain variable regions are derived from the rearrangement of a human Vκ gene segment) than lambda-type antibodies (antibodies whose light chain variable regions are derived from the rearrangement of a Vλ gene segment).

Each sub-repertoire comprises at least two antibodies, for example, each sub-repertoire comprises or consists of at least 10, 15, 20, 50, 100, 10³, 10⁴, 10⁵, 10⁶, 10⁷, 10⁸, 10⁹, 10¹⁰, 10¹¹, 10¹², 10¹³, or 10¹⁴ antibodies.

Examples of a desirable antibody characteristic are affinity for binding a predetermined antigen or epitope (eg, as determined by surface plasmon resonance), completion with a known antibody for binding to a predetermined antigen or epitope, epitopic specificity of the antibody (eg, as determined by X-ray crystallography, competition with a known antibody for antigen binding wherein the known antibody specifically binds to the antigen (eg, as determined by surface plasmon resonance, eg, Biacore™), performance in ELISA or another immunoassay, a desirable biophysical characteristic (eg, melting temperature, pl, solution state, degree of aggregation, storage profile etc).

The lambda-type antibodies can comprise any constant region, eg, Cλ, Cκ or CH (optionally wherein the C is endogenous). The kappa-type antibodies can comprise any constant region, eg, Cλ, Cκ or CH (optionally wherein the C is endogenous).

Methods of immunisation for use in the invention are well known to the skilled person and may involve a classic prime-boost regime, RIMMS or any other protocol. An adjuvant may be administered with the antigen, as is known in the art.

The second repertoire comprises at least two antibodies, for example, each sub-repertoire comprises or consists of at least 10, 15, 20, 50, 100, 10³, 10⁴, 10⁵, 10⁶, 10⁷, 10^{8,} 10⁹, 10¹⁰, 10¹¹, 10¹², 10¹³, or 10¹⁴ antibodies.

Human heavy chain variable regions are variable regions derived from recombination of a human VH gene segment with a D and JH gene segment (eg, wherein the D and JH gene segments are also human). Human light chain variable regions are variable regions derived from recombination of a human VL gene segment with a JL gene segment (eg, wherein the JL gene segment is also human).

In an embodiment, the lambda vertebrates express substantially no kappa-type antibodies. For example, less than 10, 5, 4, 3, 2, 1 or 0.5% of kappa antibodies are endogenous.

In an embodiment, endogenous kappa antibody expression is substantially inactive in the lambda vertebrates. For example, less than 10, 5, 4, 3, 2, 1 or 0.5% of kappa antibodies are endogenous.

In an embodiment, the kappa vertebrates express substantially no lambda-type antibodies. For example, less than 10, 5, 4, 3, 2, 1 or 0.5% of lambda antibodies are endogenous. Due to the relatively low endogenous lambda expression in mice, when the vertebrates are mice, it may not be necessary to carry out any specific genetic manipulation in the mice to achieve substantially inactive lambda expression.

In an embodiment, endogenous lambda antibody expression is substantially inactive in the kappa vertebrates. For example, less than 10, 5, 4, 3, 2, 1 or 0.5% of lambda antibodies are endogenous. Due to the relatively low endogenous lambda expression in mice, when the vertebrates are mice, it may not be necessary to carry out any specific genetic manipulation in the mice to achieve substantially inactive lambda expression.

In an embodiment, formation (eg, first or last immunisation) of the lambda sub-repertoire is separated by no more than 12, 9, 6, 5, 4, 3, 2 or 1 months or 3, 2 or 1 week or 6, 5, 4, 3, 2, or 1 day from the formation (eg, first or last immunisation) of the kappa sub-repertoire. Thus, the vertebrates form a population and are used as such.

In an embodiment, the second repertoire is formed by pooling the selected lambda and kappa antibodies.

In an embodiment, lambda and kappa antibodies are selected on the basis of affinity of binding to said antigen (higher affinity being preferable to lower affinity).

In an embodiment, affinity is determined by surface plasmon resonance.

In an embodiment, the kappa and lambda vertebrates share the same genetic background, with the exception of the light chain loci thereof (and optionally heavy chain loci thereof).

In an embodiment, the kappa and lambda vertebrates are derived from transgenic non-human vertebrate ancestor embryonic stem cells that have been genetically modified to include human immunoglobulin locus DNA, the ancestor stem cells being identical or related; optionally wherein the genome of the ancestor stem cells comprise a common sequence junction that is a junction between a non-human vertebrate sequence and a human sequence (eg, the ancestor genomes comprise a common transgenic immunoglobulin locus or a common human/non-human vertebrate (eg, human/mouse or human/rat) DNA junction).

Disclosed herein is a population of transgenic non-human vertebrates (optionally mice or rats), wherein the population provides a repertoire of different human VH gene segments, the repertoire being divided between two or more vertebrates of said population,
(a) a first vertebrate of said population comprising a transgenic heavy chain locus comprising one or more human VH gene segments (first VH gene sub-repertoire), D segments and J segments operably connected upstream of a constant region ; and
(b) a second vertebrate of said population comprising a transgenic heavy chain locus comprising one or more human VH gene segments (second VH gene sub-repertoire), D segments and J segments operably connected upstream of a constant region;
(c) wherein the first VH gene sub-repertoire is different from the second VH gene sub-repertoire for expression of first and second antibody heavy chain sequence repertoires respectively that are different from each other, whereby the population provides a synthetic repertoire of antibody heavy chain sequences.

In an embodiment, the population comprises a third non-human vertebrate, the third vertebrate comprising a transgenic heavy chain locus comprising one or more human VH gene segments (third VH gene sub-repertoire), D segments and J segments operably connected upstream of a constant region; wherein the third VH gene sub-repertoire is different from the first and second VH gene sub-repertoires for expression of a third antibody heavy chain sequence repertoire that is different from the first and second antibody heavy chain sequence repertoires, whereby the population provides a synthetic repertoire of antibody heavy chain sequences.

In an embodiment, the vertebrates have been immunised with the same antigen (eg, a human antigen).

In an embodiment, the repertoire provided by said population comprises a substantially complete repertoire of functional human VH gene segments; optionally providing at least 6 different human JH gene segments, 27 different human D segments and at least 40 different human VH gene segments. In an embodiment, the human VH gene segment repertoire provided by said population comprises at least 20, 25, 30, 35 or 40 different human VH gene segments.

In an embodiment, the J segments of each transgenic heavy chain locus are human JH gene segments; optionally wherein each heavy chain locus comprises a substantially complete functional repertoire of human JH gene segments.

In an embodiment, each heavy chain locus comprises at least 2, 3, 4, 5 or 6 different human JH gene segments.

In an embodiment, the D segments of each transgenic heavy chain locus are human D gene segments; optionally wherein each heavy chain locus comprises a substantially complete functional repertoire of human D gene segments.

In an embodiment, each heavy chain locus comprises at least 5, 10, 15, 20, 25, 26 or 27 different human D gene segments.

In an embodiment, the heavy chain loci of said vertebrates comprise identical human D and JH gene segment repertoires, but differ in their VH gene repertoires.

In an embodiment, each heavy chain locus comprises at least two human JH gene segments selected from the group consisting of J1, J2, J3, J4, J5 and J6; optionally all of the gene segments of the group.

In an embodiment, each heavy chain locus comprises at least 10 human D gene segments selected from the group consisting of D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26, D6-25 and D7-27; optionally all of the gene segments of the group.

In an embodiment, the population comprises a third vertebrate as described above, wherein each vertebrate comprises human VH gene segments selected from the group consisting of V6-1, V1-2, V1-3, V4-4, V7-41, V2-5, V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46, V3-48, V3-49, V5-51, V3-53, V1-58, V4-59, V4-61, V3-64, V3-66, V1-69, V2-70, V3-72, V3-73 and V3-74;
wherein the population provided by the repertoire comprises a substantially complete human functional VH gene repertoire.

In an embodiment, endogenous antibody heavy chain expression has been inactivated in the vertebrates.

In an embodiment, two, more or all of said vertebrates of the population have been immunised with the same antigen, wherein two or more of the immunisations are separated by no more than 12, 9, 6, 5, 4, 3, 2 or 1 months or 3, 2 or 1 week or 6, 5, 4, 3, 2, or 1 day.

In an embodiment, the vertebrates share the same genetic background, with the exception of the heavy chain loci thereof (and optionally one or more of the light chain loci thereof).

In an embodiment, the vertebrates are derived from transgenic non-human vertebrate ancestor embryonic stem cells that have been genetically modified to include human immunoglobulin locus DNA, the ancestor stem cells being identical or related; optionally wherein the genome of the ancestor stem cells comprise a common sequence junction that is a junction between a non-human vertebrate sequence and a human sequence (eg, the ancestor genomes comprise a common transgenic immunoglobulin locus or a common human/non-human vertebrate (eg, human/mouse or human/rat) DNA junction).

The disclosure provides a population of transgenic non-human vertebrates (optionally mice or rats, wherein the population provides a repertoire of different human VL gene segments, the repertoire being divided between two or more vertebrates of said population,
a first vertebrate of said population comprising a transgenic light chain locus comprising one or more human VL gene segments (first VL gene sub-repertoire) and J segments operably connected upstream of a constant region ; and
a second vertebrate of said population comprising a transgenic light chain locus comprising one or more human VL gene segments (second VL gene sub-repertoire) and J segments operably connected upstream of a constant region;
wherein the first VL gene sub-repertoire is different from the second VL gene sub-repertoire for expression of first and second antibody light chain sequence repertoires respectively that are different from each other, whereby the population provides a synthetic repertoire of antibody light chain sequences.

In an embodiment, the population comprises a third non-human vertebrate, the third vertebrate comprising a transgenic light chain locus comprising one or more human VL gene segments (third VL gene sub-repertoire) and J segments operably connected upstream of a constant region; wherein the third VL gene sub-repertoire is different from the first and second VH gene sub-repertoires for expression of a third antibody light chain sequence repertoire that is different from the first and second antibody light chain sequence repertoires, whereby the population provides a synthetic repertoire of antibody light chain sequences.

In an embodiment, the vertebrates have been immunised with the same antigen (eg, a human antigen).

In an embodiment, the J segments of each transgenic light chain locus are human JL gene segments; optionally wherein each light chain locus comprises a substantially complete functional repertoire of human Jκ or Jλ gene segments.

In an embodiment, the VL gene segment repertoire provided by said population comprises a substantially complete repertoire of functional human Vκ gene segments; optionally providing at least 5 different human Jκ gene segments and at least 40 different human Vκ gene segments.

In an embodiment, the VL gene segment repertoire provided by said population comprises at least 20, 25, 30, 35 or 40 different human Vκ gene segments.

In an embodiment, the VL gene segment repertoire provided by said population comprises a substantially complete repertoire of functional human Vλ gene segments; optionally providing at least 5 different human Jλ gene segments and 30 different human Vλ gene segments.

In an embodiment, the VL gene segment repertoire provided by said population comprises at least 20, 25 or 30 different human Vλ gene segments.

In an embodiment, each light chain locus comprises at least 2, 3, 4, 5 or 6 different human Jκ or Jλ gene segments.

In an embodiment, the light chain loci of said vertebrates comprise identical human JL gene segment repertoires, but differ in their VL gene repertoires.

In an embodiment, endogenous antibody kappa and/or lambda light chain expression has been inactivated in the vertebrates.

In an embodiment, said transgenic light chain loci of the vertebrates are kappa light chain loci.

In an embodiment, said transgenic light chain loci of the vertebrates are lambda light chain loci.

In an embodiment, the light chain loci of said vertebrates comprise identical human JL gene segment repertoires, but differ in their VL gene repertoires.

In an embodiment, each light chain locus comprises at least 2, 3, 4 or 5 human Jκ gene segments or at least 2, 3, 4 or 5 human Jλ gene segments.

In an embodiment, two, more or all of said vertebrates of the population have been immunised with the same antigen, wherein two or more of the immunisations are separated by no more than 12, 9, 6, 5, 4, 3, 2 or 1 months or 3, 2 or 1 week or 6, 5, 4, 3, 2, or 1 day.

In an embodiment, the vertebrates share the same genetic background, with the exception of said transgenic light chain loci thereof (and optionally one or more of the heavy chain loci thereof).

In an embodiment, the vertebrates are derived from transgenic non-human vertebrate ancestor embryonic stem cells that have been genetically modified to include human immunoglobulin locus DNA, the ancestor stem cells being identical or related; optionally wherein the genome of the ancestor stem cells comprise a common sequence junction that is a junction between a non-human vertebrate sequence and a human sequence (eg, the ancestor genomes comprise a common transgenic immunoglobulin locus or a common human/non-human vertebrate (eg, human/mouse or human/rat) DNA junction).

The disclosure provides a population of non-human vertebrates (optionally mice or rats), wherein the genome of each vertebrate comprises:
One or more transgenic immunoglobulin heavy chain loci, each locus comprising one or more human V gene segments, one or more human D gene segments and one or more human J gene segments upstream of one or more heavy chain constant regions; and
One or more transgenic immunoglobulin light chain loci, each locus comprising one or more human V_{L} gene segments and one or more human J_{L} gene segments upstream of one or more light chain constant regions;
Wherein in each vertebrate the gene segments in transgenic heavy chain loci are operably linked to the constant region thereof, and the gene segments in transgenic light chain loci are operably linked to the constant region thereof, so that upon immunisation the vertebrate is capable of producing an antibody comprising heavy chains produced by recombination of a heavy chain locus and light chains produced by recombination of a light chain locus, wherein the heavy and light chains comprise human variable regions;
Wherein the population comprises
   (i) a first vertebrate type (lambda vertebrates) wherein said light chain loci comprise one or more human Vλ gene segments, wherein following rearrangement the loci express light chain sequences comprising variable region sequences derived from human Vλ gene segments (human lambda light chain sequences), wherein the lambda vertebrates express more lambda light chain sequences than kappa light chain sequences (sequences of light chains comprising variable region sequences derived from Vκ gene segments); and
   (ii) a second vertebrate type (kappa vertebrates) wherein said light chain loci comprise one or more human Vκ gene segments, wherein following rearrangement the loci express light chain sequences comprising variable region sequences derived from human Vκ gene segments (human kappa light chain sequences), wherein the kappa vertebrates express more kappa light chain sequences than lambda light chain sequences (sequences of light chains comprising variable regions derived from Vλ gene segments);
wherein the vertebrates of said population can be immunised with the same antigen to produce a repertoire of antibodies comprising human heavy and light chain variable regions, wherein the repertoire comprises a sub-repertoire of human lambda antibodies (lambda sub-repertoire) produced by vertebrates of the first type and a sub-repertoire of human kappa antibodies (kappa sub-repertoire) produced by vertebrates of the second type.

In an embodiment, the vertebrates have been immunised with the same antigen; optionally a human antigen.

In an embodiment, immunisation of the lambda vertebrates is separated by no more than 12, 9, 6, 5, 4, 3, 2 or 1 months or 3, 2 or 1 week or 6, 5, 4, 3, 2, or 1 day from the immunisation of the kappa vertebrates.

In an embodiment, the lambda vertebrates express substantially no kappa-type antibodies.

In an embodiment, endogenous kappa antibody expression is substantially inactive in the lambda vertebrates.

In an embodiment, the kappa vertebrates express substantially no lambda-type antibodies.

In an embodiment, endogenous lambda antibody expression is substantially inactive in the kappa vertebrates.

Disclosed herein is an animal house or a laboratory containing a population according to the disclosure. For example, vertebrates of the population can be housed in the same cage or in the same collection of cages in the same animal house, building or laboratory. The cages or vertebrates themselves may be labelled so that they are part of the same population or experiment. They may be owned by the same owner, eg, the same company, or in the control of a single person or company. They may be allocated for use in the same research programme or series of related research experiments aimed at discovering one or more antibodies or antibody chains against a common antigen or related antigens. Thus, the vertebrates provide a population and are used as such. It is indicative of a population, that the vertebrates are discussed in the context of the same research programme or immunisation schedule or experiment or set of experiments in a laboratory notebook or a set of laboratory notebooks that relate to the same research programme or immunisation schedule or experiment or set of experiments. For example, such a programme, schedule or experiment(s) may relate to immunisation of the vertebrates of a population with the same antigen.

Disclosed herein is a selected repertoire of antibodies produced according to the method disclosed herein, or a second repertoire of antibodies produced according to the method disclosed herein, wherein the antibodies of the selected or second repertoire have been selected for binding a common antigen (eg, a human antigen) from a population of non-human vertebrates.

In an embodiment, in the repertoire, the vertebrates are derived from transgenic non-human vertebrate ancestor embryonic stem cells that have been genetically modified to include human immunoglobulin locus DNA, the ancestor stem cells being identical or related; optionally wherein the genome of the ancestor stem cells comprise a common sequence junction that is a junction between a non-human vertebrate sequence and a human sequence (eg, the ancestor genomes comprise a common transgenic immunoglobulin locus or a common human/non-human vertebrate (eg, human/mouse or human/rat) DNA junction).

In an embodiment, in the repertoire, the population is according to any configuration, aspect, embodiment, example or description of the invention herein.

The disclosure provides a non-human vertebrate (optionally a mouse or rat), wherein the genome of each vertebrate comprises:
One or more transgenic immunoglobulin heavy chain loci, each locus comprising one or more human V gene segments, one or more human D gene segments and one or more human J gene segments upstream of one or more heavy chain constant regions; and
One or more transgenic immunoglobulin light chain loci (eg, lambda loci or kappa loci), each locus comprising a human V_{λ} gene segment repertoire and one or more human Jλ gene segments upstream of one or more light chain constant regions;
Wherein following rearrangement the light chain loci express light chain sequences comprising variable region sequences derived from human Vλ gene segments (human lambda light chain sequences);
Wherein the kappa (and optionally endogenous lambda) light chain expression has been substantially inactivated so that the vertebrate expresses more human lambda light chain sequences than kappa light chain sequences (sequences of light chains comprising variable region sequences derived from Vκ gene segments);
Wherein endogenous heavy chain expression has been substantially inactivated; and Wherein each said transgenic light chain locus comprises a substantially complete functional V_{λ} gene segment repertoire of a human.

Such vertebrates express light chains wherein all or substantially all light chains comprise a human lambda light chain sequence and the vertebrates express substantially no endogenous Ig chains or kappa chains. Such vertebrates are useful as a population of lambda vertebrates for use in the seventh and tenth configuration disclosed herein(ie, embodiments of the invention where there are separate lambda vertebrates and kappa vertebrates).

For example, less than 10, 5, 4, 3, 2, 1 or 0.5% of kappa antibodies are endogenous or no kappa antibodies are endogenous.

For example, less than 10, 5, 4, 3, 2, 1 or 0.5% of lambda antibodies are endogenous or no lambda antibodies are endogenous. Due to the relatively low endogenous lambda expression in mice, when the vertebrates are mice, it may not be necessary to carry out any specific genetic manipulation in the mice to achieve substantially inactive lambda expression.

For example, less than 10, 5, 4, 3, 2, 1 or 0.5% of heavy antibodies are endogenous or no heavy antibodies are endogenous.

In an embodiment of the vertebrate, each said transgenic light chain locus comprises at least 20, 25,26, 27, 28 or 29 human V_{λ} gene segments selected from the group consisting of V3-1, V2-8, V3-9, V3-10, V2-11, V3-12, V3-16, V2-18,V3-19, V3-21, V3-22, V2-23, V3-25, V3-27, V1-36, V5-37, V5-39, V1-40, V7-43, V1-44, V5-45, V7-46, V1-74, V9-49, V1-51, V5-52, V6-57, V4-60, V8-61 and V4-69; optionally all of the gene segments of the group.

In an embodiment of the vertebrate, each said transgenic light chain locus comprises a substantially complete functional J_{λ} gene segment repertoire of a human.

In an embodiment of the vertebrate, each said transgenic light chain locus comprises at least 3 or 4 human J_{λ} gene segments selected from the group consisting of J1, J2, J3, J6 and J7; optionally all of the gene segments of the group.

In an embodiment of the vertebrate, each said transgenic heavy chain locus comprises a substantially complete functional VH gene segment repertoire of a human.

In an embodiment of the vertebrate, each said transgenic heavy chain locus comprises at least 30, 35, 36, 37, 38 or 39 human VH gene segments selected from the group consisting of V6-1, V1-2, V1-3, V4-4, V7-41, V2-5, V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46, V3-48, V3-49, V5-51, V3-53, V1-58, V4-59, V4-61, V3-64, V3-66, V1-69, V2-70, V3-72, V3-73 and V3-74; optionally all of the gene segments of the group.

In an embodiment of the vertebrate, each said transgenic heavy chain locus comprises at least 4 or 5 human JH gene segments selected from the group consisting of J1, J2, J3, J4, J5, J6; optionally all of the gene segments of the group.

In an embodiment of the vertebrate, each said transgenic heavy chain locus comprises at least 15, 20, 21, 22, 23, 24, 25 or 26 human D gene segments selected from the group consisting of D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26, D6-25 and D7-27; optionally all of the gene segments of the group.

Disclosed herein is a population of non-human vertebrates (optionally mice or rats), wherein each vertebrate is according to the invention, wherein the population provides a human VH gene segment repertoire that is divided between two or more vertebrates of said population as per the invention.

Disclosed herein is a population of non-human vertebrates (optionally mice or rats), wherein each vertebrate is according to the disclosure, wherein the population provides a human VH gene segment repertoire that is divided between first, second and third vertebrates, wherein
the first vertebrate comprises a human VH gene repertoire comprising 5 or 6 gene segments from the group consisting of V6-1, V1-2, V1-3, V4-4, V7-41 and V2-5;
the second vertebrate comprises a human VH gene repertoire comprising 15, 16, 17, 18, 19, 20 or 21 gene segments from the group consisting of V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46 and V3-48; and the third vertebrate comprises a human VH gene repertoire comprising 10, 11, 12 or 13 gene segments from the group consisting of V3-49, V5-51, V3-53, V1-58, V4-59, V4-61, V3-64, V3-66, V1-69, V2-70, V3-72, V3-73 and V3-74;
Wherein the VH gene repertoires of the vertebrates are different from each other;
Wherein the population provides a substantially complete functional VH gene segment repertoire of a human and a substantially complete functional Vλ gene segment of a human, such that following immunisation of the vertebrates of the population with an antigen a synthetic repertoire of antibodies can be produced that is derived from the substantially complete functional human VH and Vλ repertoires substantially in the absence of kappa chain expression and endogenous heavy chain expression.

In an embodiment of the population,
the first vertebrate comprises a human VH gene repertoire consisting of V6-1, V1-2, V1-3, V4-4, V7-41 and V2-5;
the second vertebrate comprises a human VH gene repertoire consisting of V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46 and V3-48; and
the third vertebrate comprises a human VH gene repertoire consisting of V3-49, V5-51, V3-53, V1-58, V4-59, V4-61, V3-64, V3-66, V1-69, V2-70, V3-72, V3-73 and V3-74.

In an embodiment of the population, the vertebrates express no kappa light chains at all.

Disclosed herein is a method of isolating an antibody (lambda-type antibody) that binds a predetermined antigen and whose heavy and light chain variable regions are derived from human VH and Vλ gene segments respectively, the method comprising immunising the vertebrate or the population of vertebrates according to the disclosure with the antigen and selecting a lambda-type antibody from said vertebrate or population.

Disclosed herein is a pharmaceutical composition comprising an antibody selected as described above or a derivative thereof that binds said antigen, together with a pharmaceutically acceptable diluent, carrier or excipient.

Examples of derivative antibodies (according to any aspect herein) are antibodies that have one or more mutations compared to the isolated antibody (eg, to improve antigen-binding affinity and/or to enhance or inactivate Fc function) Such mutants specifically bind the antigen. Mutation or adaptation to produce a derivative includes, eg, mutation to produce Fc enhancement or inactivation. A derivative can be an antibody following conjugation to a toxic payload or reporter or label or other active moiety. In another example, a chimaeric antibody chain or antibody is modified by replacing one or all human constant regions thereof by a corresponding human constant region. For example, all constant regions of an antibody isolated from such a cell or vertebrate are replaced with human constant regions to produce a fully human antibody (ie, comprising human variable and constant regions). Such an antibody is useful for administration to human patients to reduce anti-antibody reaction by the patient.

Disclosed herein is a method of providing a synthetic antibody heavy chain sequence repertoire, the method comprising providing a heavy chain variable region gene segment repertoire that is divided across the genomes of two or more non-human vertebrates in which endogenous heavy chain expression is substantially inactive, the repertoire gene segments in the genomes being provided as part of transgenic heavy chain loci comprising one or more VH gene segments, one or more D gene segments and one or JH gene segments functionally connected upstream of a heavy chain constant region (eg, Cmu and/or Cgamma), wherein the genomes can express different repertoires of antibody heavy chain sequences derived from VH, D and JH gene segments;
Wherein the gene segment repertoire is selected from the group consisting of:
(a) a VH gene repertoire (eg, a human VH gene repertoire or a substantially complete functional human VH gene repertoire);
(b) a D gene repertoire (eg, a human D gene repertoire or a substantially complete functional human D gene repertoire); and
(c) a JH gene repertoire (eg, a human JH gene repertoire or a substantially complete functional human JH gene repertoire);
Optionally wherein the D and JH segments in the loci are human D and JH segments.

Disclosed herein is a method of providing a synthetic antibody kappa chain sequence repertoire, the method comprising providing a kappa chain variable region gene segment repertoire that is divided across the genomes of two or more non-human vertebrates in which endogenous kappa chain (and optionally also endogenous lambda chain) expression is substantially inactive, the repertoire gene segments in the genomes being provided as part of transgenic light chain loci comprising one or more Vκ gene segments and one or more Jκ gene segments functionally connected upstream of a light chain constant region (eg, Cκ and/or Cλ), wherein the genomes can express different repertoires of antibody kappa chain sequences derived from Vκ and Jκ gene segments;
Wherein the gene segment repertoire is selected from the group consisting of:
(a) a Vκ gene repertoire (eg, a human Vκ gene repertoire or a substantially complete functional human Vκ gene repertoire); and
(c) a Jκ gene repertoire (eg, a human Jκ gene repertoire or a substantially complete functional human Jκ gene repertoire);
Optionally wherein the Jκ segments in the loci are human Jκ segments.

Disclosed herein is a method of providing a synthetic antibody lambda chain sequence repertoire, the method comprising providing a lambda chain variable region gene segment repertoire that is divided across the genomes of two or more non-human vertebrates in which endogenous lambda chain (and optionally also endogenous kappa chain) expression is substantially inactive, the repertoire gene segments in the genomes being provided as part of transgenic light chain loci comprising one or more Vλ gene segments and one or more Jλ gene segments functionally connected upstream of a light chain constant region (eg, Cκ and/or Cλ), wherein the genomes can express different repertoires of antibody lambda chain sequences derived from Vλ and Jλ gene segments;
Wherein the gene segment repertoire is selected from the group consisting of:
(a) a Vλ gene repertoire (eg, a human Vλ gene repertoire or a substantially complete functional human Vλ gene repertoire); and
(c) a Jλ gene repertoire (eg, a human Jλ gene repertoire or a substantially complete functional human Jλ gene repertoire);
Optionally wherein the Jλ segments in the loci are human Jλ segments.

Disclosed herein is a method of providing a synthetic antibody light chain sequence repertoire, the method comprising providing
a Vκ gene repertoire in the genomes of a first group of non-human vertebrates in which endogenous lambda chain (and optionally also endogenous kappa chain) expression is substantially inactive, the Vκ genes in the genomes being provided as part of transgenic light chain loci comprising one or more Jκ gene segments functionally connected upstream of a light chain constant region (eg, Cκ and/or Cλ, eg, human Cλ), wherein the genomes can express repertoires of antibody kappa light chain sequences derived from Vκ and Jκ gene segments substantially in the absence of lambda light chain expression; and
a Vλ gene repertoire in the genomes of a second group of non-human vertebrates in which endogenous kappa chain (and optionally also endogenous lambda chain) expression is substantially inactive, the Vλ genes in the genomes being provided as part of transgenic light chain loci comprising one or more Jλ gene segments functionally connected upstream of a light chain constant region (eg, Cκ and/or Cλ, eg, human Cλ), wherein the genomes can express repertoires of antibody lambda light chain sequences derived from human Vλ and Jλ gene segments substantially in the absence of kappa light chain expression;
Optionally wherein the Vκ gene repertoire is a human Vκ gene repertoire (eg, a substantially complete functional human Vκ gene repertoire), the Vλ gene repertoire is a human Vλ gene repertoire (eg, a substantially complete functional human Vλ gene repertoire), the Jκ segments in the loci are human Jκ segments and the Jλ segments in the loci are human Jλ segments;
Optionally wherein the genomes of the first and second groups comprise a substantially complete functional VH gene repertoire of a human.

Disclosed herein is a population of non-human vertebrates for generating antibodies, the population comprising the first and second groups of vertebrates described above, optionally wherein the vertebrates of the population have been immunised with the same antigen (eg, a human antigen).

Disclosed herein is a synthetic repertoire of antibody heavy chain sequences, antibody light chain sequences, antibody kappa chain sequences, antibody lambda chain sequences, or a repertoire of antibodies obtained from a population of non-human vertebrates according to the disclosure; or wherein the repertoire is obtained from a vertebrate of the disclosure.

In an embodiment of the repertoire, the population of vertebrates have been immunised with the same antigen (eg, a human antigen).

In an embodiment of the repertoire, the population of vertebrates are naive.

In an embodiment of any configuration, the vertebrates are naive (ie, not immunised with a predetermined antigen, as the term is understood in the art; for example, such a vertebrate that has been kept in a relatively sterile environment as provided by an animal house used for R&D). In another example, the vertebrates have been immunised with a predetermined antigen, eg, an antigen bearing a human epitope. In another example, the population comprises naive and immunised vertebrates.

In an embodiment of the repertoire, the genomes of the vertebrates comprise a common transgenic immunoglobulin locus or a common human/non-human vertebrate (eg, human/mouse or human/rat) DNA junction.

In an embodiment of the repertoire, the members of the repertoire bind a common antigen and have been generated in the same research programme.

Disclosed herein is a method of providing a synthetic antibody heavy chain repertoire, the method comprising
(a) Dividing a human VH gene segment repertoire (eg, a substantially complete functional human VH gene repertoire) across the genomes of at least first and second non-human vertebrates (eg, mice or rats), the repertoire comprising
   a first cluster of VH gene segments corresponding to a distal VH gene cluster of the heavy chain locus of a human; and
   a second cluster of VH gene segments corresponding to a proximal VH gene cluster of the heavy chain locus of a human, wherein the proximal cluster is arranged proximally to the distal cluster in said human locus;
   Wherein the distal cluster is provided in a heavy chain locus of said first vertebrate upstream of one or more D gene segments, one or more JH gene segments and one or more constant regions;
   Wherein the proximal cluster is provided in a heavy chain locus of said second vertebrate upstream of one or more D gene segments, one or more JH gene segments and one or more constant regions;
   Wherein the proximal VH gene cluster is not present between the distal cluster and the D gene segments in the heavy chain locus of the first vertebrate (optionally wherein no further VH gene segments are present between the distal cluster and the D gene segments in the heavy chain locus of the first vertebrate); and
(b) Expressing said heavy chain loci of the first and second vertebrates to provide a repertoire of synthetic antibody heavy chains.

A cluster of human gene segments (eg, VH gene segments) refers to a collection of gene segments present in a human Ig locus (eg, a human heavy chain locus) from a first gene segment to a second, downstream (more 3') gene segment in a human genome (eg, in a germline human Ig locus). Examples of gene segment arrangements are found in the IMGT database (or by reference to Kabat or the other antibody resources available to the skilled person and described herein). For example, the gene segments in a cluster are all human VH. For example, the gene segments in a cluster are all human Vκ gene segments. For example, the gene segments in a cluster are all human Vλ gene segments. For example, the gene segments in a cluster are all human JH gene segments. For example, the gene segments in a cluster are all human Jλ gene segments. For example, the gene segments in a cluster are all human Jκ gene segments. For example, the gene segments in a cluster are all human D gene segments. For example, a human germline heavy chain Ig locus comprises the following gene cluster (in the 3' to 5' direction, ie, in the proximal to distal direction, ie in the downstream to upstream direction): V6-1, V1-2, V1-3, V4-4, V7-41. For use in the present invention, the V gene segments in this cluster can be provided in this order (the human germline order) or a different order from that shown here. If this cluster is deemed to be a "proximal cluster" in the present invention, a "distal cluster" could be (in the 3' to 5' direction, ie, in the proximal to distal direction, ie in the downstream to upstream direction): V3-49, V5-51, V3-53, V1-58, V4-59, V4-61, V3-64, V3-66, V1-69, V2-70, V3-72, V3-73 and V3-74. A medial cluster (see below) could be (in the 3' to 5' direction, ie, in the proximal to distal direction, ie in the downstream to upstream direction): V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46 and V3-48. In an example, the gene segment clusters are discrete (ie, do not share a common gene segment). In an example two or more clusters are overlapping - for example, a first gene segment cluster (such as a proximal cluster) comprises one or more gene segments that are comprised by a second gene segment cluster (such as a medial or distal cluster) and the first and second clusters are different.

In an embodiment of the method, a third vertebrate comprises a third cluster of VH gene segments corresponding to a medial VH gene cluster of the heavy chain locus of a human, wherein the medial cluster is arranged between the distal and proximal clusters in said human locus; wherein the medial cluster is provided in a heavy chain locus of said third vertebrate upstream of one or more D gene segments, one or more JH gene segments and one or more constant regions; wherein the heavy chain locus of the third vertebrate does not comprise the distal, medial and proximal clusters in an arrangement corresponding to the arrangement of said human (optionally wherein the heavy chain locus of the third vertebrate does not comprise the distal and proximal clusters); the method further comprising expressing said heavy chain locus of the third vertebrate, whereby the repertoire of synthetic antibody heavy chains is provided by expression from the first, second and third vertebrate loci.

In an embodiment of the method, the vertebrates have been immunised with the same antigen (eg, a human antigen).

In an embodiment of the method, the vertebrates are naive.

In an embodiment of the method, the vertebrates have a common collection of light chain loci. For example, the kappa chain loci alleles are identical in the vertebrates and/or the lambda chain loci alleles are identical in the vertebrates. This simplifies construction of vertebrate variants for producing the population and also simplifies breeding.

In an embodiment, the method comprises comprising selecting one or more antibody heavy chains (eg, as part of an antibodies) from said repertoire according to a desired characteristic (eg, affinity for biding an antigen).

Disclosed herein is a method of providing a synthetic antibody kappa chain repertoire, the method comprising
(a) Dividing a human Vκ gene segment repertoire (eg, a substantially complete functional human Vκ gene repertoire) across the genomes of at least first and second non-human vertebrates (eg, mice or rats), the repertoire comprising
   a first cluster of Vκ gene segments corresponding to a distal Vκ gene cluster of the kappa chain locus of a human; and
   a second cluster of Vκ gene segments corresponding to a proximal Vκ gene cluster of the kappa chain locus of a human, wherein the proximal cluster is arranged proximally to the distal cluster in said human locus;
   Wherein the distal cluster is provided in a kappa chain locus of said first vertebrate upstream of one or more Jκ gene segments and one or more constant regions;
   Wherein the proximal cluster is provided in a kappa chain locus of said second vertebrate upstream of one or more Jκ gene segments and one or more constant regions;
   Wherein the proximal Vκ gene cluster is not present between the distal cluster and the Jκ gene segments in the kappa chain locus of the first vertebrate (optionally wherein no further Vκ gene segments are present between the distal cluster and the Jκ gene segments in the kappa chain locus of the first vertebrate); and
(b) Expressing said kappa chain loci of the first and second vertebrates to provide a repertoire of synthetic antibody kappa chains.

In an embodiment of the method, a third vertebrate comprises a third cluster of Vκ gene segments corresponding to a medial Vκ gene cluster of the kappa chain locus of a human, wherein the medial cluster is arranged between the distal and proximal clusters in said human locus; wherein the medial cluster is provided in a kappa chain locus of said third vertebrate upstream of one or more Jκ gene segments and one or more constant regions; wherein the kappa chain locus of the third vertebrate does not comprise the distal, medial and proximal clusters in an arrangement corresponding to the arrangement of said human (optionally wherein the kappa chain locus of the third vertebrate does not comprise the distal and proximal clusters); the method further comprising expressing said kappa chain locus of the third vertebrate, whereby the repertoire of synthetic antibody kappa chains is provided by expression from the first, second and third vertebrate loci.

Disclosed herein is a method of providing a synthetic antibody lambda chain repertoire, the method comprising
(a) Dividing a human Vλ gene segment repertoire (eg, a substantially complete functional human Vλ gene repertoire) across the genomes of at least first and second non-human vertebrates (eg, mice or rats), the repertoire comprising
   a first cluster of Vλ gene segments corresponding to a distal Vλ gene cluster of the lambda chain locus of a human; and
   a second cluster of Vλ gene segments corresponding to a proximal Vλ gene cluster of the lambda chain locus of a human, wherein the proximal cluster is arranged proximally to the distal cluster in said human locus;
   Wherein the distal cluster is provided in a lambda chain locus of said first vertebrate upstream of one or more Jλ gene segments and one or more constant regions;
   Wherein the proximal cluster is provided in a lambda chain locus of said second vertebrate upstream of one or more Jλ gene segments and one or more constant regions;
   Wherein the proximal Vλ gene cluster is not present between the distal cluster and the Jλ gene segments in the lambda chain locus of the first vertebrate (optionally wherein no further Vλ gene segments are present between the distal cluster and the Jλ gene segments in the lambda chain locus of the first vertebrate); and
(b) Expressing said lambda chain loci of the first and second vertebrates to provide a repertoire of synthetic antibody lambda chains.

In an embodiment of the method, a third vertebrate comprises a third cluster of Vλ gene segments corresponding to a medial Vλ gene cluster of the lambda chain locus of a human, wherein the medial cluster is arranged between the distal and proximal clusters in said human locus; wherein the medial cluster is provided in a lambda chain locus of said third vertebrate upstream of one or more Jλ gene segments and one or more constant regions; wherein the lambda chain locus of the third vertebrate does not comprise the distal, medial and proximal clusters in an arrangement corresponding to the arrangement of said human (optionally wherein the lambda chain locus of the third vertebrate does not comprise the distal and proximal clusters); the method further comprising expressing said lambda chain locus of the third vertebrate, whereby the repertoire of synthetic antibody lambda chains is provided by expression from the first, second and third vertebrate loci.

In an embodiment of the method, the vertebrates have been immunised with the same antigen (eg, a human antigen).

In an embodiment of the method, the vertebrates are naive.

In an embodiment of the method, the vertebrates have a common collection of heavy chain loci.

In an embodiment, the method comprises selecting one or more antibody kappa or lambda chains (eg, as part of an antibodies) from said repertoire according to a desired characteristic (eg, affinity for biding an antigen).

As explained in the examples, the inventors carried out sectoring using a repertoire in which they inverted human gene segments that are naturally in an opposite 5'-3' orientation in a human germline genome (eg, see the Ig kappa locus arrangement, eg, as shown in the IMGT database and Lefranc, M.-P., Exp. Clin. Immunogenet., 18, 161-174 (2001) - see 5' gene segments marked with "D"). The distribution of human gene segment usage in transgenic non-human vertebrates surprisingly demonstrated that artificially inverted gene segments were used for rearrangement and expression, the usage unexpectedly being relatively high (Fig. 10). These findings indicate that, although these inverted gene segments are rarely used in humans, they are used in transgenic vertebrates and cells of the disclosure and can contribute to a transgenic Ig locus expression well once they are inverted to an orientation opposite to a wild-type human germline orientation.

Thus, the disclosure provides a non-human vertebrate (optionally a mouse or a rat) or vertebrate cell (optionally a mouse cell or a rat cell) whose genome comprises a transgenic antibody chain locus comprising one or more human V gene segments and one or more human J gene segments (and optionally one or more human D gene segments) upstream of a constant region, the locus comprising one or more inverted vertebrate species gene segments, the inverted gene segment(s) being present in the locus in a 5'-3' orientation that is opposite to the vertebrate species germline orientation of such segment(s), and wherein the non-human vertebrate or cell is capable of expressing an antibody chain sequence comprising a sequence that is derived from an inverted gene segment.

In one example, the cell is a B-cell, hybridoma, ES cell or iPS cell. ES cells and iPS cells can be used to develop corresponding non-human vertebrates (vertebrates of the disclosure) in which the inverted gene segment(s) are functional as the inventors surprisingly observed.

In an embodiment, the inverted gene segment(s) are V gene segments.

In an embodiment, the vertebrate species is selected from human, mouse, rat, rabbit, guinea pig, chicken, a fish, a bird, a reptile, a *Camelid,* bovine, chimpanzee, a non-human primate and a primate.

In an embodiment, the vertebrate species is human and optionally also the vertebrate of the disclosure is a mouse or rat.

Disclosed herein is a non-human vertebrate (optionally a mouse or a rat) or vertebrate cell (optionally a mouse cell or a rat cell) whose genome comprises a transgenic antibody chain locus comprising one or more human V gene segments and one or more human J gene segments (and optionally one or more human D gene segments) upstream of a constant region, wherein the locus comprises one or more inverted human gene segments, the inverted human gene segment(s) being present in the locus in a 5'-3' orientation that is opposite to the human germline orientation of such segment(s), and wherein the vertebrate or cell is capable of expressing an antibody chain sequence comprising a variable region that is derived from recombination of an inverted gene segment.

In an embodiment, the inverted gene segment(s) are or comprise human Vκ gene segment(s).

In an embodiment, the inverted Vκ gene segment(s) comprise one or more or all of V_{K}2D-40, 1D-39, 1D-33, 2D-30, 2D-29, 2D-28, 2D-26, 3D-20, 1D-17, 1D-16, 3D-15, 1D-13, 1D-12, 3D-11, 1D-43, 1D-8 and 3D-7.

In an embodiment, the locus comprises human Vκ gene segments comprising Vκ4-1 and/or 5-2 with one or more or all of Vκ2D-40, 1D-39, 1D-33, 2D-30, 2D-29, 2D-28, 2D-26, 3D-20, 1D-17, 1D-16, 3D-15, 1D-13, 1D-12, 3D-11, 1D-43, 1D-8 and 3D-7, wherein all of the human Vκ gene segments are in the same orientation as the constant region of the locus. Additionally or alternatively, for example, the locus comprises one, more or all of human Vκ2D-40, 1D-39, 1D-33 and 2D-30 3' of one more or all of Vκ3D-7, 1D-8, 1D-43 and 3D-11. In one embodiment, Vκ2D-40, 1D-39, 1D-33 and 2D-30 are present in this order 3' to 5' in the locus. Additionally or alternatively, in one embodiment, Vκ3D-7, 1D-8, 1D-43 and 3D-11 are present in this order 3' to 5' in the locus.

In one example, the 5'-most inverted Vκ gene segment in the locus is Vκ3D-20. In another example, it is Vκ2D-40 (eg, if the sequence of naturally-inverted human Vκ gene segments found in a wild-type human chromosome 2 are provided as this sequence, but in reverse orientation - with Vκ2D-40 as the 5'-most inverted gene segment in the locus of the disclosure and Vκ3D-7 is the 3'-most inverted gene segment).

In an embodiment, the Vκ gene segment(s) comprise human Vκ2D-40.

In an embodiment, the Vκ gene segment(s) comprise human Vκ1D-39, for example human Vκ2D-40 next to human Vκ1D-39 (eg, in 5' to 3' direction: human Vκ2D-40 next to human Vκ1D-39).

In an embodiment, the constant region is human. In another embodiment, the constant region is a mouse or rat constant region, eg, a constant region endogenous to the non-human vertebrate.

Disclosed herein is a method of providing an artificial human antibody variable region repertoire, the method comprising inserting one or more human V gene segment(s) (inverted gene segments) upstream of one or more J gene segments, optionally one or more D gene segments, and a constant region in an antibody chain locus of a non-human vertebrate or non-human vertebrate cell, the V gene segment(s) being present in the locus in a 5'-3' orientation that is opposite to the human germline orientation of such segment(s), and wherein the non-human vertebrate or cell (or a non-human vertebrate progeny derived from the cell) is capable of expressing an antibody chain sequence comprising a variable region sequence that is derived from recombination of an inverted gene segment.

In an embodiment, the vertebrate or cell or inverted gene segment(s) is as recited above.

Disclosed herein is a method of providing an artificial human antibody variable region repertoire, the method comprising isolating serum or a lymphoid cell (eg, spleen cell or B-cell) from a vertebrate of the disclosure, and optionally isolating from the serum or cell one or more antibodies that specifically bind a predetermined antigen.

Disclosed herein is an antibody isolated in the method described above, or a fragment or derivative thereof.

It will be understood that particular embodiments described herein are shown by way of illustration and not as limitations of the invention and (unless the context states otherwise) embodiments can be applied to any of the configurations of the invention described herein. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term

"about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

All of the compositions, populations, vertebrates, antibodies, repertoires and/or methods disclosed and claimed herein can be made and executed without undue experimentation in light of the present disclosure.

The present invention is described in more detail in the following non limiting Examples.

### EXAMPLES

### EXAMPLE 1: Light Chain Repertoire Sectoring

The inventors realised that the prior art mice provide for immunoglobulin loci in which all of the desired heavy, kappa and lambda gene segment repertoires are provided together in the genome of the cells of the individual mice. In doing this, the inventors realised that the prior art designs have not addressed the limitation of the restricted accessible B-cell component size (around 2x10⁸ B-cells in a mouse, for example) and the concomitant restriction on the overall antibody chain diversity that can be expressed and accessed in any one mouse.

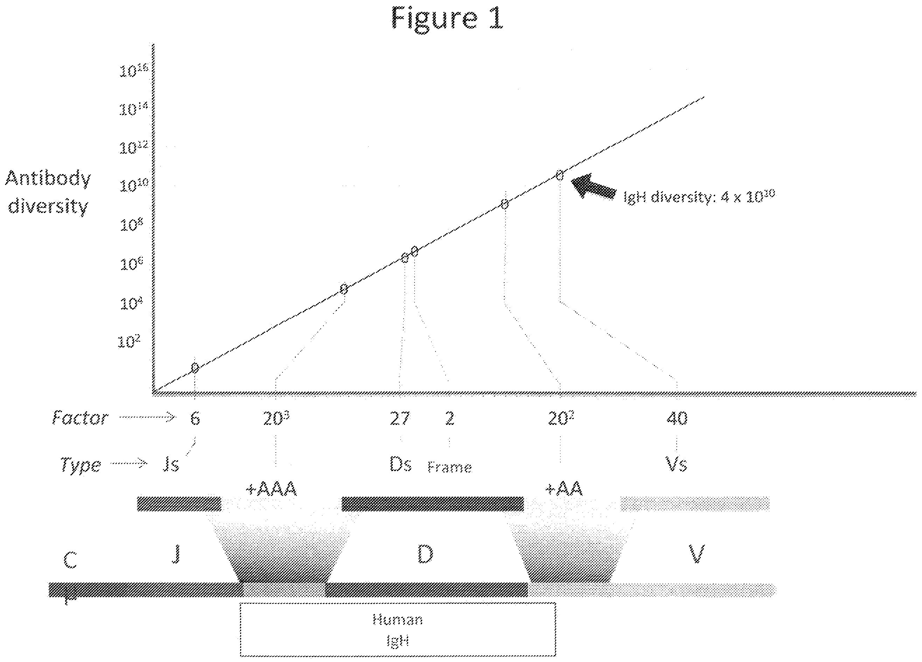

This is illustrated by reference to figures 1 to 4. Figure 1 shows a calculation of potential diversity for a transgenic heavy chain locus comprising a complete functional repertoire of human VH, D and JH gene segments (in this example, 6 different human JH, 27 different human D and 40 different human VH germline gene segments). The calculation takes into account junctional diversity (typically a 3 nucleotide addition at the J-D junction and a 2 nucleotide addition at the D-V junction). The calculation of potential diversity of heavy chain variable region sequences produced by recombination of the gene segments is 4x10¹⁰.

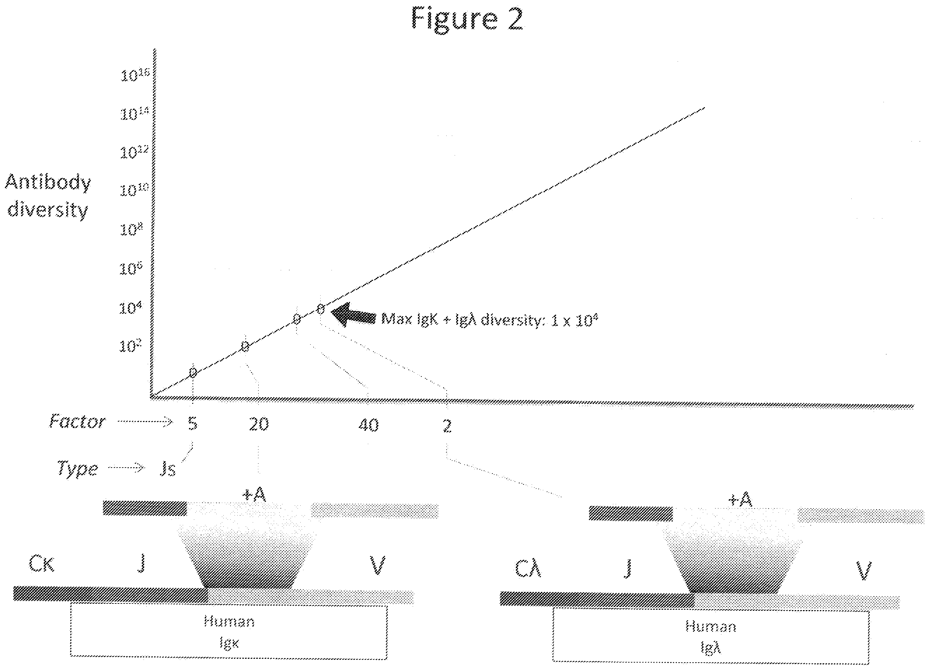

Figure 2 shows a calculation of potential diversity for a transgenic kappa and lambda light chain loci, each comprising a complete functional repertoire of human VH, D and JH gene segments (in this example, 5 different human JL and 40 different human VL germline gene segments in each light chain locus). The calculation takes into account junctional diversity (typically a 1 nucleotide addition at the V-J junction). The calculation of potential diversity of kappa and lambda chain variable region sequences produced by recombination of the gene segments is 1x10⁴.

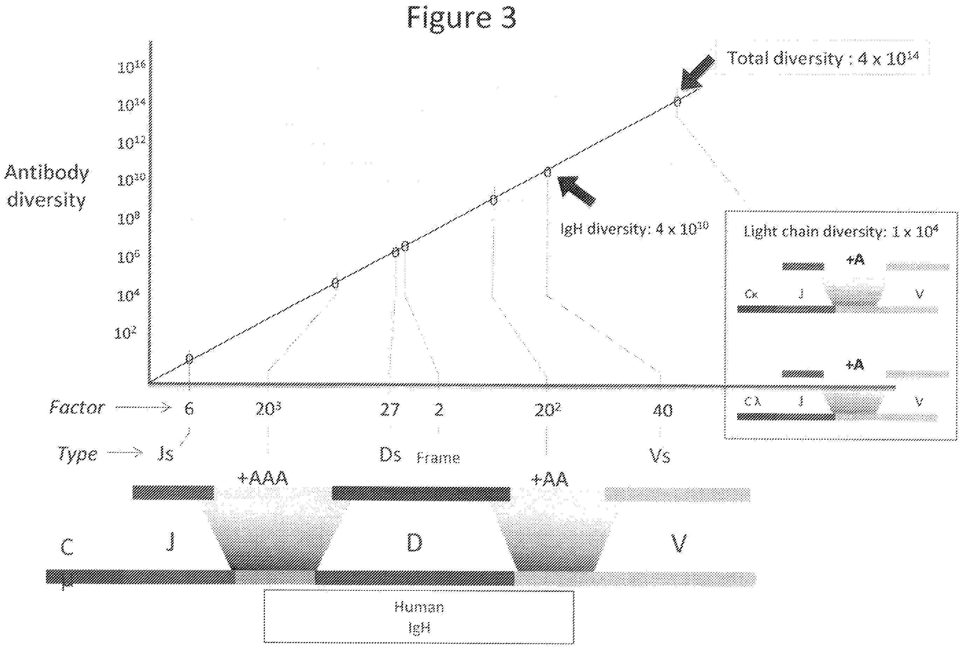

Figure 3 shows a calculation of potential diversity for transgenic heavy, kappa and lambda light chain loci (ie, the calculation of total potential heavy/light chain variable region combinations). The calculated result is a potential diversity of 4x10¹⁴.

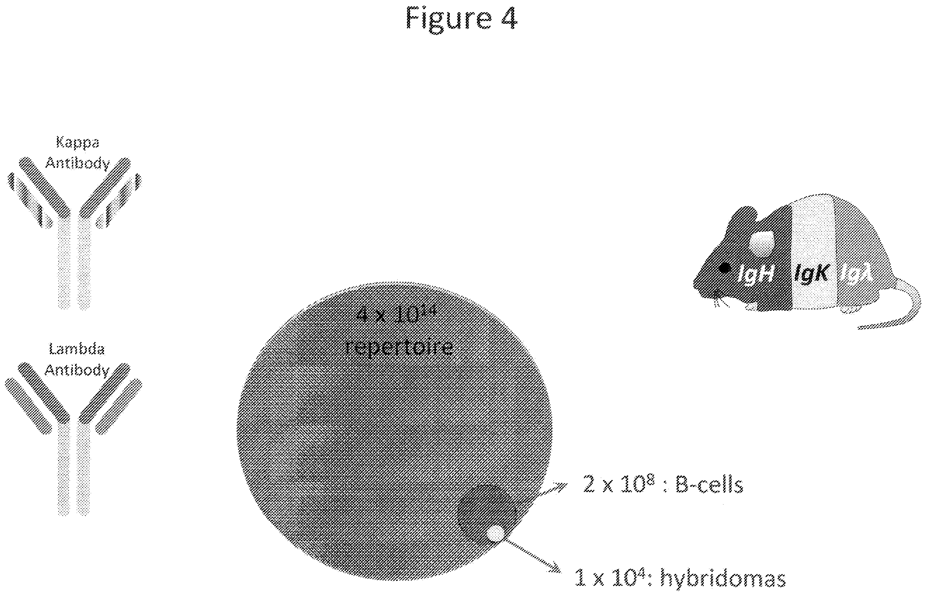

Figure 4 schematically illustrates the disadvantage of the existing theoretical designs where mice will comprise human heavy, kappa and lambda gene repertoires together in the same mouse. To date, the state of the art has not reported the successful production of a mouse containing complete functional human heavy, kappa and lambda gene segment repertoires altogether in one mouse genome. Nevertheless, if the skilled person did do this (by, for example, using the complete repertoires described in Figs 1-3) from a possible antibody variable region repertoire size of 4x10¹⁴ only a maximum of around 2x10⁸ antibodies can be sampled from the accessible B-cell compartment (actually the art often struggles even to sample at anywhere near this level). Thus, a huge amount of the potential diversity cannot be sampled. This is further compounded by the problem of kappa antibody bias in mice to the detriment of sampling the lambda sequence space more fully. There also can be biases in individual V regions that may dominate the immune response following immunisation; in this case, more rarely-used gene segments may not be accessed even though they may provide for desirable antibodies (eg, with desired affinities and/or desired epitopic recognition). The issue is even more stark when one considers that of the maximum 2x10⁸ B-cells that can be accessed, typically only a small fraction of these generate hybridomas when using hybridoma generation methods routinely in the art (hybridoma generation being the standard method for enabling workable access to a research and production supply of a desired monoclonal antibody). Typically, after the application of hybridoma generation technologies, only up to around 1x10⁴ hybridomas can be generated. Thus, the acute problem of poor diversity sampling will be readily apparent to the skilled addressee: of the original potential repertoire, only 1x10⁴ of the potential 4x10¹⁴ combinations can maximally be accessed. This is a miniscule amount of the diversity which severely limits the discovery of new and useful antibodies from mice designed according to the prior art concepts.

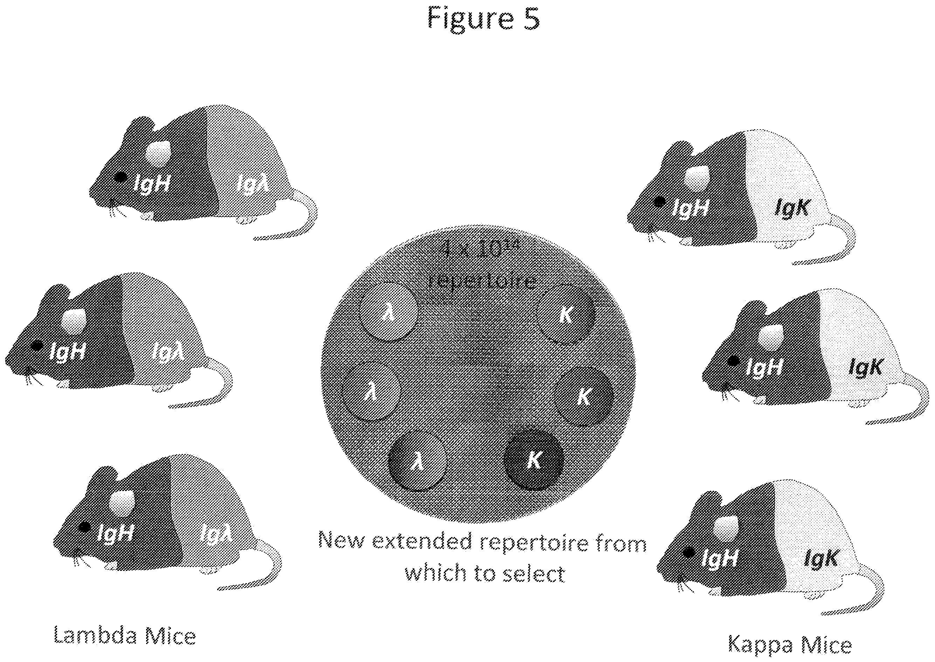

The present inventors have addressed this problem by sectoring the overall desired repertoire in transgenic animals so that the repertoire is instead accessed not in a single genome, but across a plurality of genomes making up a population. This is schematically illustrated in Figure 5 for an example where the light chain repertoire is sectored to separate lambda gene segment diversity from kappa gene segment diversity. This is possible, for example, by the provision of separate lambda vertebrates and kappa vertebrates according to the disclosure. In the example of figure 5, all mice comprise the same transgenic heavy chain locus (the locus illustrated in figure 1 in which there is a complete human functional diversity of V, D and J gene segments). In this example, the complete functional human Vλ gene segment repertoire has been divided across three mice, so that the mice comprise different human Vλ gene segment sub-repertoires (in this case the sub-repertoires are not overlapping, although it is possible for them to overlap as long as they are not identical in their collections of gene segments). Similarly, a complete functional Vκ gene segment repertoire has been sectored across three different kappa vertebrates (right-hand side of the figure). The large circle at the centre of the figure represents the potential overall diversity of 4x10¹⁴ and each smaller circle represents the accessible B-cell compartment of the respective mice. It can be readily appreciated that this example allows for six individual samplings (sub-repertoires) of the overall potential sequence space. Thus, these sub-repertoires together provide a population diversity in which the overall repertoire size is significantly greater than possible with the conventional design shown in figure 4. Furthermore, in addition to significantly extending the repertoire for sampling, importantly the repertoires in the lambda mice are novel, synthetic repertoires that are not seen in conventional transgenic mice that express prior art kappa and lambda antibody ratios together in the same mouse. For example, when the expression of kappa light chain sequences has been inactivated, all antibodies in the lambda mice will comprise lambda light chains, and this is not seen in mice in nature. Similarly (eg, where the endogenous lambda expression is totally inactivated or negligible) in the kappa mice kappa-type antibodies are essentially exclusively expressed. When taken together, the lambda-type and kappa-type repertoires of antibodies expressed in the lambda and kappa mice (both in naive mice (those not immunised with a predetermined antigen or substantially not exposed to foreign antigen) and immunised mice) provide an overall antibody repertoire that is novel, usefully extended and synthetic (not seen in nature).

### EXAMPLE 2: Human VH Gene Segment Repertoire & Human Light Chain Repertoire Sectoring

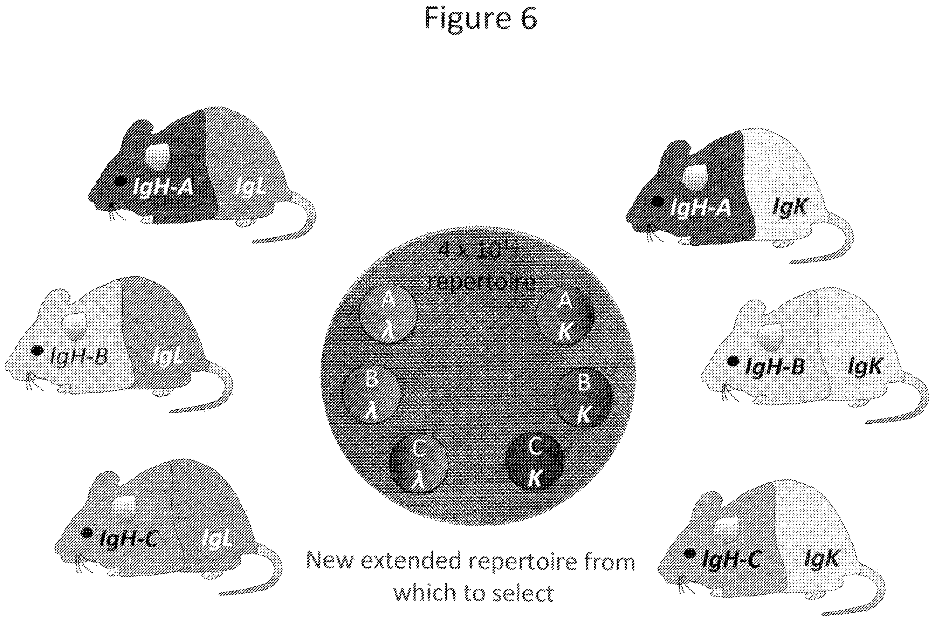

Figure 6 shows a schematic example where the heavy chain VH gene segment repertoire is sectored according to the disclosure. In this example, the repertoire is a complete human functional VH gene repertoire divided across three mice (mice "A", "B" and "C") as follows:-
A... V6-1, V1-2, V1-3, V4-4, V7-41, V2-5, V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46, V3-48, V3-49, V5-51, V3-53, V1-58, V4-59, V4-61, V3-64, V3-66, V1-69, V2-70, V3-72, V3-73 & V3-74
B... V6-1, V1-2, V1-3, V4-4, V7-41, V2-5, V4-28, V3-30, V4-31, V3-33, V4-34, V4-39, V3-43, V1-45, V1-46 & V3-48
C... V6-1, V1-2, V1-3, V4-4, V7-41, V2-5, V3-49, V5-51, V3-53, V1-58, V4-59, V4-61, V3-64, V3-66, V1-69, V2-70, V3-72, V3-73 & V3-74

In this example, although the V gene repertoires of mice A, B and C overlap, the repertoires are unique. In an alternative example, the repertoires do not overlap, eg, the VH gene repertoires are non-overlapping and comprise or consist of as follows:-
**A'...** V6-1, V1-2, V1-3, V4-4, V7-41 and V2-5
**B'...** V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46 and V3-48
**C'...** V3-49, V5-51, V3-53, V1-58, V4-59, V4-61, V3-64, V3-66, V1-69, V2-70, V3-72, V3-73 and V3-74

In another alternative example, the VH gene repertoires comprise or consist of as follows:-
**S...** V6-1, V1-2, V1-3, V4-4, V7-41, V2-5, V3-7, V1-8, V3-9, V3-11, V3-13, V3-15, V1-18, V3-20, V3-21, V3-23, V1-24 and V2-26
**T...** V4-28, V3-30, V4-31, V3-33, V4-34, V4-39, V3-43, V1-45, V1-46 and V3-48
**V...** V3-49, V5-51, V3-53, V1-58, V4-59, V4-61, V3-64, V3-66, V1-69, V2-70, V3-72, V3-73 and V3-74

The VH gene sub-repertoire of each mouse is provided in a transgenic heavy chain locus comprising a complete functional human JH gene repertoire and a complete functional human D gene repertoire. Endogenous heavy chain expression is inactivated. It is possible to provide each mouse with the same light chain loci (eg, one or more transgenic lambda locus and one or more transgenic kappa locus, wherein each light chain locus comprises human VL and JL gene segments). In the example of figure 6, however, the lambda and kappa gene repertoires have also been sectored according to the invention as per the illustration in Figure 5. In either example, endogenous kappa chain expression is inactivated in the lambda mice (and endogenous lambda chain expression is optionally inactivated in the kappa mice).

In one example, the JH and D repertoire consists of or comprises

### D & JH Repertoire 1

J1, J2, J3, J4, J5 and J6
D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D1-26 and D7-27

In another example, the JH and D repertoire consists of or comprises

### D & JH Repertoire 2

J1, J2, J3, J4, J5 and J6
D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26 and D7-27

In another alternative example, the VH, DH and JH gene repertoires comprise or consist of human gene segments as follows:-

### V, D, J Repertoire 1

V6-1, V1-2, V1-3, V4-4, V7-41, V2-5, V3-7, V1-8, V3-9, V3-11, V3-13, V3-15, V1-18, V3-20, V3-21, V3-23, V1-24 and V2-26 (optionally wherein this represents the 5' to 3' order of the V gene segments)
J1, J2, J3, J4, J5 and J6
D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26 and D7-27

### V, D, J Repertoire 2

V4-28, V3-30, V4-31, V3-33, V4-34, V4-39, V3-43, V1-45, V1-46 and V3-48 (optionally wherein this represents the 5' to 3' order of the V gene segments)
J1, J2, J3, J4, J5 and J6
D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26 and D7-27 (optionally wherein this represents the 5' to 3' order of the D gene segments)

### V, D, J Repertoire 3

V3-49, V5-51, V3-53, V1-58, V4-59, V4-61, V3-64, V3-66, V1-69, V2-70, V3-72, V3-73 and V3-74 (optionally wherein this represents the 5' to 3' order of the V gene segments)
J1, J2, J3, J4, J5 and J6
D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26 and D7-27 (optionally wherein this represents the 5' to 3' order of the D gene segments)

In an example, a population of first and second non-human vertebrates (eg, mice) according to the disclosure are provided which differ in their heavy chain gene segment repertoires (and optionally comprise identical human light chain gene segment repertoires), wherein the heavy chain gene segment repertoires comprise identical repertoires of human VH gene segments and different human D gene segment repertoires,
Wherein the first vertebrate D gene segment repertoire comprises D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D1-26 and D7-27;
Wherein the second vertebrate D gene segment repertoire comprises D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26 and D7-27; and optionally
the vertebrates comprise identical JH gene repertoires (eg, J1, J2, J3, J4, J5 and J6).

In an embodiment of this example, each human VH gene segment repertoire comprises or consists of human VH gene segments (a) V6-1, V1-2, V1-3, V4-4, V7-41, V2-5, V3-7, V1-8, V3-9, V3-11, V3-13, V3-15, V1-18, V3-20, V3-21, V3-23, V1-24 and V2-26 and/or (b) V4-28, V3-30, V4-31, V3-33, V4-34, V4-39, V3-43, V1-45, V1-46 and V3-48 and/or (c) V3-49, V5-51, V3-53, V1-58, V4-59, V4-61, V3-64, V3-66, V1-69, V2-70, V3-72, V3-73 and V3-74. For example, each human VH gene segment repertoire comprises or consists of human VH gene segments (a), (b) and (c).

As seen in figure 6, each mouse provides a unique sub-repertoire of antibodies and antibody heavy and light chain sequences. The design enables the human Vλ diversity to be explored much more fully than present with prior art designs, due to the sectoring of the kappa and lambda repertoires as explained above. Furthermore, sectoring of the human VH gene repertoire provides for new gene segment arrangements in the heavy chain loci (bringing distal gene segments more proximal and providing non-natural combinations of VH gene segments together in single loci). This enables exploration of the human VH gene diversity more fully as hitherto previously possible. Additionally, in the illustrated design the unique VH gene repertoires are explored separately in mice in the context of lambda vertebrates separately from kappa vertebrates, which in turn provides for novel combinations not seen in nature or hitherto previously possible.

The transgenic mice shown in Figures 5 and 6 provide useful sources of antibody and antibody chain repertoires for sampling following immunisation with antigen. For example, all of the mice shown in the population of Figure 5 or 6 (or a combination of all of the mice in both figures) can be immunised with the same human antigen and desirable antibodies can be selected, eg, for relatively high affinity binding to the antigen or for binding in certain epitopic regions of the antigen (eg, as determined by competition with an anti-antigen antibody of known epitopic specificity). The selected antibodies thereby provide a selected repertoire of antibodies (optionally pooled) from which one or more preferred drug candidates can be selected. This selection can be on the basis of the information already obtained in the first round of selection, and/or a further set of experiments (eg, ELISA and/or surface plasmon resonance (eg, using Biacore™) to determine affinities within the selected repertoire) can be performed to enable the selection from the repertoire.

### EXAMPLE 3: Producing New Light Chain & Human Vκ Region Repertoires By Gene Sectoring Human Vκ Gene Repertoires

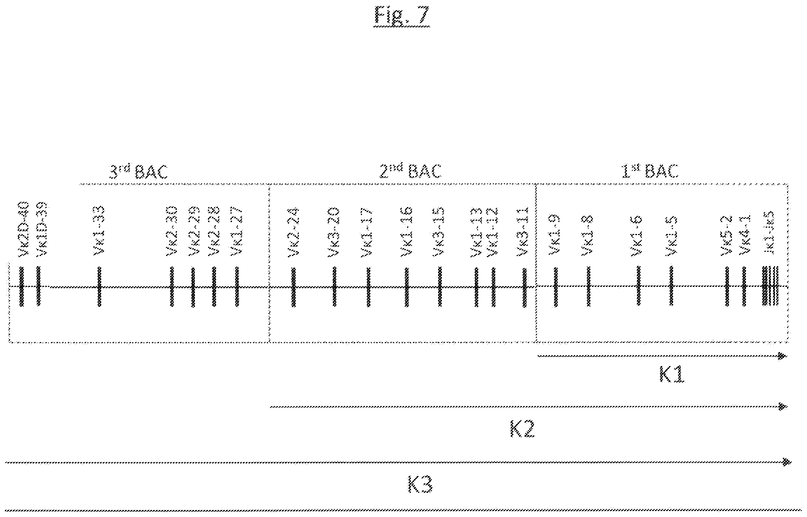

A functional human gene segment repertoire (from Vκ2D-40 to Jκ5, as shown in Fig 7) was sectored by the inventors to produce three different transgenic kappa chain alleles (denoted K1, K2 and K3) and corresponding mice. The transgenic alleles were expressed in the mice and the kappa chain repertoires were assessed at the RNA transcript level.

Insertion of human kappa gene segments from a 1st IGK BAC into the IGK locus of mouse AB2.1 ES cells (Baylor College of Medicine) was performed to create a light chain allele denoted the K1 allele. The inserted human sequence corresponds to the sequence of human chromosome 2 from position 89312220 to position 89159079 and comprises functional kappa gene segments Vκ1-9, Vκ1-8, Vκ1-6, Vκ1-5, Vκ5-2, Vκ4-1, Jκ1, Jκ2, Jκ3, Jκ4 and Jκ5 (Fig. 7). The insertion was made between positions 70674755 and 70674756 on mouse chromosome 6, which is upstream of the mouse Cκ region. The mouse Vκ and Jκ gene segments were retained in the locus, immediately upstream of (5' of) the inserted human kappa DNA. The mouse lambda loci were left intact.

A second allele, K2 was constructed in which more human functional Vκ gene segments were inserted upstream (5') of the 5'-most Vκ inserted in the K1 allele by the sequential insertion of human DNA from a second BAC. The inserted human sequence from BAC2 corresponds to the sequence of human chromosome 2 from position 89478399 to position 89312221 and comprises functional kappa gene segments Vκ2-24, Vκ3-20, Vκ1-17, Vκ1-16, Vκ3-15, Vκ1-13, Vκ1-12, Vκ3-11 (Fig. 7).

A third allele, K3 was constructed in which more human functional Vκ gene segments were inserted upstream (5') of the 5'-most Vκ inserted in the K2 allele by the sequential insertion of human DNA from a third BAC. The inserted sequence corresponds to the sequence of human chromosome 2 from position 89889512 to position 89902788 and from position 89609410 to position 89478400, and comprises functional kappa gene segments, Vκ2D-40, Vκ1D-39, Vκ1-33, Vκ2-30, Vκ2-29, Vκ2-28 and Vκ1-27 (Fig. 7).

Human gene segments Vκ2D-40 and Vκ1D-39 are naturally inverted (opposite orientation to other functional gene segments, eg those other Vκ and the Jκ in BACs1-3, when in natural human genomes) and relatively rarely used in humans (see Ig kappa locus representation in the IMGT database and Eur J Immunol. 1994 Apr; 24(4):827-36, "A directory of human germ-line V kappa segments reveals a strong bias in their usage", J Cox et *al*)*.* In the current strategy, these gene segment sequences were inverted from their natural state so that they were present in BAC3 in the same 5'-3' orientation as the other human gene segments. The inventors were interested in determining if functionality of gene segments that have been inverted during human evolution can be re-orientated in the transgenic animals, thereby enhancing functionality and thus participation in providing an expressed repertoire.

Mice bearing either the K1, K2 or K3 insertion into an endogenous kappa locus were generated from the ES cells using standard procedures. The other endogenous kappa locus was inactivated in the mice by insertion of an inactivating sequence comprising neo^{R} into the mouse Jκ-Cκ intron (to produce the "KA" allele). Standard 5'-RACE was carried out to analyse RNA transcripts from the transgenic kappa light chain loci in the K1, K2 and K3 mice.

The K3 mice showed high usage of human Vκ gene segments, 92.8% of rearranged transcripts using human Vκ gene segments and only 7.2% of them using mouse Vκ gene segments (Fig. 8). Compared to K1 and K2, K3 showed improved human Vκ usage (K1: 78%; K2:83% and K3: 93%) (Fig. 9).

The distribution of human Vκ usage from K3 mice demonstrated that the two transplanted and inverted Vκ2D-40 and Vκ1D-39 surprisingly were used for rearrangement and expression, the usage of Vκ1D-39 unexpectedly being relatively high (Fig. 10). These data indicate that, although these two Vκ gene segments are rarely used in humans, they are used and can contribute to the transgenic IgK locus expression well once they are inverted and have the same orientation to the human Jκ gene segments. Further exemplification of the surprising effect of gene segment inversion according to the disclosure is shown in Example 6 below.

The distribution of human Vκ usage from K1 to K3 mice also demonstrated that insertion of different repertoires of human Vκ gene segments changes the usage of human Vκs (Fig. 11). While not wishing to be bound by any particular theory, the inventors surmise that this is due to the varying competition among the Vκs, which determine their relative usage. This was also surprisingly observed with the human Jκ usage. For example, the inventors surprisingly observed that with the 3rd BAC insertion, the Jκ4 usage is increased (Fig. 12). Thus, the human Vκ gene repertoire sectoring according to the invention not only altered the repertoire of expressed human Vκ gene segments, but also altered the expressed Jκ profile. Thus, the inventors surprisingly realised that they had discovered a way to provide differing repertoires of antibody chains by gene repertoire sectoring. The collection of K1, K2 and K3 as a combined population is useful, the inventors realised, to produce a novel repertoire of antibody chains and antibodies that can be selected against a desired antigen. For example, the K1, K2 and K3 mice can be immunised with the same human antigen and anti-antigen antibodies from the totally of antibodies in the mice can be selected on the basis of affinity and/or target epitope recognition or another desirable feature.

Further exemplification of sectoring was established by insertion of yet more human IgK DNA (to create K4 mice), as disclosed in Example 6 below.

In summary, the analysis of kappa light chain sequences revealing differential usage and thus differing light chain sequence repertoires could be produced by the gene sectoring technique. The repertoires differed in their human V and J gene segment usage. Additionally, the proportions of particular human gene segments shared by mice could be altered in the expression profiles by the gene sectoring technique of the present invention.

### Example 4

### Producing New Heavy Chain and Human VH Region Repertoires By Gene Sectoring Human V_{H} Gene Repertoires

A functional human gene segment repertoire (from V_{H}2-26 to J_{H}6, see the IMGT database for the structure of the human IgH locus;
http://www.imgt.org/IMGTrepertoire/index.php?section=LocusGenes&repertoire=locus&species=h uman&group=IGK) was sectored by the inventors to produce three different transgenic heavy chain alleles (denoted S1, S2 and S3) and corresponding mice. The transgenic alleles were expressed in the mice and the heavy chain repertoires were assessed at the RNA transcript level.

Insertion of human heavy gene segments from a 1st IGH BAC into the IGH locus of mouse AB2.1 ES cells (Baylor College of Medicine) was performed to create a heavy chain allele denoted the S1 allele. The inserted human sequence corresponds to the sequence of human chromosome 14 from position 106494908 to position 106328951 and comprises functional heavy gene segments V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6 (in 5' to 3' order). The insertion was made between positions 114666435 and 114666436 on mouse chromosome 12, which is upstream of the mouse Cµ region. The mouse V_{H}, D and J_{H} gene segments were retained in the locus, immediately upstream of (5' of) the inserted human heavy chain DNA.

A second allele, S2 was constructed in which more human functional V_{H} gene segments were inserted upstream (5') of the 5'-most V_{H} inserted in the S1 allele by the sequential insertion of human DNA from a second BAC (BAC2). The inserted human sequence from BAC2 corresponds to the sequence of human chromosome 14 from position 106601551 to position 106494909 and comprises functional heavy chain gene segments V_{H}3-13, V_{H}3-11, V_{H}3-9, V_{H}1-8, V_{H}3-7.

A third allele, S3 was constructed in which more human functional V_{H} gene segments were inserted upstream (5') of the 5'-most V_{H} inserted in the S2 allele by the sequential insertion of human DNA from a third BAC (BAC3). The inserted sequence corresponds to the sequence of human chromosome 14 from position 106759988 to position 106609301, and comprises functional heavy chain gene segments, V_{H}2-26, V_{H}1-24, V_{H}3-23, V_{H}3-21, V_{H}3-20, V_{H}1-18, and V_{H}3-15.

Mice bearing either the S1, S2 or S3 insertion into an endogenous heavy chain locus were generated from the ES cells using standard procedures. The other endogenous heavy chain locus was inactivated in the mice by insertion of an inactivating sequence comprising neo^{R} into the mouse J_{H}-Cµ intron (to produce the "HA" allele). Standard 5'-RACE was carried out to analyse RNA transcripts from the transgenic heavy chain light chain loci in the S1, S2 and S3 mice.

The S3 mice showed high usage of human V_{H} gene segments, 62% of rearranged transcripts using human V_{H} gene segments and 38% of them using mouse Vκ gene segments (Fig. 13). Human V_{H} usage was as follows - S1: 16%; S2: 64% and S3: 62% (Fig. 13).

The distribution of human V_{H} usage from S1 to S3 mice also demonstrated that insertion of different repertoires of human V_{H} gene segments changes the usage of human V_{H}s (Fig. 14) and thus the repertoire of expressed human heavy chain variable regions is different between the mice. While not wishing to be bound by any particular theory, the inventors surmise that this is due to the varying competition among the V_{H}s, which determine their relative usage. Thus, the inventors realised that they had surprisingly discovered a way to provide differing repertoires of antibody heavy chains and heavy chain variable regions by gene repertoire sectoring. The collection of S1, S2 and S3 as a combined population is useful, the inventors realised, to produce a novel repertoire of antibody heavy chains and antibodies that can be selected against a desired antigen. For example, the S1, S2 and S3 mice (eg, wherein endogenous heavy and light chain expression has been inactivated and optionally where the mice express light chains comprising human variable regions) can be immunised with the same human antigen and anti-antigen antibodies from the totally of antibodies in the mice can be selected on the basis of affinity and/or target epitope recognition or another desirable feature.

Further sectoring of the VH gene repertoire was performed by constructing a fourth heavy chain allele, denoted "V6", in which more human functional V_{H} gene segments were inserted upstream (5') of the 5'-most V_{H} inserted in the S1 allele by the sequential insertion of human DNA from a BAC (BAC6). The inserted human sequence from BAC6 corresponds to the sequence of human chromosome 14 from position 107147078 to position 106995083 and comprises functional heavy chain gene segments V_{H}3-66, V_{H}3-64, V_{H}4-61, V_{H}4-59, V_{H}1-58, V_{H} 3-53, V_{H}5-51, V_{H}3-49 (Fig. 16).

As shown in Fig. 15, S2 and V6 mice showed greater human VH gene segment usage than S1 mice. The usage in S2 compared to V6 was, furthermore, different. See also Fig. 16.

In summary, the analysis of heavy chain sequences revealing differential usage and thus differing heavy chain and human heavy chain variable region sequence repertoires could be produced by the gene sectoring technique. The repertoires differed in their human V_{H}, D and J_{H} gene segment usage. Additionally, the proportions of particular human gene segments shared by mice could be altered in the expression profiles by the gene sectoring technique of the present invention (Figs. 14 & 16).

### Example 5

### Producing New Heavy Chain and Human V Region Repertoires By Gene Sectoring Human D Gene Repertoires

Assessment of D gene segment sectoring can be performed as follows. A functional human gene segment repertoire (from V_{H}2-26 to J_{H}6, see the IMGT database for the structure of the human IgH locus;
http://www.imgt.org/IMGTrepertoire/index.php?section=LocusGenes&repertoire=locus&species=h uman&group=IGK) is sectored to produce two different transgenic heavy chain alleles (denoted S3F and S3FD) and corresponding mice. Endogenous heavy chain variable regions are inactivated by inversion as described in WO2011004192. The transgenic alleles are expressed in the mice and the heavy chain repertoires are assessed at the RNA transcript level.

Using BAC insertion techniques similar to those described for Example 4, human heavy gene segments are inserted from a 1st IGH BAC into the IGH locus of mouse AB2.1 ES cells (Baylor College of Medicine) is performed to create a heavy chain allele denoted the S1 allele. The insertion is made between positions 114666435 and 114666436 on mouse chromosome 12, which is upstream of the mouse Cµ region. The mouse V_{H}, D and J_{H} gene segments are retained in the locus, immediately upstream of (5' of) the inserted human heavy chain DNA (and subsequently inverted to inactivate at a later stage).

A second allele, S2 is constructed in which more human functional V_{H} gene segments are inserted upstream (5') of the 5'-most V_{H} inserted in the S1 allele by the sequential insertion of human DNA from a second BAC (BAC2). The inserted human sequence from BAC2 corresponds to the sequence of human chromosome 14 from position 106601551 to position 106494909 and comprises functional heavy chain gene segments V_{H}3-13, V_{H}3-11, V_{H}3-9, V_{H}1-8, V_{H}3-7.

A third allele, S3 is constructed in which more human functional V_{H} gene segments is inserted upstream (5') of the 5'-most V_{H} inserted in the S2 allele by the sequential insertion of human DNA from a third BAC (BAC3). The inserted sequence corresponds to the sequence of human chromosome 14 from position 106759988 to position 106609301, and comprises functional heavy chain gene segments, V_{H}2-26, V_{H}1-24, V_{H}3-23, V_{H}3-21, V_{H}3-20, V_{H}1-18, and V_{H}3-15.

Two different versions of the heavy chain allele are made, differing only in their human D gene segment repertoires:
version 1 (denoted S3F) had a human gene segment repertoire V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6 (in 5' to 3' order).
version 2 (denoted S3FD) had a human gene segment repertoire V_{H}2-5, V_{H}7-4-1, V_{H}4-4, V_{H}1-3, V_{H}1-2, V_{H}6-1, D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6 (in 5' to 3' order). The underlined segments do not appear in S3F.

Mice bearing either the S3F or S3FD insertion into an endogenous heavy chain locus are generated from the ES cells using standard procedures. The other endogenous heavy chain locus is inactivated in the mice by insertion of an inactivating sequence comprising neo^{R} into the mouse J_{H}-Cµ intron (to produce the "HA" allele). Standard 5'-RACE is carried out to analyse RNA transcripts from the transgenic heavy chain light chain loci in the S3F and S3FD mice.

It is expected that the analysis of heavy chain sequences will reveal differential D gene segment usage (even for D segments found in both S3F and S3FD repertoires), and possibly different V and/or J gene segment usage, and thus differing heavy chain and human heavy chain variable region sequence repertoires will be produced by the gene sectoring technique.

### Example 6

### Plural Inverted Human Gene Segments Are Used For Ig Chain Rearrangement & Expression

In Example 3, we describe the generation of K1, K2 and K3 mice. K4 mice were generated as follows by the insertion of further DNA from the Vκ gene cluster of human chromosome 2.

A fourth allele, K4 was constructed in which more human functional Vκ gene segments were inserted upstream (5') of the 5'-most Vκ inserted in the K3 allele by the sequential insertion of human DNA from a fourth BAC. The inserted sequence corresponds to the sequence of human chromosome 2 from position [90062244] to position [90276666], and comprises functional kappa gene segments [Vκ3D-20, Vκ1D-17, Vκ1D-16, Vκ3D-15, Vκ1D-13, Vκ1D-12, Vκ3D-11, Vκ1D-43, Vκ1D-8, Vκ3D-7] (Fig. 17). These ten Vκs are in the distal cluster of Vκ segment and naturally in an opposite orientation to the Jκ exons in germline human chromosome 2 but were inverted to the proximal human Jκs and the mouse Cκ in this transgenic allele.

The K4 mice showed high usage of human Vκs, 94.5% of rearranged transcripts using human Vκs and only 5.5% of them using mouse Vκs (Fig. 18). Compared to K1, K2 and K3, K4 showed improved human Vκ usage to K1 and K2 (K1: 78%; K2:83%), and similar usage to K3 (K3: 93%) (Fig. 19). All the Vκs from the distal cluster are rarely used in human (Cox, JPL et al, "A directory of human germ-lime Vκ segments reveals a strong bias in their usage", Eur. J. Immunol. 1994. 24: 827-836). The distribution of human Vκ usage from K4 mice demonstrated that these Vκs were frequently used for rearrangement and expression (Fig. 20). These data indicate that although these Vκs from the distal cluster are rarely used in human, they are used very well once they are inverted as per the disclosure. The distribution of human Vκ usage in K4 mice also demonstrated that the insertion of human Vκ BACs changed the usage of Vκs versus K1 to K3 mice (Fig. 20). We hypothesize that the competition among those Vκs would determine their relative usage. This also applies to the Jκ usage. With the 3^{rd} BAC insertion, the Jκ4 usage is increased and this is retained following the 4^{th} BAC insertion (Fig. 21).

## Claims

1. Use of a mouse or rat in sectoring an antibody gene segment repertoire, wherein the genome of the mouse or rat comprises:
i) One or more transgenic immunoglobulin heavy chain loci, each locus comprising one or more human V gene segments, one or more human D gene segments and one or more human J gene segments operably linked upstream of one or more heavy chain constant regions, wherein the heavy chain constant region(s) comprise(s) a C gamma gene segment; and
ii) One or more transgenic immunoglobulin light chain loci, each locus comprising a human V_{λ} gene segment repertoire and one or more human Jλ gene segments operably linked upstream of one or more light chain constant regions;
Wherein following rearrangement the light chain loci express light chain sequences comprising variable region sequences derived from human Vλ gene segments (human lambda light chain sequences);
Wherein the kappa (and optionally endogenous lambda) light chain expression has been inactivated so that the mouse or rat expresses no kappa-type antibodies;
Wherein endogenous heavy chain expression has been inactivated; and
Wherein each said transgenic light chain locus comprises a complete functional V_{λ} gene segment repertoire of a human and
Wherein the mouse or rat expresses the human lambda light chain sequences upon immunisation to provide a repertoire of antibodies.

2. The use of claim 1, wherein each said transgenic light chain locus comprises at least 20, 25,26, 27, 28 or 29 human V_{λ} gene segments selected from the group consisting of V3-1, V2-8, V3-9, V3-10, V2-11, V3-12, V3-16, V2-18,V3-19, V3-21, V3-22, V2-23, V3-25, V3-27, V1-36, V5-37, V5-39, V1-40, V7-43, V1-44, V5-45, V7-46, V1-74, V9-49, V1-51, V5-52, V6-57, V4-60, V8-61 and V4-69; optionally all of the gene segments of the group.

3. The use of claim 1 or 2, wherein each said transgenic light chain locus comprises a complete functional J_{λ} gene segment repertoire of a human.

4. The use of any one of claims 1 to 3, wherein each said transgenic light chain locus comprises at least 3 or 4 human J_{λ} gene segments selected from the group consisting of J1, J2, J3, J6 and J7; optionally all of the gene segments of the group.

5. The use of any one of claims 1 to 4, wherein each said transgenic heavy chain locus comprises a complete functional VH gene segment repertoire of a human.

6. The use of claim 5, wherein each said transgenic heavy chain locus comprises at least 30, 35, 36, 37, 38 or 39 human VH gene segments selected from the group consisting of V6-1, V1-2, V1-3, V4-4, V7-41, V2-5, V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46, V3-48, V3-49, V5-51, V3-53, V1-58, V4-59, V4-61, V3-64, V3-66, V1-69, V2-70, V3-72, V3-73 and V3-74; optionally all of the gene segments of the group.

7. The use of any one of claims 1 to 6, wherein each said transgenic heavy chain locus comprises at least 4 or 5 human JH gene segments selected from the group consisting of J1, J2, J3, J4, J5, J6; optionally all of the gene segments of the group.

8. The use of any one of claims 1 to 7, wherein each said transgenic heavy chain locus comprises at least 15, 20, 21, 22, 23, 24, 25 or 26 human D gene segments selected from the group consisting of D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26, D6-25 and D7-27; optionally all of the gene segments of the group.

9. The use of any one of claims 1-8, wherein the heavy chain constant regions are non-human vertebrate heavy chain constant regions.

10. The use of any one of claims 1-8, wherein the heavy chain constant regions are mouse or rat.

11. The use of any one of claims 1-8, wherein the heavy chain constant regions are endogenous and/or the light chain constant regions are endogenous.

12. Use of a population of mice or rats in sectoring an antibody gene segment repertoire, wherein the population comprises a first mouse or rat type comprising a genome as defined in any one of claims 1 to 11 and the population additionally comprises a second mouse or rat type (kappa mice or rats) wherein the genome of the second mouse or rat type comprises:
a. one or more transgenic immunoglobulin heavy chain loci, each locus comprising one or more human V gene segments, one or more human D gene segments and one or more human J gene segments upstream of one or more heavy chain constant regions; and
b. one or more transgenic immunoglobulin light chain loci, each locus comprising one or more human V_{L} gene segments and one or more human J_{L} gene segments upstream of one or more light chain constant regions;
wherein said light chain loci comprise one or more human Vκ gene segments, wherein following rearrangement the loci express light chain sequences comprising variable region sequences derived from human Vκ gene segments (human kappa light chain sequences),
wherein each kappa mouse or rat expresses more human kappa light chain sequences than lambda light chain sequences (sequences of light chains comprising variable regions derived from Vλ gene segments); and
wherein the first mouse or rat type expressing the human lambda light chain sequences does not share common human light chain gene segments with the kappa mice or rats and wherein the mice or rats of said population are capable of producing a repertoire of antibodies comprising human heavy and light chain variable regions when immunised with the same antigen, wherein the repertoire comprises a sub-repertoire of human lambda antibodies (lambda sub-repertoire) produced by mice or rats of the first type and a sub-repertoire of human kappa antibodies (kappa sub-repertoire) produced by mice or rats of the second type.

13. Use of a population of mice or rats in sectoring an antibody gene segment repertoire, wherein the genome of each mouse or rat comprises:
(a) One or more transgenic immunoglobulin heavy chain loci, each locus comprising one or more human V gene segments, one or more human D gene segments and one or more human J gene segments operably linked upstream of one or more heavy chain constant regions, wherein the heavy chain constant region(s) comprise(s) a Cgamma gene segment; and
(b) One or more transgenic immunoglobulin light chain loci, each locus comprising a human V_{λ} gene segment repertoire and one or more human Jλ gene segments operably linked upstream of one or more light chain constant regions;
Wherein following rearrangement the light chain loci express light chain sequences comprising variable region sequences derived from human Vλ gene segments (human lambda light chain sequences);
Wherein the kappa and endogenous lambda light chain expression are inactive so that each mouse or rat expresses more human lambda light chain sequences than kappa light chain sequences (sequences of light chains comprising variable region sequences derived from Vκ gene segments);
Wherein endogenous heavy chain expression has been inactivated; and
Wherein the human Vλ gene segment repertoire is divided across the mouse or rat genomes, wherein first and second individual members of the population comprise a different sub-repertoire of human V_{λ} gene segments and the genomes can express different repertoires of antibody lambda chain sequences,
and each of the mice or rats of the population is immunised with the same antigen and one or more antibodies (lambda-type antibodies) that bind the antigen whose heavy and light chain variable regions are derived from human VH and Vλ gene segments respectively are isolated.

14. The use of claim 13, wherein the V_{λ} gene segment repertoire is a complete functional V_{λ} gene segment repertoire, optionally wherein the mice or rats comprise the following human V_{λ} gene segments V3-1, V2-8, V3-9, V3-10, V2-11, V3-12, V3-16, V2-18,V3-19, V3-21, V3-22, V2-23, V3-25, V3-27, V1-36, V5-37, V5-39, V1-40, V7-43, V1-44, V5-45, V7-46, V1-74, V9-49, V1-51, V5-52, V6-57, V4-60, V8-61 and V4-69.

## Patentansprüche

1. Verwendung einer Maus oder Ratte zum Sektorisieren eines Antikörper-Gen-Segment-Repertoires, wobei das Genom der Maus oder Ratte umfasst:
i) einen oder mehrere transgene Immunglobulin-Schwerketten-Loci, wobei jeder Locus eines oder mehrere humane V-Gensegmente, eines oder mehrere humane D-Gensegmente und eines oder mehrere humane J-Gensegmente umfasst, die funktionsmäßig stromaufwärts von einer oder mehreren konstanten Regionen der schweren Kette verknüpft sind, wobei die konstante(n) Region(en) der schweren Kette ein C-Gamma-Gensegment umfasst (umfassen); und
ii) einen oder mehrere transgene Immunglobulin-Leichtketten-Loci, wobei jeder Locus ein humanes Vλ-Gensegment-Repertoire und eines oder mehrere menschliche Jλ-Gensegmente umfasst, die funktionsmäßig stromaufwärts von einer oder mehreren konstanten Regionen der leichten Kette verknüpft sind;
wobei nach der Umlagerung die Loci der leichten Kette Sequenzen der leichten Kette exprimieren, die Sequenzen variabler Regionen umfassen, die von humanen Vλ-Gensegmenten (Sequenzen der leichten Lambda-Kette) abgeleitet sind;
wobei die Expression der leichten Kette von Kappa (und gegebenenfalls endogenem Lambda) inaktiviert wurde, so dass die Maus oder Ratte keine Antikörper vom Kappa-Typ exprimiert;
wobei die endogene Expression der schweren Kette inaktiviert wurde; und
wobei jeder transgene Locus der leichten Kette ein vollständiges funktionelles V_{λ}-Gen-Segment-Repertoire eines Menschen umfasst und
wobei die Maus oder Ratte die Sequenzen der humanen leichten Lambda-Kette nach Immunisierung exprimiert, um ein Repertoire an Antikörpern bereitzustellen.

2. Verwendung nach Anspruch 1, wobei jeder Locus der transgenen leichten Kette mindestens 20, 25, 26, 27, 28 oder 29 humane Vx-Gensegmente umfasst, die ausgewählt sind aus der Gruppe bestehend aus V3-1, V2-8, V3-9, V3-10, V2-11, V3-12, V3-16, V2-18, V3-19, V3-21, V3-22, V2-23, V3-25, V3-27, V1-36, V5-37, V5-39, V1-40, V7-43, V1-44, V5-45, V7-46, V1-74, V9-49, V1-51, V5-52, V6-57, V4-60, V8-61 und V4-69; wahlweise allen der Gensegmente der Gruppe.

3. Verwendung nach Anspruch 1 oder 2, wobei jeder transgene Locus der leichten Kette ein vollständiges funktionelles J_{λ}-Gen-Segment-Repertoire eines Menschen umfasst.

4. Verwendung nach einem der Ansprüche 1 bis 3, wobei jeder Locus der transgenen leichten Kette mindestens 3 oder 4 humane J_{λ}-Gensegmente umfasst, die ausgewählt sind aus der Gruppe bestehend aus J1, J2, J3, J6 und J7; wahlweise allen der Gensegmente der Gruppe.

5. Verwendung nach einem der Ansprüche 1 bis 4, wobei jeder transgene Locus der schweren Kette ein vollständiges funktionelles VH-Gen-Segment-Repertoire eines Menschen umfasst.

6. Verwendung nach Anspruch 5, wobei jeder Locus der transgenen schweren Kette mindestens 30, 35, 36, 37, 38 oder 39 humane VH-Gensegmente umfasst, die ausgewählt sind aus der Gruppe bestehend aus V6-1, V1-2, V1-3, V4-4, V7-41, V2-5, V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46, V3-48, V3- 49, V5-51, V3-53, V1-58, V4-59, V4-61, V3-64, V3-66, V1-69, V2-70, V3-72, V3-73 und V3-74; wahlweise allen der Gensegmente der Gruppe.

7. Verwendung nach einem der Ansprüche 1 bis 6, wobei jeder Locus der transgenen schweren Kette mindestens 4 oder 5 humane JH-Gensegmente umfasst, die ausgewählt sind aus der Gruppe bestehend aus J1, J2, J3, J4, J5, J6; wahlweise allen der Gensegmente der Gruppe.

8. Verwendung nach einem der Ansprüche 1 bis 7, wobei jeder Locus der transgenen schweren Kette mindestens 15, 20, 21, 22, 23, 24, 25 oder 26 humane D-Gensegmente umfasst, die ausgewählt sind aus der Gruppe bestehend aus D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5- 24, D1-26, D6-25 und D7-27; wahlweise allen der Gensegmente der Gruppe.

9. Verwendung nach einem der Ansprüche 1 bis 8, wobei die konstanten Regionen der schweren Kette nicht-humane konstante Regionen der schweren Kette von Wirbeltieren sind.

10. Verwendung nach einem der Ansprüche 1 bis 8, wobei die konstanten Regionen der schweren Kette von Maus oder Ratte sind.

11. Verwendung nach einem der Ansprüche 1 bis 8, wobei die konstanten Regionen der schweren Kette endogen sind und/oder die konstanten Regionen der leichten Kette endogen sind.

12. Verwendung einer Population von Mäusen oder Ratten bei der Sektorisierung eines Antikörper-Gen-Segment-Repertoires, wobei die Population einen ersten Maus- oder Rattentyp umfasst, der ein Genom nach einem der Ansprüche 1 bis 11 umfasst, und die Population zusätzlich einen zweiten Maus- oder Rattentyp umfasst (Kappa-Mäuse oder Ratten), wobei das Genom des zweiten Maus- oder Rattentyps umfasst:
a. einen oder mehrere transgene Immunglobulin-Schwerketten-Loci, wobei jeder Locus eines oder mehrere humane V-Gensegmente, eines oder mehrere humane D-Gensegmente und eines oder mehrere humane J-Gensegmente stromaufwärts einer oder mehrerer konstanter Regionen der schweren Kette umfasst; und
b. einen oder mehrere transgene Immunglobulin-Leichtketten-Loci, wobei jeder Locus eines oder mehrere humane V_{L}-Gensegmente und eines oder mehrere menschliche J_{L}-Gensegmente stromaufwärts von einer oder mehreren konstanten Regionen der leichten Kette umfasst;
wobei die Loci der leichten Kette eines oder mehrere humane V_{κ}-Gensegmente umfassen, wobei nach der Umlagerung die Loci Sequenzen der leichten Kette exprimieren, die Sequenzen variabler Regionen umfassen, die von humanen V_{κ}-Gensegmenten abgeleitet sind (humane Sequenzen der leichten Kappa-Kette),
wobei jede Kappa-Maus oder -Ratte mehr humane Kappa-Sequenzen der leichten Kette als Lambda-Sequenzen der leichten Kette exprimiert (Sequenzen von leichten Ketten, die variable Regionen umfassen, die von Vλ -Gensegmenten abgeleitet sind); und
wobei der erste Maus- oder Rattentyp, der die Sequenzen der humanen leichten Lambda-Kette exprimiert, keine gemeinsamen Genabschnitte der humanen leichten Kette mit den Kappa-Mäusen oder Ratten teilt und wobei die Mäuse oder Ratten der Population ein Repertoire von Antikörpern produzieren können, die humane variable Regionen der leichten und der schweren Kette bei Immunisierung mit dem gleichen Antigen umfassen, wobei das Repertoire ein Unterrepertoire von humanen Lambda-Antikörpern (Lambda-Unterrepertoire) umfasst, das von Mäusen oder Ratten des ersten Typs produziert wurde, und ein Unterrepertoire von humanen Kappa-Antikörpern (Kappa-Unterrepertoire), das von Mäusen oder Ratten des zweiten Typs produziert wurde.

13. Verwendung einer Population von Mäusen und Ratten zum Sektorisieren eines Antikörper-Gen-Segment-Repertoires, wobei das Genom jeder Maus oder Ratte umfasst:
(a) einen oder mehrere transgene Immunglobulin-Schwerketten-Loci, wobei jeder Locus eines oder mehrere humane V-Gensegmente, eines oder mehrere humane D-Gensegmente und eines oder mehrere humane J-Gensegmente umfasst, die funktionsmäßig stromaufwärts von einer oder mehreren konstanten Regionen der schweren Kette verknüpft sind, wobei die konstante(n) Region(en) der schweren Kette ein C-Gamma-Gensegment umfasst (umfassen); und
(b) einen oder mehrere transgene Immunglobulin-Leichtketten-Loci, wobei jeder Locus ein humanes Vλ-Gensegment-Repertoire und eines oder mehrere menschliche Jλ-Gensegmente umfasst, die funktionsmäßig stromaufwärts von einer oder mehreren konstanten Regionen der leichten Kette verknüpft sind;
wobei nach der Umlagerung die Loci der leichten Kette Sequenzen der leichten Kette exprimieren, die Sequenzen variabler Regionen umfassen, die von humanen Vλ-Gensegmenten (Sequenzen der leichten Lambda-Kette) abgeleitet sind;
wobei die Kappa- und endogene Expression der leichten Lambda-Kette inaktiv sind, so dass jede Maus oder Ratte mehr humane Sequenzen der leichten Lambda-Kette exprimiert als Sequenzen der leichten Kappa-Kette (Sequenzen der leichten Ketten, die Sequenzen variabler Regionen umfassen, die von V_{κ}-Gensegmenten abgeleitet sind);
wobei die endogene Expression der schweren Kette inaktiviert wurde; und
wobei das humane V_{λ}-Gen-Segment-Repertoire über das Maus- oder Ratten-Genom aufgeteilt ist, wobei erste und zweite einzelne Mitglieder der Population ein unterschiedliches Unterrepertoire von humanen Vx-Gen-Segmenten umfassen und die Genome unterschiedliche Repertoires von Antikörper-Lambda-Ketten-Sequenzen exprimieren können,
und jede der Mäuse oder Ratten der Population mit dem gleichen Antigen und einem oder mehreren Antikörpern (Lambda-Typ-Antikörper) immunisiert wird, die das Antigen binden, dessen variable Regionen der schweren und leichten Kette von humanen VH- bzw. Vλ-Gensegmenten abgeleitet sind, isoliert.

14. Verwendung nach Anspruch 13, wobei das Vx-Gen-Segment-Repertoire ein vollständiges funktionelles V_{λ}-Gen-Segment-Repertoire ist, wobei die Mäuse oder Ratten wahlweise die folgenden humanen V_{λ}-Gensegmente umfassen V3-1, V2-8, V3-9, V3-10, V2-11, V3-12, V3-16, V2-18, V3-19, V3- 21, V3-22, V2-23, V3-25, V3-27, V1-36, V5-37, V5-39, V1-40, V7-43, V1-44, V5-45, V7-46, V1-74, V9-49, V1-51, V5-52, V6-57, V4-60, V8-61 und V4-69.

## Revendications

1. Utilisation d'une souris ou d'un rat dans la sectorisation d'un répertoire de segments géniques d'anticorps, dans laquelle le génome de la souris ou du rat comprend :
i) Un ou plusieurs loci de chaîne lourde d'immunoglobine transgénique, chaque locus comprenant un ou plusieurs segments géniques V humains, un ou plusieurs segments géniques D humains et un ou plusieurs segments géniques J humains liés de manière opérationnelle en amont d'une ou plusieurs régions constantes de chaîne lourde, dans laquelle la (les) région(s) constante(s) de chaîne lourde comprend (comprennent) un segment génique gamma C ; et
ii) Un ou plusieurs loci de chaîne légère d'immunoglobine transgénique, chaque locus comprenant un répertoire de segments géniques V_{λ} humains et un ou plusieurs segments géniques Jλ humains liés de manière opérationnelle en amont d'une ou plusieurs régions constantes de chaîne légère ;
Dans laquelle après le réagencement les loci de chaîne légère expriment des séquences de chaînes légères comprenant des séquences de régions variables dérivées de segments géniques Vλ humains (séquences de chaînes légères lambda humaines) ;
Dans laquelle l'expression de la chaîne légère kappa (et optionnellement lambda endogène) a été inactivée de sorte que la souris ou le rat n'exprime pas d'anticorps de type kappa ;
Dans laquelle l'expression de la chaîne lourde endogène a été inactivée ; et
Dans laquelle chaque dit locus de chaîne légère transgénique comprend un répertoire complet de segments géniques V_{λ} fonctionnels d'un humain et
Dans laquelle la souris ou le rat exprime les séquences de chaînes légères lambda humaines lors de l'immunisation pour fournir un répertoire d'anticorps.

2. Utilisation selon la revendication 1, dans laquelle chaque dit locus de chaîne légère transgénique comprend au moins 20, 25, 26, 27, 28 ou 29 segments géniques V_{λ} humains choisis dans le groupe constitué de V3-1, V2-8, V3-9, V3-10, V2-11, V3-12, V3-16, V2-18, V3-19, V3-21, V3-22, V2-23, V3-25, V3-27, V1-36, V5-37, V5-39, V1-40, V7-43, V1-44, V5-45, V7-46, V1-74, V9-49, V1-51, V5-52, V6-57, V4-60, V8-61 et V4-69; optionnellement tous les segments géniques du groupe.

3. Utilisation selon la revendication 1 ou 2, dans laquelle chaque dit locus de chaîne légère transgénique comprend un répertoire complet de segments géniques J_{λ} fonctionnels d'un humain.

4. Utilisation selon l'une quelconque des revendications 1 à 3, dans laquelle chaque dit locus de chaîne légère transgénique comprend au moins 3 ou 4 segments géniques J_{λ} humains choisis dans le groupe constitué de J1, J2, J3, J6 et J7 ; optionnellement tous les segments géniques du groupe.

5. Utilisation selon l'une quelconque des revendications 1 à 4, dans laquelle chaque dit locus de chaîne lourde transgénique comprend un répertoire complet de segments géniques VH fonctionnels d'un humain.

6. Utilisation selon la revendication 5, dans laquelle chaque dit locus de chaîne lourde transgénique comprend au moins 30, 35, 36, 37, 38 ou 39 segments géniques VH humains choisis dans le groupe constitué de V6-1, V1-2, V1-3, V4-4, V7-41, V2-5, V3-7, V1-8, V3-9, V3-11, V3-15, V1-18, V3-20, V3-21, V3-23, V1-24, V2-26, V4-28, V3-30, V4-31, V3-33, V4-34, V4-39, V3-43, V1-45, V1-46, V3-48, V3-49, V5-51, V3-53, V1-58, V4-59, V4-61, V3-64, V3-66, V1-69, V2-70, V3-72, V3-73 et V3-74; optionnellement tous les segments géniques du groupe.

7. Utilisation selon l'une quelconque des revendications 1 à 6, dans laquelle chaque dit locus de chaîne lourde transgénique comprend au moins 4 ou 5 segments géniques JH humains choisis dans le groupe constitué de J1, J2, J3, J4, J5, J6 ; optionnellement tous les segments géniques du groupe.

8. Utilisation selon l'une quelconque des revendications 1 à 7, dans laquelle chaque dit locus de chaîne lourde transgénique comprend au moins 15, 20, 21, 22, 23, 24, 25 ou 26 segments géniques D humains choisis dans le groupe constitué de D1-1, D2-2, D3-3, D4-4, D5-5, D6-6, D1-7, D2-8, D3-9, D3-10, D4-11, D5-12, D6-13, D1-14, D2-15, D3-16, D4-17, D5-18, D6-19, D1-20, D2-21, D3-22, D4-23, D5-24, D1-26, D6-25 et D7-27; optionnellement tous les segments géniques du groupe.

9. Utilisation selon l'une quelconque des revendications 1 à 8, dans laquelle les régions constantes de chaîne lourde sont des régions constantes de chaîne lourde de vertébré non humain.

10. Utilisation selon l'une quelconque des revendications 1 à 8, dans laquelle les régions constantes de chaîne lourde sont de souris ou de rat.

11. Utilisation selon l'une quelconque des revendications 1 à 8, dans laquelle les régions constantes de chaîne lourde sont endogènes et/ou les régions constantes de chaîne légère sont endogènes.

12. Utilisation d'une population de souris ou de rats dans la sectorisation d'un répertoire de segments géniques d'anticorps, dans laquelle la population comprend un premier type de souris ou de rat comprenant un génome selon l'une quelconque des revendications 1 à 11 et la population comprend en outre un second type de souris ou de rat (souris ou rats kappa) dans laquelle le génome du second type de souris ou de rat comprend :
a. un ou plusieurs loci de chaîne lourde d'immunoglobine transgénique, chaque locus comprenant un ou plusieurs segments géniques V humains, un ou plusieurs segments géniques D humains et un ou plusieurs segments géniques J humains en amont d'une ou plusieurs régions constantes de chaîne lourde ; et
b. un ou plusieurs loci de chaîne légère d'immunoglobine transgénique, chaque locus comprenant un ou plusieurs segments géniques V_{L} humains et un ou plusieurs segments géniques J_{L} humains en amont d'une ou plusieurs régions constantes de chaîne légère ;
dans laquelle lesdits loci de chaîne légère comprennent un ou plusieurs segments géniques Vκ humains, dans laquelle après le réagencement les loci expriment des séquences de chaînes légères comprenant des séquences de régions variables dérivées de segments géniques Vκ humains (séquences de chaînes légères kappa humaines),
dans laquelle chaque souris ou rat kappa exprime davantage les séquences de chaînes légères kappa humaines que les séquences de chaînes légères lambda (séquences de chaînes légères comprenant des régions variables dérivées de segments géniques Vλ) ; et
dans laquelle le premier type de souris ou de rat exprimant les séquences de chaînes légères lambda humaines ne partage pas de segments géniques de chaînes légères humaines communs avec les souris ou les rats kappa et dans laquelle les souris ou les rats de ladite population sont capables de produire un répertoire d'anticorps comprenant des régions variables de chaîne lourde et légère humaine lorsqu'ils sont immunisés par le même antigène, dans laquelle le répertoire comprend un sous-répertoire d'anticorps lambda humains (sous-répertoire lambda) produits par des souris ou des rats du premier type et un sous-répertoire d'anticorps kappa humains (sous-répertoire kappa) produits par des souris ou des rats du second type.

13. Utilisation d'une population de souris ou de rats dans la sectorisation d'un répertoire de segments géniques d'anticorps, dans laquelle le génome de chaque souris ou rat comprend :
(a) Un ou plusieurs loci de chaîne lourde d'immunoglobine transgénique, chaque locus comprenant un ou plusieurs segments géniques V humains, un ou plusieurs segments géniques D humains et un ou plusieurs segments géniques J humains liés de manière opérationnelle en amont d'une ou plusieurs régions constantes de chaîne lourde, dans laquelle la (les) région(s) constante(s) de chaîne lourde comprend (comprennent) un segment génique gamma C ; et
(b) Un ou plusieurs loci de chaîne légère d'immunoglobine transgénique, chaque locus comprenant un répertoire de segments géniques V_{λ} humains et un ou plusieurs segments géniques Jλ humains liés de manière opérationnelle en amont d'une ou plusieurs régions constantes de chaîne légère ;
Dans laquelle après le réagencement les loci de chaîne légère expriment des séquences de chaînes légères comprenant des séquences de régions variables dérivées de segments géniques Vλ humains (séquences de chaînes légères lambda humaines) ;
Dans laquelle l'expression des chaînes légères kappa et lambda endogène est inactive de sorte que chaque souris ou rat exprime davantage les séquences de chaînes légères lambda humaines que les séquences de chaînes légères kappa (séquences de chaînes légères comprenant des séquences de régions variables dérivées de segments géniques VK) ;
Dans laquelle l'expression de la chaîne lourde endogène a été inactivée ; et
Dans laquelle le répertoire de segments géniques V_{λ} humains est divisé dans les génomes de la souris ou du rat, dans laquelle les premier et second membres individuels de la population comprennent un sous-répertoire différent de segments géniques V_{λ} humains et les génomes peuvent exprimer différents répertoires de séquences de chaînes lambda d'anticorps,
et chacun des souris ou des rats de la population est immunisé par le même antigène et un ou plusieurs anticorps (anticorps de type lambda) qui se lient à l'antigène dont les régions variables de chaîne lourde et légère sont dérivées de segments géniques VH et Vλ humains respectivement sont isolés.

14. Utilisation selon la revendication 13, dans laquelle le répertoire de segments géniques V_{λ} est un répertoire complet de segments géniques V_{λ} fonctionnels, optionnellement dans laquelle les souris ou les rats comprennent les segments géniques V_{λ} humains suivants V3-1, V2-8, V3-9, V3-10, V2-11, V3-12, V3-16, V2-18, V3-19, V3-21, V3-22, V2-23, V3-25, V3-27, V1-36, V5-37, V5-39, V1-40, V7-43, V1-44, V5-45, V7-46, V1-74, V9-49, V1-51, V5-52, V6-57, V4-60, V8-61 et V4-69.


### SEQUENCE LISTING

<110> Kymab
<120> SYNTHETICALLY-EXTENDED & ETHNICALLY DIVERSE SUPERHUMAN IMMUNOGLOBULIN GENE REPERTOIRES
<130> 25077.21
<160> 7
<170> PatentIn version 3.5
<210> 1
   <211> 287
   <212> DNA
   <213> Homo sapiens
<400> 1
   gacatccagt tgacccagtc tccatccttc ctgtctgcat ctgtaggaga cagagtcacc       60
   atcacttgct gggccagtca gggcattagc agttatttag cctggtatca gcaaaaacca      120
   gggaaagccc ctaagctcct gatctatgct gcatccactt tgcaaagtgg ggtcccatca      180
   aggttcagcg gcagtggatc tgggacagaa ttcactctca caatcagcag cctgcagcct      240
   gaagattttg caacttatta ctgtcaacag cttaatagtt accctcc                    287
<210> 2
   <211> 287
   <212> DNA
   <213> Homo sapiens
<400> 2
   gaaattgtgt tgacacagtc tccagccacc ctgtctttgt ctccagggga aagagccacc       60
   ctctcctgca gggccagtca gagtgttagc agctacttag cctggtacca acagaaacct      120
   ggccaggctc ccaggctcct catctatgat gcatccaaca gggccactgg catcccagcc      180
   aggttcagtg gcagtgggtc tgggacagac ttcactctca ccatcagcag cctagagcct      240
   gaagattttg cagtttatta ctgtcagcag cgtagcaact ggcctcc                    287
<210> 3
   <211> 287
   <212> DNA
   <213> Homo sapiens
<400> 3
   gacatccaga tgacccagtc tccatcttcc gtgtctgcat ctgtaggaga cagagtcacc       60
   atcacttgtc gggcgagtca gggtattagc agctggttag cctggtatca gcagaaacca      120
   gggaaagccc ctaagctcct gatctatgct gcatccagtt tgcaaagtgg ggtcccatca      180
   aggttcagcg gcagtggatc tgggacagat ttcactctca ccatcagcag cctgcagcct      240
   gaagattttg caacttacta ttgtcaacag gctaacagtt tccctcc                    287
<210> 4
   <211> 287
   <212> DNA
   <213> Homo sapiens
<400> 4
   gacatccaga tgacccagtc tccatcttcc gtgtctgcat ctgtaggaga cagagtcacc     60
   atcacttgtc gggcgagtca gggtattagc agctggttag cctggtatca gcagaaacca    120
   gggaaagccc ctaagctcct gatctatgct gcatccagtt tgcaaagtgg ggtcccatca    180
   aggttcagcg gcagtggatc tgggacagat ttcactctca ccatcagcag cctgcagcct    240
   gaagattttg caacttacta ttgtcaacag gctaacagtt tccctcc                  287
<210> 5
   <211> 287
   <212> DNA
   <213> Homo sapiens
<400> 5
   gccatctgga tgacccagtc tccatcctta ctctctgcat ctacaggaga cagagtcacc     60
   atcagttgtc ggatgagtca gggcattagc agttatttag cctggtatca gcaaaaacca    120
   gggaaagccc ctgagctcct gatctatgct gcatccactt tgcaaagtgg ggtcccatca    180
   aggttcagtg gcagtggatc tgggacagat ttcactctca ccatcagctg cctgcagtct    240
   gaagattttg caacttatta ctgtcaacag tattatagtt tccctcc                  287
<210> 6
   <211> 287
   <212> DNA
   <213> Homo sapiens
<400> 6
   gaaattgtgt tgacacagtc tccagccacc ctgtctttgt ctccagggga aagagccacc     60
   ctctcctgca gggccagtca gagtgttagc agctacttag cctggtacca gcagaaacct    120
   ggccaggctc ccaggctcct catctatgat gcatccaaca gggccactgg catcccagcc    180
   aggttcagtg gcagtgggcc tgggacagac ttcactctca ccatcagcag cctagagcct    240
   gaagattttg cagtttatta ctgtcagcag cgtagcaact ggcatcc                  287
<210> 7
   <211> 287
   <212> DNA
   <213> Homo sapiens
<400> 7
   gccatccagt tgacccagtc tccatcctcc ctgtctgcat ctgtaggaga cagagtcacc      60
   atcacttgcc gggcaagtca gggcattagc agtgctttag cctggtatca gcagaaacca     120
   gggaaagctc ctaagctcct gatctatgat gcctccagtt tggaaagtgg ggtcccatca     180
   aggttcagcg gcagtggatc tgggacagat ttcactctca ccatcagcag cctgcagcct     240
   gaagattttg caacttatta ctgtcaacag tttaatagtt accctcc                  287